# EUROPEAN PATENT APPLICATION

(11) **EP 4 089 115 A1**
(43) Date of publication of application: **16.11.2022**
(21) Application number: 21738755.4
(22) Date of filing: 08.01.2021
(51) Int. Cl.: C07K 16/28, C07K 19/00, C12N 15/13, C12N 15/63, C12N 5/10, A61K 39/395, A61P 35/00

(54) **ANTI-TIGIT ANTIBODIES AND USAGE METHOD**

(30) Priority: 10.01.2020 CN 202010024565
(71) Applicant: Shanghai Henlius Biotech, Inc., Shanghai 201210 (CN)
(72) Inventor: YANG, Ming, Shanghai 201210 (CN); XU, Wenfeng, Shanghai 201210 (CN); JIANG, Wei-Dong, Shanghai 201210 (CN); XUE, Jie, Shanghai 201210 (CN)
(74) Representative: Rentsch Partner AG
(86) International application number: PCT/CN2021/070903
(87) International publication number: WO 2021/139777

(57) **Abstract**

The present invention relates to anti-TIGIT antibodies that bind to "T cell immunoreceptor with Ig and ITIM domains (TIGIT)", including multispecific anti-TIGIT antibodies with binding specificity for TIGIT and one or more additional antigen, and methods of using the same. In certain embodiments, the anti-TIGIT antibodies comprises a single domain antibody that binds to TIGIT.

## Description

### CROSS-REFERENCE TO RELATED APPLICATIONS

This application claims priority to a Chinese Patent Application with Application No. CN202010024565.9, filed January 10, 2020, the contents of which is incorporated by reference in its entirety, and to which priority is claimed.

### FIELD

The present invention relates to antibodies that bind to T cell immunoreceptor with Ig and ITIM domains (TIGIT), including multispecific anti-TIGIT antibodies with binding specificity for TIGIT and one or more additional antigen, and methods of using the same.

### BACKGROUND

T cell immunoreceptor with Ig and ITIM domains (TIGIT) is an immune checkpoint receptor expressed by immune cells such as activated T cells and Natural Killer cells (NK cells) and mediates immunosuppression. The ligands of TIGIT includes PVR (CD155), which has been identified on dendritic cells (DCs), macrophages as well as many human cancer cells and have been shown to downregulate T cell activation and cytokine secretion upon binding to TIGIT. Inhibition of the TIGIT/PVR interaction can mediate potent antitumor activity of immune cells. Given the significant role for TIGIT in immune checkpoint regulation, there remains a need in the art for the development of therapeutic molecules and methods to modulate TIGIT-mediated immune cell regulation for immune therapy and cancer treatment.

### SUMMARY OF THE INVENTION

The present disclosure provides isolated monoclonal antibodies that bind specifically to TIGIT with high affinity, including multispecific antibodies that binds to TIGIT and one or more additional target. In certain embodiments, the anti-TIGIT antibody comprises a single domain antibody that binds to TIGIT. This disclosure further provides methods of making and using the antibodies, immunoconjugates and pharmaceutical compositions comprising the antibodies, e.g., for treating diseases and disorders, e.g., cancer. The invention is based, in part, on the discovery of single domain anti-TIGIT antibodies that bind to TIGIT, which can increase an immune response in immune cells and provide improved anti-tumor efficacy.

In certain embodiments, the anti-TIGIT antibody comprises a single domain antibody that binds to TIGIT. In certain embodiments, the single domain antibody binds to TIGIT with a KD of about 1×10⁻⁷ M or less. In certain embodiments, the single domain antibody binds to TIGIT with a KD of about 1×10⁻⁸ M or less. In certain embodiments, the single domain antibody binds to TIGIT with a KD of about 5×10⁻⁹ M or less. In certain embodiments, the single domain antibody binds to TIGIT with a KD of about 2×10⁻⁹ M or less.

In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 94, a heavy chain variable region CDR2 comprising amino acids having the sequence set forth in SEQ ID NO: 95, and a heavy chain variable region CDR3 comprising amino acids having the sequence set forth in SEQ ID NO: 96. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 98, a heavy chain variable region CDR2 comprising amino acids having the sequence set forth in SEQ ID NO: 99, and a heavy chain variable region CDR3 comprising amino acids having the sequence set forth in SEQ ID NO: 100. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 102, a heavy chain variable region CDR2 comprising amino acids having the sequence set forth in SEQ ID NO: 103, and a heavy chain variable region CDR3 comprising amino acids having the sequence set forth in SEQ ID NO: 104. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 106, a heavy chain variable region CDR2 comprising amino acids having the sequence set forth in SEQ ID NO: 107, and a heavy chain variable region CDR3 comprising amino acids having the sequence set forth in SEQ ID NO: 108. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 110, a heavy chain variable region CDR2 comprising amino acids having the sequence set forth in SEQ ID NO: 111, and a heavy chain variable region CDR3 comprising amino acids having the sequence set forth in SEQ ID NO: 112. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 114, a heavy chain variable region CDR2 comprising amino acids having the sequence set forth in SEQ ID NO: 115, and a heavy chain variable region CDR3 comprising amino acids having the sequence set forth in SEQ ID NO: 116. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 118, a heavy chain variable region CDR2 comprising amino acids having the sequence set forth in SEQ ID NO: 119, and a heavy chain variable region CDR3 comprising amino acids having the sequence set forth in SEQ ID NO: 120. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 122, a heavy chain variable region CDR2 comprising amino acids having the sequence set forth in SEQ ID NO: 123, and a heavy chain variable region CDR3 comprising amino acids having the sequence set forth in SEQ ID NO: 124. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 126, a heavy chain variable region CDR2 comprising amino acids having the sequence set forth in SEQ ID NO: 127, and a heavy chain variable region CDR3 comprising amino acids having the sequence set forth in SEQ ID NO: 128. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 130, a heavy chain variable region CDR2 comprising amino acids having the sequence set forth in SEQ ID NO: 131, and a heavy chain variable region CDR3 comprising amino acids having the sequence set forth in SEQ ID NO: 132. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 134, a heavy chain variable region CDR2 comprising amino acids having the sequence set forth in SEQ ID NO: 135, and a heavy chain variable region CDR3 comprising amino acids having the sequence set forth in SEQ ID NO: 136. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 138, a heavy chain variable region CDR2 comprising amino acids having the sequence set forth in SEQ ID NO: 139, and a heavy chain variable region CDR3 comprising amino acids having the sequence set forth in SEQ ID NO: 140. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 142, a heavy chain variable region CDR2 comprising amino acids having the sequence set forth in SEQ ID NO: 143, and a heavy chain variable region CDR3 comprising amino acids having the sequence set forth in SEQ ID NO: 144. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 146, a heavy chain variable region CDR2 comprising amino acids having the sequence set forth in SEQ ID NO: 147, and a heavy chain variable region CDR3 comprising amino acids having the sequence set forth in SEQ ID NO: 148. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 150, a heavy chain variable region CDR2 comprising amino acids having the sequence set forth in SEQ ID NO: 151, and a heavy chain variable region CDR3 comprising amino acids having the sequence set forth in SEQ ID NO: 152. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 154, a heavy chain variable region CDR2 comprising amino acids having the sequence set forth in SEQ ID NO: 155, and a heavy chain variable region CDR3 comprising amino acids having the sequence set forth in SEQ ID NO: 156. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 158, a heavy chain variable region CDR2 comprising amino acids having the sequence set forth in SEQ ID NO: 159, and a heavy chain variable region CDR3 comprising amino acids having the sequence set forth in SEQ ID NO: 160. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 162, a heavy chain variable region CDR2 comprising amino acids having the sequence set forth in SEQ ID NO: 163, and a heavy chain variable region CDR3 comprising amino acids having the sequence set forth in SEQ ID NO: 164. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 166, a heavy chain variable region CDR2 comprising amino acids having the sequence set forth in SEQ ID NO: 167, and a heavy chain variable region CDR3 comprising amino acids having the sequence set forth in SEQ ID NO: 168. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 170, a heavy chain variable region CDR2 comprising amino acids having the sequence set forth in SEQ ID NO: 171, and a heavy chain variable region CDR3 comprising amino acids having the sequence set forth in SEQ ID NO: 172. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 174, a heavy chain variable region CDR2 comprising amino acids having the sequence set forth in SEQ ID NO: 175, and a heavy chain variable region CDR3 comprising amino acids having the sequence set forth in SEQ ID NO: 176. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 178, a heavy chain variable region CDR2 comprising amino acids having the sequence set forth in SEQ ID NO: 179, and a heavy chain variable region CDR3 comprising amino acids having the sequence set forth in SEQ ID NO: 180. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 182, a heavy chain variable region CDR2 comprising amino acids having the sequence set forth in SEQ ID NO: 183, and a heavy chain variable region CDR3 comprising amino acids having the sequence set forth in SEQ ID NO: 184. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 186, a heavy chain variable region CDR2 comprising amino acids having the sequence set forth in SEQ ID NO: 187, and a heavy chain variable region CDR3 comprising amino acids having the sequence set forth in SEQ ID NO: 188. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 190, a heavy chain variable region CDR2 comprising amino acids having the sequence set forth in SEQ ID NO: 191, and a heavy chain variable region CDR3 comprising amino acids having the sequence set forth in SEQ ID NO: 192.

In certain embodiments, the single domain antibody comprises a heavy chain variable region comprising: a) a heavy chain variable region CDR1 comprises an amino acid sequence of any one of SEQ ID NOs: 94, 98, 102, 106, 110, 114, 118, 122, 126, 130, 134, 138, 142, 146, 150, 154, 158, 162, 166, 170, 174, 178, 182, 186 and 190, or a variant thereof comprising up to about 3 amino acid substitutions; b) a heavy chain variable region CDR2 comprises an amino acid sequence of any one of SEQ ID NOs: 95, 99, 103, 107, 111, 115, 119, 123, 127, 131, 135, 139, 143, 147, 151, 155, 159, 163, 167, 171, 175, 179, 183, 187 and 191, or a variant thereof comprising up to about 3 amino acid substitutions; and c) a heavy chain variable region CDR3 comprises an amino acid sequence of any one of SEQ ID NOs: 96, 100, 104, 108, 112, 116, 120, 124, 128, 132, 136, 140, 144, 148, 152, 156, 160, 164, 168, 172, 176, 180, 184, 188 and 192, or a variant thereof comprising up to about 3 amino acid substitutions.

In certain embodiments, the single domain antibody comprises a heavy chain variable region that comprises a CDR1 domain, a CDR2 domain and a CDR3 domain, wherein the CDR1 domain, the CDR2 domain and the CDR3 domain respectively comprise a CDR1 domain, a CDR2 domain and a CDR3 domain comprised in a reference heavy chain variable region comprising the amino acid sequence selected from the group consisting of SEQ ID NOs: 97, 101, 105, 109, 113, 117, 121, 125, 129, 133, 137, 141, 145, 149, 153, 157, 161, 165, 169, 173, 177, 181, 185, 189 and 193.

In certain embodiments, the single domain antibody comprises a heavy chain variable region CDR1 comprising amino acids having the sequence set forth in SEQ ID NO: 94, a heavy chain variable region CDR2 comprising amino acids having the sequence set forth in SEQ ID NO: 95, and a heavy chain variable region CDR3 comprising amino acids having the sequence set forth in SEQ ID NO: 96. In certain embodiments, the single domain antibody comprises a heavy chain variable region CDR1 comprising amino acids having the sequence set forth in SEQ ID NO: 98, a heavy chain variable region CDR2 comprising amino acids having the sequence set forth in SEQ ID NO: 99, and a heavy chain variable region CDR3 comprising amino acids having the sequence set forth in SEQ ID NO: 100. In certain embodiments, the single domain antibody comprises a heavy chain variable region CDR1 comprising amino acids having the sequence set forth in SEQ ID NO: 102, a heavy chain variable region CDR2 comprising amino acids having the sequence set forth in SEQ ID NO: 103, and a heavy chain variable region CDR3 comprising amino acids having the sequence set forth in SEQ ID NO: 104. In certain embodiments, the single domain antibody comprises a heavy chain variable region CDR1 comprising amino acids having the sequence set forth in SEQ ID NO: 106, a heavy chain variable region CDR2 comprising amino acids having the sequence set forth in SEQ ID NO: 107, and a heavy chain variable region CDR3 comprising amino acids having the sequence set forth in SEQ ID NO: 108. In certain embodiments, the single domain antibody comprises a heavy chain variable region CDR1 comprising amino acids having the sequence set forth in SEQ ID NO: 110, a heavy chain variable region CDR2 comprising amino acids having the sequence set forth in SEQ ID NO: 111, and a heavy chain variable region CDR3 comprising amino acids having the sequence set forth in SEQ ID NO: 112. In certain embodiments, the single domain antibody comprises a heavy chain variable region CDR1 comprising amino acids having the sequence set forth in SEQ ID NO: 114, a heavy chain variable region CDR2 comprising amino acids having the sequence set forth in SEQ ID NO: 115, and a heavy chain variable region CDR3 comprising amino acids having the sequence set forth in SEQ ID NO: 116. In certain embodiments, the single domain antibody comprises a heavy chain variable region CDR1 comprising amino acids having the sequence set forth in SEQ ID NO: 118, a heavy chain variable region CDR2 comprising amino acids having the sequence set forth in SEQ ID NO: 119, and a heavy chain variable region CDR3 comprising amino acids having the sequence set forth in SEQ ID NO: 120. In certain embodiments, the single domain antibody comprises a heavy chain variable region CDR1 comprising amino acids having the sequence set forth in SEQ ID NO: 122, a heavy chain variable region CDR2 comprising amino acids having the sequence set forth in SEQ ID NO: 123, and a heavy chain variable region CDR3 comprising amino acids having the sequence set forth in SEQ ID NO: 124. In certain embodiments, the single domain antibody comprises a heavy chain variable region CDR1 comprising amino acids having the sequence set forth in SEQ ID NO: 126, a heavy chain variable region CDR2 comprising amino acids having the sequence set forth in SEQ ID NO: 127, and a heavy chain variable region CDR3 comprising amino acids having the sequence set forth in SEQ ID NO: 128. In certain embodiments, the single domain antibody comprises a heavy chain variable region CDR1 comprising amino acids having the sequence set forth in SEQ ID NO: 130, a heavy chain variable region CDR2 comprising amino acids having the sequence set forth in SEQ ID NO: 131, and a heavy chain variable region CDR3 comprising amino acids having the sequence set forth in SEQ ID NO: 132. In certain embodiments, the single domain antibody comprises a heavy chain variable region CDR1 comprising amino acids having the sequence set forth in SEQ ID NO: 134, a heavy chain variable region CDR2 comprising amino acids having the sequence set forth in SEQ ID NO: 135, and a heavy chain variable region CDR3 comprising amino acids having the sequence set forth in SEQ ID NO: 136. In certain embodiments, the single domain antibody comprises a heavy chain variable region CDR1 comprising amino acids having the sequence set forth in SEQ ID NO: 138, a heavy chain variable region CDR2 comprising amino acids having the sequence set forth in SEQ ID NO: 139, and a heavy chain variable region CDR3 comprising amino acids having the sequence set forth in SEQ ID NO: 140. In certain embodiments, the single domain antibody comprises a heavy chain variable region CDR1 comprising amino acids having the sequence set forth in SEQ ID NO: 142, a heavy chain variable region CDR2 comprising amino acids having the sequence set forth in SEQ ID NO: 143, and a heavy chain variable region CDR3 comprising amino acids having the sequence set forth in SEQ ID NO: 144. In certain embodiments, the single domain antibody comprises a heavy chain variable region CDR1 comprising amino acids having the sequence set forth in SEQ ID NO: 146, a heavy chain variable region CDR2 comprising amino acids having the sequence set forth in SEQ ID NO: 147, and a heavy chain variable region CDR3 comprising amino acids having the sequence set forth in SEQ ID NO: 148. In certain embodiments, the single domain antibody comprises a heavy chain variable region CDR1 comprising amino acids having the sequence set forth in SEQ ID NO: 150, a heavy chain variable region CDR2 comprising amino acids having the sequence set forth in SEQ ID NO: 151, and a heavy chain variable region CDR3 comprising amino acids having the sequence set forth in SEQ ID NO: 152. In certain embodiments, the single domain antibody comprises a heavy chain variable region CDR1 comprising amino acids having the sequence set forth in SEQ ID NO: 154, a heavy chain variable region CDR2 comprising amino acids having the sequence set forth in SEQ ID NO: 155, and a heavy chain variable region CDR3 comprising amino acids having the sequence set forth in SEQ ID NO: 156. In certain embodiments, the single domain antibody comprises a heavy chain variable region CDR1 comprising amino acids having the sequence set forth in SEQ ID NO: 158, a heavy chain variable region CDR2 comprising amino acids having the sequence set forth in SEQ ID NO: 159, and a heavy chain variable region CDR3 comprising amino acids having the sequence set forth in SEQ ID NO: 160. In certain embodiments, the single domain antibody comprises a heavy chain variable region CDR1 comprising amino acids having the sequence set forth in SEQ ID NO: 162, a heavy chain variable region CDR2 comprising amino acids having the sequence set forth in SEQ ID NO: 163, and a heavy chain variable region CDR3 comprising amino acids having the sequence set forth in SEQ ID NO: 164. In certain embodiments, the single domain antibody comprises a heavy chain variable region CDR1 comprising amino acids having the sequence set forth in SEQ ID NO: 166, a heavy chain variable region CDR2 comprising amino acids having the sequence set forth in SEQ ID NO: 167, and a heavy chain variable region CDR3 comprising amino acids having the sequence set forth in SEQ ID NO: 168. In certain embodiments, the single domain antibody comprises a heavy chain variable region CDR1 comprising amino acids having the sequence set forth in SEQ ID NO: 170, a heavy chain variable region CDR2 comprising amino acids having the sequence set forth in SEQ ID NO: 171, and a heavy chain variable region CDR3 comprising amino acids having the sequence set forth in SEQ ID NO: 172. In certain embodiments, the single domain antibody comprises a heavy chain variable region CDR1 comprising amino acids having the sequence set forth in SEQ ID NO: 174, a heavy chain variable region CDR2 comprising amino acids having the sequence set forth in SEQ ID NO: 175, and a heavy chain variable region CDR3 comprising amino acids having the sequence set forth in SEQ ID NO: 176. In certain embodiments, the single domain antibody comprises a heavy chain variable region CDR1 comprising amino acids having the sequence set forth in SEQ ID NO: 178, a heavy chain variable region CDR2 comprising amino acids having the sequence set forth in SEQ ID NO: 179, and a heavy chain variable region CDR3 comprising amino acids having the sequence set forth in SEQ ID NO: 180. In certain embodiments, the single domain antibody comprises a heavy chain variable region CDR1 comprising amino acids having the sequence set forth in SEQ ID NO: 182, a heavy chain variable region CDR2 comprising amino acids having the sequence set forth in SEQ ID NO: 183, and a heavy chain variable region CDR3 comprising amino acids having the sequence set forth in SEQ ID NO: 184. In certain embodiments, the single domain antibody comprises a heavy chain variable region CDR1 comprising amino acids having the sequence set forth in SEQ ID NO: 186, a heavy chain variable region CDR2 comprising amino acids having the sequence set forth in SEQ ID NO: 187, and a heavy chain variable region CDR3 comprising amino acids having the sequence set forth in SEQ ID NO: 188. In certain embodiments, the single domain antibody comprises a heavy chain variable region CDR1 comprising amino acids having the sequence set forth in SEQ ID NO: 190, a heavy chain variable region CDR2 comprising amino acids having the sequence set forth in SEQ ID NO: 191, and a heavy chain variable region CDR3 comprising amino acids having the sequence set forth in SEQ ID NO: 192.

In certain embodiments, the single domain antibody comprises a heavy chain variable region comprising an amino acid sequence having at least about 90% sequence identity to an amino acid sequence selected from the group consisting of SEQ ID NOs: 97, 101, 105, 109, 113, 117, 121, 125, 129, 133, 137, 141, 145, 149, 153, 157, 161, 165, 169, 173, 177, 181, 185, 189 and 193.

In certain embodiments, the single domain antibody comprises a heavy chain variable region comprising the amino acid sequence set forth in SEQ ID NO: 97. In certain embodiments, the single domain antibody comprises a heavy chain variable region comprising the amino acid sequence set forth in SEQ ID NO: 101. In certain embodiments, the single domain antibody comprises a heavy chain variable region comprising the amino acid sequence set forth in SEQ ID NO: 105. In certain embodiments, the single domain antibody comprises a heavy chain variable region comprising the amino acid sequence set forth in SEQ ID NO: 109. In certain embodiments, the single domain antibody comprises a heavy chain variable region comprising the amino acid sequence set forth in SEQ ID NO: 113. In certain embodiments, the single domain antibody comprises a heavy chain variable region comprising the amino acid sequence set forth in SEQ ID NO: 117. In certain embodiments, the single domain antibody comprises a heavy chain variable region comprising the amino acid sequence set forth in SEQ ID NO: 121. In certain embodiments, the single domain antibody comprises a heavy chain variable region comprising the amino acid sequence set forth in SEQ ID NO: 125. In certain embodiments, the single domain antibody comprises a heavy chain variable region comprising the amino acid sequence set forth in SEQ ID NO: 129. In certain embodiments, the single domain antibody comprises a heavy chain variable region comprising the amino acid sequence set forth in SEQ ID NO: 133. In certain embodiments, the single domain antibody comprises a heavy chain variable region comprising the amino acid sequence set forth in SEQ ID NO: 137. In certain embodiments, the single domain antibody comprises a heavy chain variable region comprising the amino acid sequence set forth in SEQ ID NO: 141. In certain embodiments, the single domain antibody comprises a heavy chain variable region comprising the amino acid sequence set forth in SEQ ID NO: 145. In certain embodiments, the single domain antibody comprises a heavy chain variable region comprising the amino acid sequence set forth in SEQ ID NO: 149. In certain embodiments, the single domain antibody comprises a heavy chain variable region comprising the amino acid sequence set forth in SEQ ID NO: 153. In certain embodiments, the single domain antibody comprises a heavy chain variable region comprising the amino acid sequence set forth in SEQ ID NO: 157. In certain embodiments, the single domain antibody comprises a heavy chain variable region comprising the amino acid sequence set forth in SEQ ID NO: 161. In certain embodiments, the single domain antibody comprises a heavy chain variable region comprising the amino acid sequence set forth in SEQ ID NO: 165. In certain embodiments, the single domain antibody comprises a heavy chain variable region comprising the amino acid sequence set forth in SEQ ID NO: 169. In certain embodiments, the single domain antibody comprises a heavy chain variable region comprising the amino acid sequence set forth in SEQ ID NO: 173. In certain embodiments, the single domain antibody comprises a heavy chain variable region comprising the amino acid sequence set forth in SEQ ID NO: 177. In certain embodiments, the single domain antibody comprises a heavy chain variable region comprising the amino acid sequence set forth in SEQ ID NO: 181. In certain embodiments, the single domain antibody comprises a heavy chain variable region comprising the amino acid sequence set forth in SEQ ID NO: 185. In certain embodiments, the single domain antibody comprises a heavy chain variable region comprising the amino acid sequence set forth in SEQ ID NO: 189. In certain embodiments, the single domain antibody comprises a heavy chain variable region comprising the amino acid sequence set forth in SEQ ID NO: 193.

In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 1, a heavy chain variable region CDR2 comprising amino acids having the sequence set forth in SEQ ID NO: 2, and a heavy chain variable region CDR3 comprising amino acids having the sequence set forth in SEQ ID NO: 3. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 5, a heavy chain variable region CDR2 comprising amino acids having the sequence set forth in SEQ ID NO: 6, and a heavy chain variable region CDR3 comprising amino acids having the sequence set forth in SEQ ID NO: 7. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 9, a heavy chain variable region CDR2 comprising amino acids having the sequence set forth in SEQ ID NO: 10, and a heavy chain variable region CDR3 comprising amino acids having the sequence set forth in SEQ ID NO: 11. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 13, a heavy chain variable region CDR2 comprising amino acids having the sequence set forth in SEQ ID NO: 14, and a heavy chain variable region CDR3 comprising amino acids having the sequence set forth in SEQ ID NO: 15. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 17, a heavy chain variable region CDR2 comprising amino acids having the sequence set forth in SEQ ID NO: 18, and a heavy chain variable region CDR3 comprising amino acids having the sequence set forth in SEQ ID NO: 19. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 21, a heavy chain variable region CDR2 comprising amino acids having the sequence set forth in SEQ ID NO: 22, and a heavy chain variable region CDR3 comprising amino acids having the sequence set forth in SEQ ID NO: 23. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 25, a heavy chain variable region CDR2 comprising amino acids having the sequence set forth in SEQ ID NO: 26, and a heavy chain variable region CDR3 comprising amino acids having the sequence set forth in SEQ ID NO: 27. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 29, a heavy chain variable region CDR2 comprising amino acids having the sequence set forth in SEQ ID NO: 30, and a heavy chain variable region CDR3 comprising amino acids having the sequence set forth in SEQ ID NO: 31. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 33, a heavy chain variable region CDR2 comprising amino acids having the sequence set forth in SEQ ID NO: 34, and a heavy chain variable region CDR3 comprising amino acids having the sequence set forth in SEQ ID NO: 35. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 37, a heavy chain variable region CDR2 comprising amino acids having the sequence set forth in SEQ ID NO: 38, and a heavy chain variable region CDR3 comprising amino acids having the sequence set forth in SEQ ID NO: 39. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 41, a heavy chain variable region CDR2 comprising amino acids having the sequence set forth in SEQ ID NO: 42, and a heavy chain variable region CDR3 comprising amino acids having the sequence set forth in SEQ ID NO: 43. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 45, a heavy chain variable region CDR2 comprising amino acids having the sequence set forth in SEQ ID NO: 46, and a heavy chain variable region CDR3 comprising amino acids having the sequence set forth in SEQ ID NO: 47. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 49, a heavy chain variable region CDR2 comprising amino acids having the sequence set forth in SEQ ID NO: 50, and a heavy chain variable region CDR3 comprising amino acids having the sequence set forth in SEQ ID NO: 51. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 53, a heavy chain variable region CDR2 comprising amino acids having the sequence set forth in SEQ ID NO: 54, and a heavy chain variable region CDR3 comprising amino acids having the sequence set forth in SEQ ID NO: 55. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 57, a heavy chain variable region CDR2 comprising amino acids having the sequence set forth in SEQ ID NO: 58, and a heavy chain variable region CDR3 comprising amino acids having the sequence set forth in SEQ ID NO: 59. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 61, a heavy chain variable region CDR2 comprising amino acids having the sequence set forth in SEQ ID NO: 62, and a heavy chain variable region CDR3 comprising amino acids having the sequence set forth in SEQ ID NO: 63. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 65, a heavy chain variable region CDR2 comprising amino acids having the sequence set forth in SEQ ID NO: 66, and a heavy chain variable region CDR3 comprising amino acids having the sequence set forth in SEQ ID NO: 67. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 69, a heavy chain variable region CDR2 comprising amino acids having the sequence set forth in SEQ ID NO: 70, and a heavy chain variable region CDR3 comprising amino acids having the sequence set forth in SEQ ID NO: 71. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 73, a heavy chain variable region CDR2 comprising amino acids having the sequence set forth in SEQ ID NO: 74, and a heavy chain variable region CDR3 comprising amino acids having the sequence set forth in SEQ ID NO: 75. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 77, a heavy chain variable region CDR2 comprising amino acids having the sequence set forth in SEQ ID NO: 78, and a heavy chain variable region CDR3 comprising amino acids having the sequence set forth in SEQ ID NO: 79. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 81, a heavy chain variable region CDR2 comprising amino acids having the sequence set forth in SEQ ID NO: 82, and a heavy chain variable region CDR3 comprising amino acids having the sequence set forth in SEQ ID NO: 83.

In certain embodiments, the single domain antibody comprises a heavy chain variable region comprising a heavy chain variable region CDR1 comprising amino acids having the sequence set forth in SEQ ID NO: 1, a heavy chain variable region CDR2 comprising amino acids having the sequence set forth in SEQ ID NO: 2, and a heavy chain variable region CDR3 comprising amino acids having the sequence set forth in SEQ ID NO: 3. In certain embodiments, the single domain antibody comprises a heavy chain variable region comprising a heavy chain variable region CDR1 comprising amino acids having the sequence set forth in SEQ ID NO: 5, a heavy chain variable region CDR2 comprising amino acids having the sequence set forth in SEQ ID NO: 6, and a heavy chain variable region CDR3 comprising amino acids having the sequence set forth in SEQ ID NO: 7. In certain embodiments, the single domain antibody comprises a heavy chain variable region comprising a heavy chain variable region CDR1 comprising amino acids having the sequence set forth in SEQ ID NO: 9, a heavy chain variable region CDR2 comprising amino acids having the sequence set forth in SEQ ID NO: 10, and a heavy chain variable region CDR3 comprising amino acids having the sequence set forth in SEQ ID NO: 11. In certain embodiments, the single domain antibody comprises a heavy chain variable region comprising a heavy chain variable region CDR1 comprising amino acids having the sequence set forth in SEQ ID NO: 13, a heavy chain variable region CDR2 comprising amino acids having the sequence set forth in SEQ ID NO: 14, and a heavy chain variable region CDR3 comprising amino acids having the sequence set forth in SEQ ID NO: 15. In certain embodiments, the single domain antibody comprises a heavy chain variable region comprising a heavy chain variable region CDR1 comprising amino acids having the sequence set forth in SEQ ID NO: 17, a heavy chain variable region CDR2 comprising amino acids having the sequence set forth in SEQ ID NO: 18, and a heavy chain variable region CDR3 comprising amino acids having the sequence set forth in SEQ ID NO: 19. In certain embodiments, the single domain antibody comprises a heavy chain variable region comprising a heavy chain variable region CDR1 comprising amino acids having the sequence set forth in SEQ ID NO: 21, a heavy chain variable region CDR2 comprising amino acids having the sequence set forth in SEQ ID NO: 22, and a heavy chain variable region CDR3 comprising amino acids having the sequence set forth in SEQ ID NO: 23. In certain embodiments, the single domain antibody comprises a heavy chain variable region comprising a heavy chain variable region CDR1 comprising amino acids having the sequence set forth in SEQ ID NO: 25, a heavy chain variable region CDR2 comprising amino acids having the sequence set forth in SEQ ID NO: 26, and a heavy chain variable region CDR3 comprising amino acids having the sequence set forth in SEQ ID NO: 27. In certain embodiments, the single domain antibody comprises a heavy chain variable region comprising a heavy chain variable region CDR1 comprising amino acids having the sequence set forth in SEQ ID NO: 29, a heavy chain variable region CDR2 comprising amino acids having the sequence set forth in SEQ ID NO: 30, and a heavy chain variable region CDR3 comprising amino acids having the sequence set forth in SEQ ID NO: 31. In certain embodiments, the single domain antibody comprises a heavy chain variable region comprising a heavy chain variable region CDR1 comprising amino acids having the sequence set forth in SEQ ID NO: 33, a heavy chain variable region CDR2 comprising amino acids having the sequence set forth in SEQ ID NO: 34, and a heavy chain variable region CDR3 comprising amino acids having the sequence set forth in SEQ ID NO: 35. In certain embodiments, the single domain antibody comprises a heavy chain variable region comprising a heavy chain variable region CDR1 comprising amino acids having the sequence set forth in SEQ ID NO: 37, a heavy chain variable region CDR2 comprising amino acids having the sequence set forth in SEQ ID NO: 38, and a heavy chain variable region CDR3 comprising amino acids having the sequence set forth in SEQ ID NO: 39. In certain embodiments, the single domain antibody comprises a heavy chain variable region comprising a heavy chain variable region CDR1 comprising amino acids having the sequence set forth in SEQ ID NO: 41, a heavy chain variable region CDR2 comprising amino acids having the sequence set forth in SEQ ID NO: 42, and a heavy chain variable region CDR3 comprising amino acids having the sequence set forth in SEQ ID NO: 43. In certain embodiments, the single domain antibody comprises a heavy chain variable region comprising a heavy chain variable region CDR1 comprising amino acids having the sequence set forth in SEQ ID NO: 45, a heavy chain variable region CDR2 comprising amino acids having the sequence set forth in SEQ ID NO: 46, and a heavy chain variable region CDR3 comprising amino acids having the sequence set forth in SEQ ID NO: 47. In certain embodiments, the single domain antibody comprises a heavy chain variable region comprising a heavy chain variable region CDR1 comprising amino acids having the sequence set forth in SEQ ID NO: 49, a heavy chain variable region CDR2 comprising amino acids having the sequence set forth in SEQ ID NO: 50, and a heavy chain variable region CDR3 comprising amino acids having the sequence set forth in SEQ ID NO: 51. In certain embodiments, the single domain antibody comprises a heavy chain variable region comprising a heavy chain variable region CDR1 comprising amino acids having the sequence set forth in SEQ ID NO: 53, a heavy chain variable region CDR2 comprising amino acids having the sequence set forth in SEQ ID NO: 54, and a heavy chain variable region CDR3 comprising amino acids having the sequence set forth in SEQ ID NO: 55. In certain embodiments, the single domain antibody comprises a heavy chain variable region comprising a heavy chain variable region CDR1 comprising amino acids having the sequence set forth in SEQ ID NO: 57, a heavy chain variable region CDR2 comprising amino acids having the sequence set forth in SEQ ID NO: 58, and a heavy chain variable region CDR3 comprising amino acids having the sequence set forth in SEQ ID NO: 59. In certain embodiments, the single domain antibody comprises a heavy chain variable region comprising a heavy chain variable region CDR1 comprising amino acids having the sequence set forth in SEQ ID NO: 61, a heavy chain variable region CDR2 comprising amino acids having the sequence set forth in SEQ ID NO: 62, and a heavy chain variable region CDR3 comprising amino acids having the sequence set forth in SEQ ID NO: 63. In certain embodiments, the single domain antibody comprises a heavy chain variable region comprising a heavy chain variable region CDR1 comprising amino acids having the sequence set forth in SEQ ID NO: 65, a heavy chain variable region CDR2 comprising amino acids having the sequence set forth in SEQ ID NO: 66, and a heavy chain variable region CDR3 comprising amino acids having the sequence set forth in SEQ ID NO: 67. In certain embodiments, the single domain antibody comprises a heavy chain variable region comprising a heavy chain variable region CDR1 comprising amino acids having the sequence set forth in SEQ ID NO: 69, a heavy chain variable region CDR2 comprising amino acids having the sequence set forth in SEQ ID NO: 70, and a heavy chain variable region CDR3 comprising amino acids having the sequence set forth in SEQ ID NO: 71. In certain embodiments, the single domain antibody comprises a heavy chain variable region comprising a heavy chain variable region CDR1 comprising amino acids having the sequence set forth in SEQ ID NO: 73, a heavy chain variable region CDR2 comprising amino acids having the sequence set forth in SEQ ID NO: 74, and a heavy chain variable region CDR3 comprising amino acids having the sequence set forth in SEQ ID NO: 75. In certain embodiments, the single domain antibody comprises a heavy chain variable region comprising a heavy chain variable region CDR1 comprising amino acids having the sequence set forth in SEQ ID NO: 77, a heavy chain variable region CDR2 comprising amino acids having the sequence set forth in SEQ ID NO: 78, and a heavy chain variable region CDR3 comprising amino acids having the sequence set forth in SEQ ID NO: 79. In certain embodiments, the single domain antibody comprises a heavy chain variable region comprising a heavy chain variable region CDR1 comprising amino acids having the sequence set forth in SEQ ID NO: 81, a heavy chain variable region CDR2 comprising amino acids having the sequence set forth in SEQ ID NO: 82, and a heavy chain variable region CDR3 comprising amino acids having the sequence set forth in SEQ ID NO: 83.

In certain embodiments, the single domain antibody comprises a heavy chain variable region comprising an amino acid sequence having at least about 90% sequence identity to an amino acid sequence selected from the group consisting of SEQ ID NOs: 4, 8, 12, 16, 20, 24, 28, 32, 36, 40, 44, 48, 52, 56, 60, 64, 68, 72, 76, 80 and 84. In certain embodiments, the single domain antibody comprises a heavy chain variable region comprising an amino acid sequence selected from the group consisting of SEQ ID NOs: 4, 8, 12, 16, 20, 24, 28, 32, 36, 40, 44, 48, 52, 56, 60, 64, 68, 72, 76, 80 and 84.

In certain embodiments, the single domain antibody comprises a humanized framework.

In certain embodiments, the anti-TIGIT antibody further comprises a Fc region. In certain embodiments, the Fc region comprises a human Fc region. In certain embodiments, the Fc region comprises a Fc region selected from the group consisting of the Fc regions of IgG, IgA, IgD, IgE and IgM. In certain embodiments, the Fc region comprises a Fc region selected from the group consisting of the Fc regions of IgG1, IgG2, IgG3 and IgG4. In certain embodiments, the Fc region comprises an IgG1 Fc region. In certain embodiments, the IgG1 Fc region comprising one or more mutation that enhances an antibody-dependent cell-mediated cytotoxicity (ADCC). In certain embodiments, the IgG1 Fc region comprises the mutations of L235V, F243L, R292P, Y300L and P396L. In certain embodiments, the IgG1 Fc region comprises the mutations of S239D, A330L and I332E. In certain embodiments, the anti-TIGIT antibody comprises the amino acid sequence set forth in SEQ ID NO: 194

In certain embodiments, the heavy chain variable region is linked to a Fc region via a linker. In certain embodiments, the linker is a peptide linker. In certain embodiments, the peptide linker comprises about four to about thirty amino acids. In certain embodiments, the peptide linker comprises about four to about fifteen amino acids. In certain embodiments, the peptide linker comprise an amino acid sequence selected from the group consisting of SEQ ID NOs: 195-220.

In certain embodiments, the anti-TIGIT antibody comprises a multispecific antibody, e.g., a bispecific antibody, a full-length immunoglobulin, a single-chain Fv (scFv) fragment, a Fab fragment, a Fab' fragment, a F(ab')2, an Fv fragment, a disulfide stabilized Fv fragment (dsFv), a (dsFv)2, a VHH, a Fv-Fc fusion, a scFv-Fc fusion, a scFv-Fv fusion, a diabody, a tribody, a tetrabody or any combination thereof.

In certain embodiments, the anti-TIGIT antibody comprises a multispecific antibody, e.g., a bispecific antibody, which comprises a second antibody moiety that specifically binds to a second antigen. In certain embodiments, the second antigen is a tumor associated antigen. In certain embodiments, the tumor associated antigen is selected from the group consisting of Her-2, EGFR, PD-L1, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1) and any combination thereof.

In certain embodiments, the second antigen is an immune checkpoint regulator. In certain embodiments, the immune checkpoint regulator is selected from the group consisting of PD1, CTLA4, LAG-3, 2B4, BTLA and any combination thereof.

In certain embodiments, the anti-TIGIT antibody is conjugated to a therapeutic agent or a label. In certain embodiments, the label is selected from the group consisting of a radioisotope, a fluorescent dye and an enzyme.

The present disclosure further provides immunoconjugates comprising any antibodies disclosed herein, linked to a therapeutic agent. In certain embodiments, the therapeutic agent is a cytotoxin. In certain embodiments, the therapeutic agent is a radioactive isotope.

The present disclosure further provides pharmaceutical compositions. In certain embodiments, the pharmaceutical composition comprises a) an antibody or a immunoconjugate disclosed herein, and b) a pharmaceutically acceptable carrier.

The present disclosure further provides nucleic acids encoding any antibodies disclosed herein. The present disclosure further provides vectors comprising any nucleic acid disclosed herein. The present disclosure further provides host cells comprising a nucleic acid or a vector disclosed herein.

The present disclosure further provides methods for preparing an antibody disclosed herein. In certain embodiments, the method comprises expressing an antibody in a host cell disclosed herein and isolating the antibody from the host cell.

The present disclosure further provides methods of reducing tumor burden in a subject. In certain embodiments, the method comprises administering to the subject an effective amount of an antibody, an immunoconjugate, or a pharmaceutical composition disclosed herein.

In certain embodiments, the method reduces the number of tumor cells. In certain embodiments, the method reduces tumor size. In certain embodiments, the method eradicates the tumor in the subject. In certain embodiments, the tumor is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer and a combination thereof.

The present disclosure further provides methods of treating and/or preventing a neoplasm in a subject. In certain embodiments, the method comprises administering to the subject an effective amount of an antibody, an immunoconjugate, or a pharmaceutical composition disclosed herein.

The present disclosure further provides methods of lengthening survival of a subject having a neoplasm. In certain embodiments, the method comprises administering to the subject an effective amount of an antibody, an immunoconjugate, or a pharmaceutical composition disclosed herein.

In certain embodiments, the neoplasm is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer and a combination thereof.

The present disclosure further provides any antibodies disclosed herein for use as a medicament. The present disclosure further provides any antibodies disclosed herein for use in treating cancer. The present disclosure further provides pharmaceutical compositions disclosed herein for use as a medicament. The present disclosure further provides pharmaceutical compositions disclosed herein for use in treating cancer. In certain embodiments, the cancer is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer and a combination thereof.

The present disclosure further provides kits comprising an antibody, an immunoconjugate, a pharmaceutical composition, a nucleic acid, a vector or a host cell disclosed herein. In certain embodiments, the kit comprise a written instruction for treating and/or preventing a neoplasm.

### BRIEF DESCRIPTION OF THE FIGURES

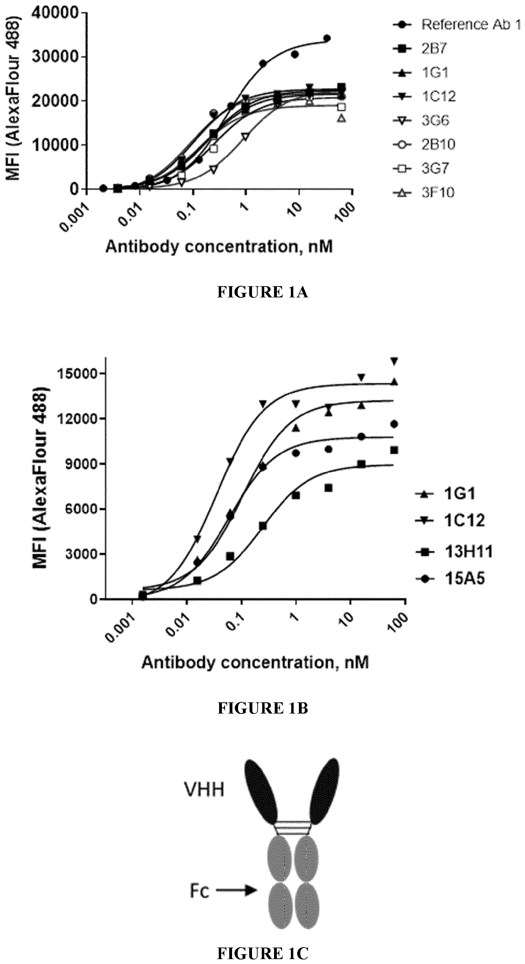

Figures 1A-1C depict whole cell binding of representative VHH bivalent antibodies to human TIGIT determined by flowcytometry assays. Figure 1A shows one assay with comparison to a reference anti-human-TIGIT antibody (Reference Ab 1). Figure 1B shows a different assay with additional VHH antibodies. Y axis represents Mean Fluorescence Intensity of AlexaFlour 488. X axis represents antibody concentrations in nanomolar. 2B7, 1G1, 1C12, 3G6, 2B10, 3G7, 3G10, 13H11, and 15A5 are VHH clones of anti-human TIGIT. EC50 values were obtained using non-liner regression method by Prism, and values are shown in the table as nanomolar. Figure 1C is a schematic of the structure of a TIGIT VHH bivalent antibody.

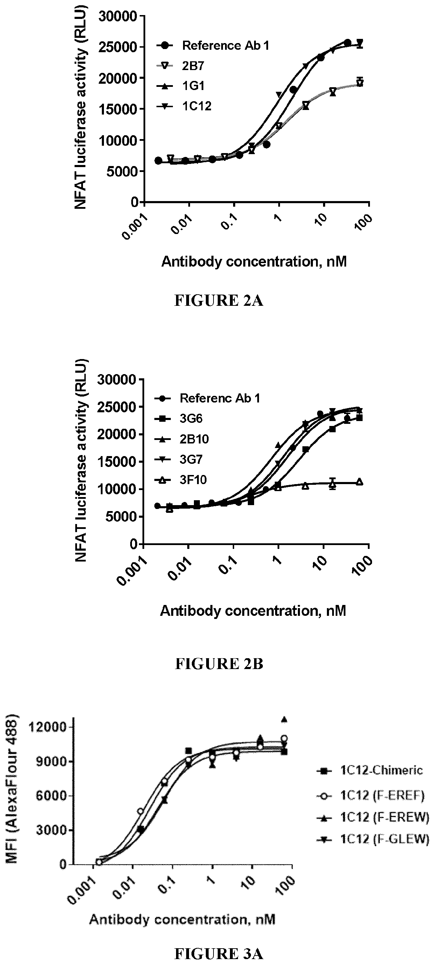

Figure 2A-2B depict the potency of representative bivalent antibodies in blocking TIGIT activity determined by luciferase reporter assays. Human TIGIT and NFAT reporter gene stably transfected Jurkat cells were co-cultured with PVR (CD155) stably transfected Raji cells in the presence of representative anti-TIGIT bivalent antibodies and a low concentration of staphylococcal enterotoxin. Y axis represents NFAT luciferase activity in relative luminescence units. X axis represents antibody concentration in nanomolar. Reference Ab 1is a reference anti-h-TIGIT antibody. 2B7, 1G1, 1C12, 3G6, 2B10, 3G7 and 3F10 are representative clones of anti-h-TIGIT. EC50 values were obtained using non-liner regression method by Prism, and values are shown in the table as nanomolar.

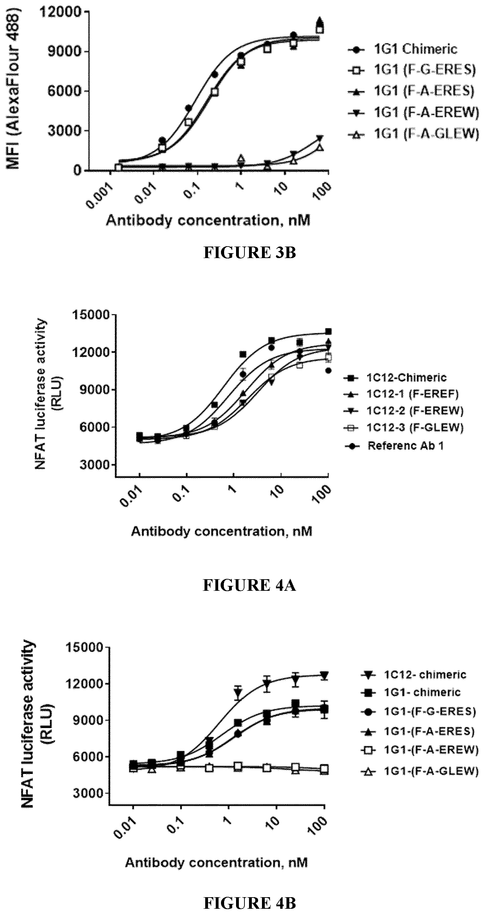

Figures 3A and 3B depict whole cell binding of humanized 1C12 and 1G1 clones to human TIGIT determined by flowcytometry assays. A representative result of 1C12 humanized versions is shown in Figure 3A. 1C12-chimeric is an antibody with Llama VHH sequence of 1C12 clone and human IgG1 CH2 and CH3 domains, 1C12(F-EREF), 1C12 (F-EREW) and 1C12(F-GLEW) are humanized versions of 1C12 clone with difference of mutations in Framework 2. A representative result of 1G1 humanized versions is shown in Figure 3B. 1G1-chimeric is an antibody with Llama VHH sequence of 1G1 clone and human IgG1 CH2 and CH3 domains. 1G1 (F-G-ERES), 1G1 (F-A-ERES), 1G1 (F-A-EREW) and 1G1 (F-A-GLEW) are four different versions of humanized 1G1 clones with different mutations in framework 2. Y axis is values of Mean Fluorescence Intensity of AlexaFlour 488. X axis is values of antibody concentrations in nanomolar.
Figures 4A and 4B depict the potency of humanized 1C12 and 1G1 clones in blocking TIGIT activity determined by luciferase reporter assays. Representative results of 1C12 humanized versions in TIGIT blockade luciferase reporter assay is shown in Figure 4A. All clones are more potent compared to Reference Ab 1, a reference anti-h-TIGIT antibody. Representative results of 1G1 humanized versions are shown in Figure 4B. Y axis represents NFAT luciferase activity (RLU, relative luminescence unite). X axis represents antibody concentrations in nanomolar.

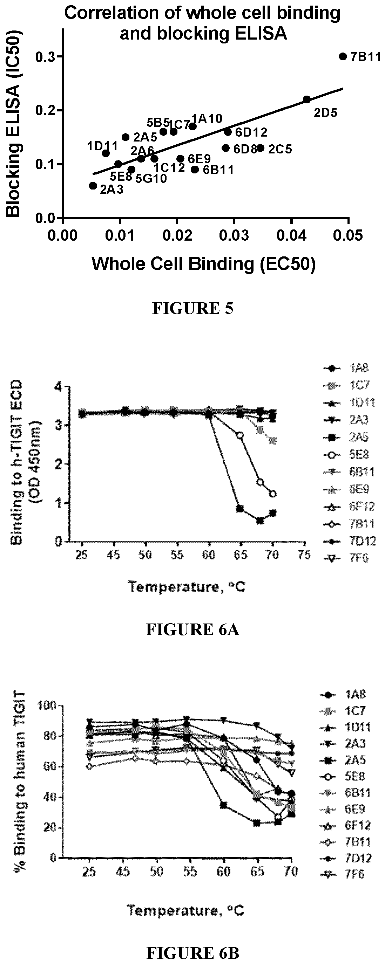

Figure 5 depicts correlations betweenIC50 values (in nanomolar) of blocking ELISA and EC50 values (in nanomolar) of whole cell binding for representative clones. The clone names are labeled in the figures. Correlations were analyzed using GraphPad Prism.
Figures 6A and 6B depict thermostability tested for representative clones. The VHH antibody samples were heated from 25 to 70 ° C for 60 min. Binding of heated samples to human TIGIT was examined using ELISA or whole cell binding. Figure 6A shows binding of heated samples of representative clones to h-TIGIT ECD in an ELISA assay with clone names labeled in the figure legends. Y axis represents OD450 obtained from the ELISA assay. X axis represents temperature for the treatment. Figure 6B shows binding of heated samples of representative clones to h-TIGIT stably expressed in Jurkat cells determined by flowcytometry. Y axis represents percentage of binding to h-TIGIT. X axis represents temperature for the treatment.

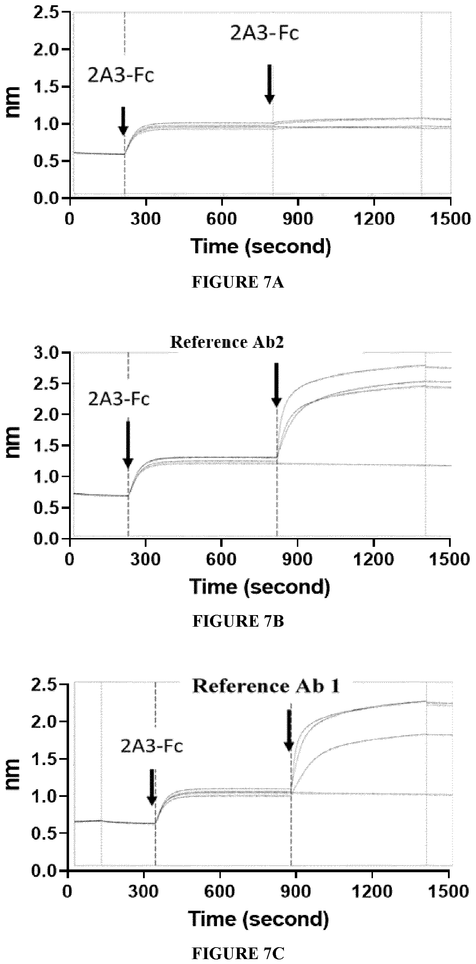

Figures 7A-7C depict the epitope at which 2A3-Fc binds to human TIGIT determined by Octet binding assay using ForteBio. Recombinant protein of his-tagged human TIGIT ECD (200 nM) was loaded on sensor. Binding was detected by injection of 2A3-Fc at three different concentrations. No additional binding was detected by second injection of 2A3-Fc shown in Figure 7A, however strong binding was detected by injection of three different concentrations of either Reference Ab 2, a reference anti-human-TIGIT antibody, shown in Figure 7B, or Reference Ab 1 shown in Figure 7C, two reference anti-h-TIGIT antibodies. The results indicate that 2A3 clone has a different binding epitope compared to Reference Ab 2and Reference Ab 1.

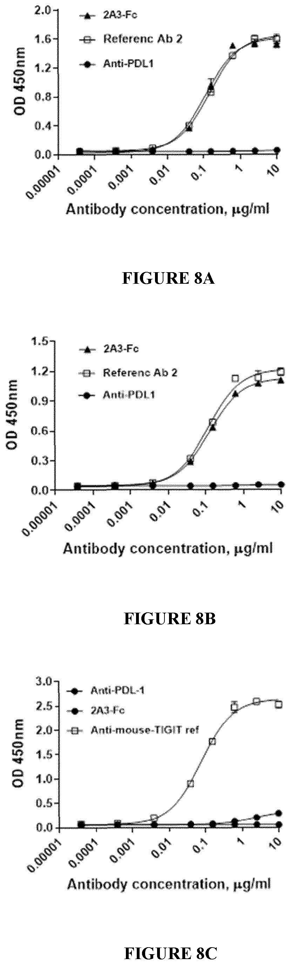

Figures 8A-8C depict cross-binding activity of 2A3-Fc to human-, cyno- and mouse-TIGIT determined by ELISA assays. Figure 8A shows binding of 2A3-Fc to recombinant human TIGIT ECD. Reference Ab 2 is an anti-h-TIGIT reference antibody. Both 2A3-Fc and Reference Ab 2 bind to h-TIGIT with similar affinity. Anti-PDL1 did not bind to h-TIGIT. Figure 8B shows binding of 2A3-Fc to recombinant cyno-TIGIT. Both 2A3-Fc and Reference Ab 2 bind to cyno-TIGIT with similar affinity. Anti-PDL1 did not bind to cyno-TIGIT. Figure 8C shows binding of 2A3-Fc to recombinant mouse-TIGIT. Neither 2A3-Fc nor Reference Ab 2 binds to mouse-TIGIT, however an anti-mouse TIGIT reference antibody (Biolegend #142101) bind to mouse-TIGIT with a high affinity. Y axis represents OD450, X axis represents antibody concentrations in µg/ml.

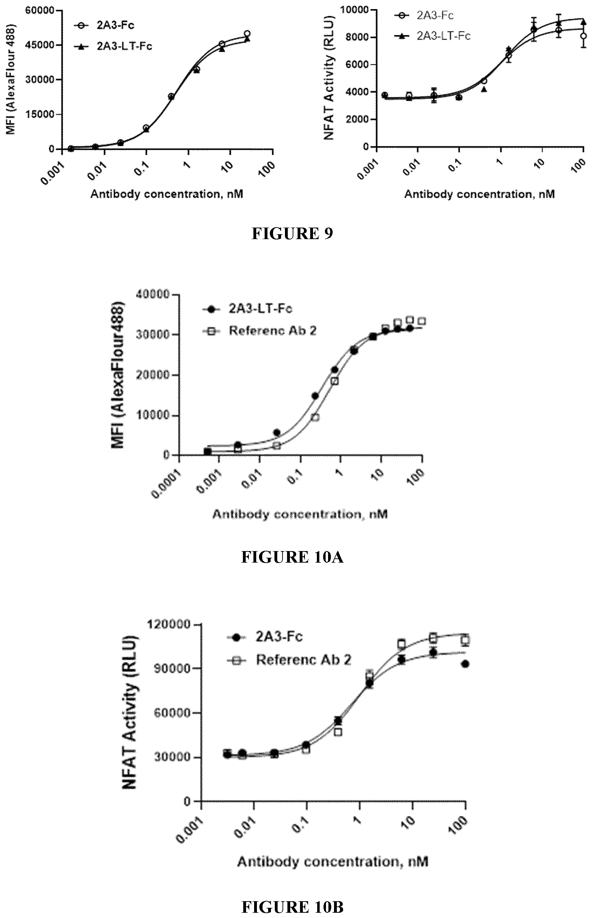

Figure 9 depicts comparison of potency of representative clone 2A3-Fc and hotspot corrected version of 2A3-LT-Fc in whole cell binding assay and human TIGIT blockade reporter assay. Y axis represents mean fluorescent intensity (MFI) in the upper panel and NFAT luciferase reporter activity in relative luminescence unite in the lower panel. X axis represents antibody concentrations in nanomolar. Hotspot corrected version 2A3-LT-Fc had similar potency compared to the parental clone 2A3-Fc.
Figures 10A and 10B depict comparison of affinity of hotspot corrected version 2A3-LT-Fc to a reference anti-h-TIGIT antibody, Reference Ab 2. Figure 10A shows the comparison of potency of hotspot corrected version 2A3-LT-Fc to Reference Ab 2 in whole cell binding. Y axis represents mean fluorescence intensity of AlexaFlour 488 determined by flowcytometry assay using CytoFlex. X axis represents antibody concentrations in nanomolar. Data shown is a representative result from three different experiments. 2A3-LT-Fc had slightly higher affinity to h-TIGIT than Reference Ab 2. Figure 10B shows the comparison of potency of hotspot corrected version 2A3-LT-Fc to Reference Ab 2 in TIGIT blockade NFAT reporter assay. Y axis represents NFAT luciferase reporter activity in relative luminescence units. X axis represents antibody concentrations in nanomolar. 2A3-LT-Fc had similar potency to that of Reference Ab 2.

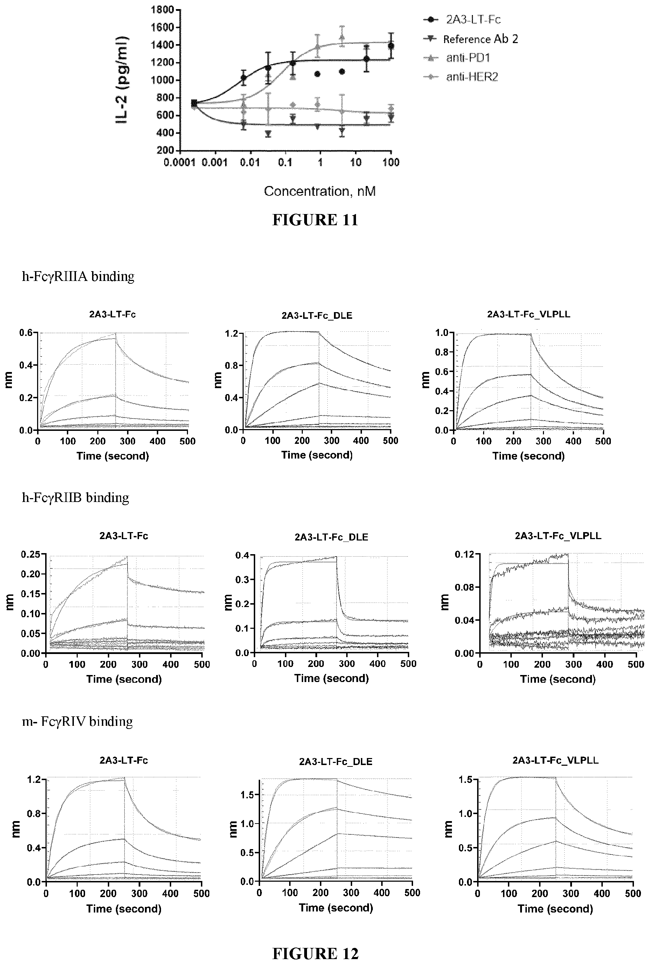

Figure 11 depicts in vitro antitumor efficacy of 2A3-LT-Fc. Mixed lymphocyte reaction assay (MLR) was performed using TIGIT+ T cells and PVR+ dendritic cells (DCs). IL-2 secretion from the T cells were measured in culture supernatant after 48 hours of coculture. An anti-PD1 antibody was used as a positive control. An anti-HER2 antibody was used as a negative control.
Figure 12 depicts binding of 2A3-LT-Fc wild type (wt) and Fc mutations (DLE and VLPLL) to human Fc γ RIIIA, human Fc γ RUB and mouse Fc γ RIV determined using Octet binding assay. Sensors were loaded with recombinant proteins of ECDs of Fc γ RIIIA, human Fc γ RIIB and mouse Fc γ RIV, association and dissociation of 2A3-LT-Fc wt, DLE or VLPLL mutants at 5 different concentrations were detected using ForteBio. Y axis represents association, dissociation & Rmax. X axis represents time in second. Both DLE and VLPLL mutants had enhanced binding affinity to human Fc γ RIIIA compared to that of wild type, DLE mutant also had enhanced binding affinity to human Fc γ RIIB, however VLPLL mutant reduced binding affinity to human Fc γ RIIB. All versions had similar affinity to mouse Fc γ RIV.

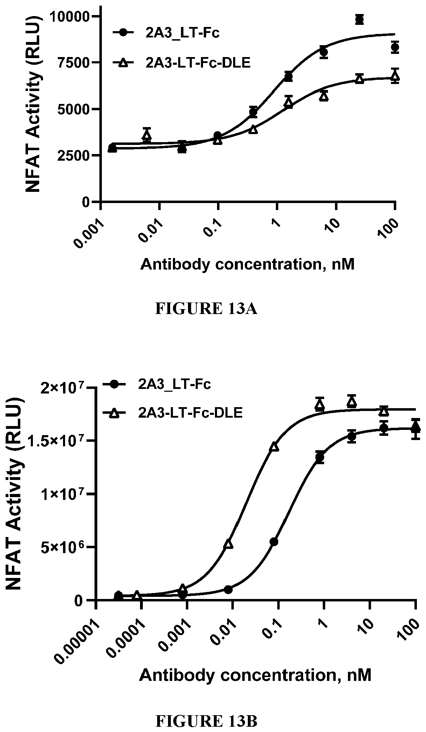

Figures 13A and 13B depict effects of TIGIT mAbs 2A3-LT-Fc wt, and DLE mutant on blocking TIGIT activity and human Fc γ RIIIA-mediated activity, respectively. Figure 13A depicts the effects of TIGIT mAbs 2A3-LT-Fc wt, and DLE mutant on blocking TIGIT activity determined using NFAT luciferase reporter assay. Human TIGIT and NFAT reporter gene stably transfected Jurkat cells were co-cultured with PVR stably transfected Raji cells. 2A3-LT-Fc wt and mutants were added and cultured for 5 hours. The TCR-mediated activity was measured by luciferase activity. Y axis represents NFAT luciferase activity in relative luminescence units. X axis represents antibody concentrations in nanomolar. Figure 13B depicts the effects of TIGIT mAbs 2A3-LT-Fc wt, and DLE mutant on human Fc γ RIIIA-mediated activity determined by Fc γ RIIIA-mediated NFAT luciferase reporter activity. Human Fc γ RIIIA and NFAT stably transfected Jurkat cells were co-cultured with human TIGIT stably transfected 293T cells in the presence of different concentrations of 2A3-LT-Fc wt, and DLE mutant for 5 hours. Luciferase activity was measured and is presented in Y axis as relative luminescence units. X axis represents antibody concentrations in nanomolar.

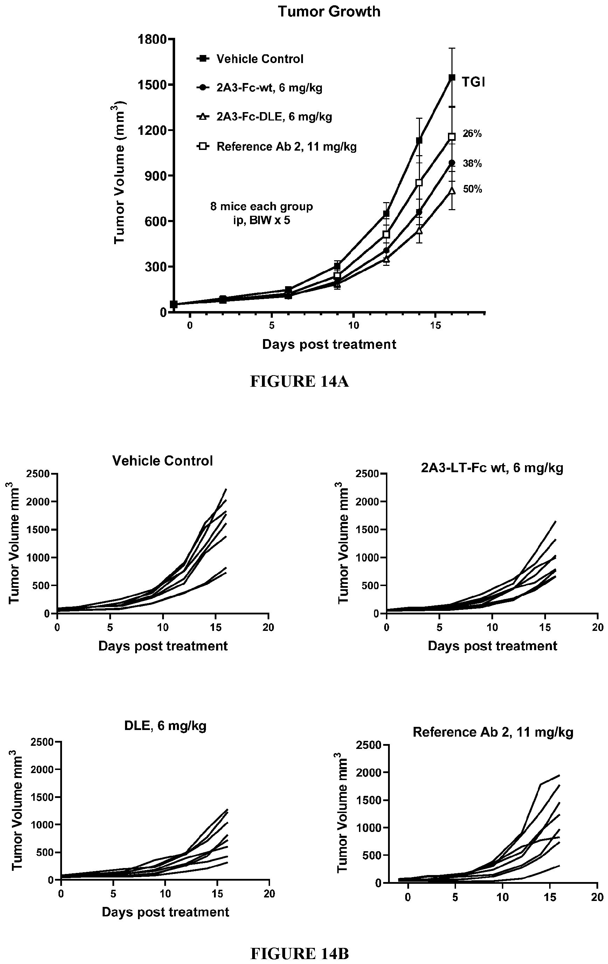

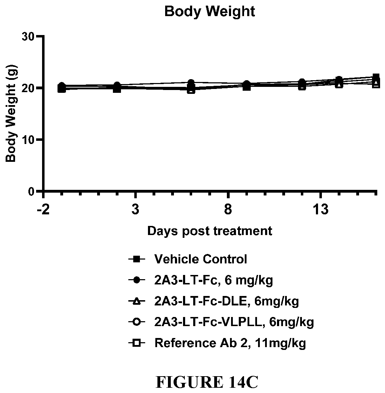

Figures 14A-14C depict in vivo efficacy analyses of the anti-TIGIT antibodies. H-TIGIT Knock-In C57BL/6 mice and MC38 murine colon cancer model were used. MC38 tumor cells were implanted one week before treatment. Treatment started when tumor volume reached about 51 mm³, when drugs were dosed intraperitoneally twice a week for 2.5 weeks at 6 mg/kg for 2A3-LT-Fc antibodies, or 11 mg/kg for reference antibody (equal to 6 mg/kg of 2A3-LT-Fc in mole/kg dosage). Figure 14A depicts tumor growth curves of tumor bearing mice treated with vehicle control, 2A3-Fc wildtype, 2A3-Fc with DLE mutations and Reference Ab 2. The results of individual tumor volume are shown in Figure 14B. Figure 14C shows that body weight changes were not significant between the groups throughout the study.

### DETAILED DESCRIPTION

The present disclosure provides isolated monoclonal antibodies that bind specifically to TIGIT with high affinity, including multispecific antibodies that binds to TIGIT and one or more additional target. In certain embodiments, the anti-TIGIT antibody comprises a single domain antibody that binds to TIGIT. This disclosure further provides methods of making and using the antibodies, immunoconjugates and pharmaceutical compositions comprising the antibodies, e.g., for treating diseases and disorders, e.g., cancer. The invention is based, in part, on the discovery of single domain anti-TIGIT antibodies that bind to TIGIT, which can increase an immune response in immune cells and provide improved anti-tumor efficacy.

For clarity and not by way of limitation the detailed description of the presently disclosed subject matter is divided into the following subsections:
1. Definitions;
2. Antibodies;
3. Methods of use;
4. Pharmaceutical formulations; and
5. Articles of manufacture.

### 1. DEFINITIONS

The term "antibody" herein is used in the broadest sense and encompasses various antibody structures, including but not limited to monoclonal antibodies, polyclonal antibodies, multispecific antibodies (e.g., bispecific antibodies), single domain antibody and antibody fragments so long as they exhibit the desired antigen-binding activity.

An "antibody fragment" refers to a molecule that comprises an antigen binding portion of an intact full-length antibody that binds to the antigen to which the intact antibody binds. Examples of antibody fragments include but are not limited to Fv, Fab, Fab', Fab'-SH, F(ab')2, diabodies, linear antibodies, single-chain antibody molecules (e.g., scFv), multispecific antibodies formed from antibody fragments, a single domain antibody, a VHH a nanobody, a domain antibody, a bivalent domain antibody, or any other fragment of an antibody that binds to an antigen. A "VHH" refers to a single domain antibody isolated from a camelid animal. In certain embodiments, a VHH comprises a variable region of a heavy chain of a camelid heavy chain antibody. In certain embodiments, a VHH has a size of no more than about 25 kDa. In certain embodiments, a VHH has a size of no more than about 20 kDa. In certain embodiments, a VHH has a size of no more than about 15 kDa.

A "full-length antibody" refers to an antibody comprising two heavy chains and two light chains. The variable regions of the light and heavy chains are responsible for antigen binding. The variable regions of a heavy chain and a light chain may be referred to as "VH" and "VL", respectively. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by well-known conventions, e.g., the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more conserved than the CDRs and form a scaffold to support the hypervariable loops. The constant regions of the heavy and light chains are not involved in antigen binding but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as lgG1 (γ1 heavy chain), lgG2 (γ2 heavy chain), lgG3 (y3 heavy chain), lgG4 (γ4 heavy chain), IgA1 (α1 heavy chain), or lgA2 (α2 heavy chain).

An "antibody that cross-competes for binding" with a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is described in Antibodies, Harlow and Lane (Cold Spring Harbor Press, Cold Spring Harbor, NY).

"Fv" is a minimum antibody fragment which contains a complete antigen-recognition and -binding site. This fragment consists of a dimer of one heavy- and one light-chain variable region in tight, non-covalent association. From the folding of these two domains emanate six hypervariable loops (3 loops in each of the heavy and light chains) that contribute the amino acid residues to antigen binding and confer antigen binding specificity to the antibody. However, even a single variable domain (or half of an Fv comprising only three CDRs specific for an antigen) can recognize and bind to an antigen, although sometimes at a lower affinity than the entire binding site.

"Single-chain Fv," also abbreviated as "sFv" or "scFv," are antibody fragments that comprise the V_{H} and V_{L} antibody domains connected into a single polypeptide chain. In some embodiments, the scFv polypeptide further comprises a polypeptide linker between the V_{H} and V_{L} domains which enables the scFv to form the desired structure for antigen binding. For a review of scFv, see Plückthun in The Pharmacology of Monoclonal Antibodies, vol. 113, Rosenburg and Moore eds., Springer-Verlag, New York, pp. 269-315 (1994).

An "acceptor human framework" or "human framework" for the purposes herein is a framework comprising the amino acid sequence of a light chain variable domain (VL) framework or a heavy chain variable domain (VH) framework derived from a human immunoglobulin framework or a human consensus framework. An acceptor human framework "derived from" a human immunoglobulin framework or a human consensus framework may comprise the same amino acid sequence thereof, or it may contain amino acid sequence changes. In certain embodiments, the number of amino acid changes are 10 or less, 9 or less, 8 or less, 7 or less, 6 or less, 5 or less, 4 or less, 3 or less, or 2 or less. In certain embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (KD). Affinity can be measured by common methods known in the art, including those described herein. Specific illustrative and exemplary embodiments for measuring binding affinity are described in the following.

An "affinity matured" antibody refers to an antibody with one or more alterations in one or more CDRs or hypervariable regions (HVRs), compared to a parent antibody which does not possess such alterations, which alterations provide improved affinity of the antibody for antigen.

"T cell immunoreceptor with Ig and ITIM domains" or "TIGIT" as used herein, refers to any native TIGIT polypeptide from any vertebrate source, including mammals such as primates (e.g., humans and cynomolgus monkeys), or any fragment thereof, and may optionally comprise up to one, up to two, up to three, up to four, up to five, up to six, up to seven, up to eight, up to nine or up to ten amino acid substitutions, additions and/or deletions. The term encompasses full-length, unprocessed TIGIT as well as any form of TIGIT that results from processing in the cell. The term also encompasses naturally occurring variants of TIGIT, e.g., splice variants or allelic variants. A non-limiting example of a human TIGIT amino acid sequence targeted by an anti-TIGIT antibody of the present disclosure is as follows:

1 MRWCLLLIWA QGLRQAPLAS GMMTGTIETT GNISAEKGGS IILQCHLSST TAQVTQVNWE

61 QQDQLLAICN ADLGWHISPS FKDRVAPGPG LGLTLQSLTV NDTGEYFCIY HTYPDGTYTG

121 RIFLEVLESS VAEHGARFQI PLLGAMAATL VVICTAVIVV VALTRKKKAL RIHSVEGDLR

181 RKSAGQEEWS PSAPSPPGSC VQAEAAPAGL CGEQRGEDCA ELHDYFNVLS YRSLGNCSFF

241 TETG [SEQ ID NO: 221].

The term "ECD of TIGIT" refers to the extracellular domain of TIGIT. For example, the ECD of the exemplary TIGIT protein shown in SEQ ID NO: 221 comprises the following amino acid sequence:

MRWCLLLIWA QGLRQAPLAS GMMTGTIETT GNISAEKGGS IILQCHLSST TAQVTQVNWE
QQDQLLAICN ADLGWHISPS FKDRVAPGPG LGLTLQSLTV NDTGEYFCIY HTYPDGTYTG
RIFLEVLESS VAEHGARF [SEQ ID NO: 222].

The terms "anti-TIGIT antibody" and "an antibody that binds to TIGIT" refer to an antibody that is capable of binding to TIGIT with sufficient affinity such that the antibody is useful as a diagnostic and/or therapeutic agent for targeting TIGIT. In one embodiment, the extent of binding of an anti-TIGIT antibody to an unrelated, non-TIGIT protein is less than about 10% of the binding of the antibody to TIGIT as measured, e.g., by a BIACORE^{®} surface plasmon resonance assay. In certain embodiments, an antibody that binds to TIGIT has a dissociation constant (KD) of < about 1 µM, < about 100 nM, < about 10 nM, < about 1 nM, < about 0.1 nM, < about 0.01 nM, or < about 0.001 nM (e.g., 10⁻⁸ M or less, e.g., from 10⁻⁸ M to 10⁻¹² M, e.g., from 10⁻⁹ M to 10⁻¹⁰ M). In certain embodiments, an anti-TIGIT antibody binds to an epitope of TIGIT that is conserved among TIGIT from different species. In certain embodiments, an anti-TIGIT antibody binds to an epitope on TIGIT that is in the ECD of the protein.

The term "chimeric" antibody refers to an antibody in which a portion of the heavy and/or light chain is derived from a particular source or species, while the remainder of the heavy and/or light chain is derived from a different source or species. In certain embodiments, a chimeric antibody disclosed herein comprises a camelid heavy chain variable region and a human Fc region.

As used herein, the term "CDR" or "complementarity determining region" is intended to mean the non-contiguous antigen combining sites within the variable region of a heavy chain and/or a light chain. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of any one of the definitions to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**Table 1: CDR definitions**

| | **Kabat¹** | | **Chothia²** | | **MacCallum³** | | **IMGT⁴** | **AHo⁵** |
|---|---|---|---|---|---|---|---|---|
| V_{H} CDR1 | | 31-35 | | 26-32 | | 30-35 | 27-38 | 25-40 |
| V_{H} CDR2 | | 50-65 | | 53-55 | | 47-58 | 56-65 | 58-77 |
| V_{H} CDR3 | | 95-102 | | 96-101 | | 93-101 | 105-117 | 109-137 |
| V_{L} CDR1 | | 24-34 | | 26-32 | | 30-36 | 27-38 | 25-40 |
| V_{L} CDR2 | | 50-56 | | 50-52 | | 46-55 | 56-65 | 58-77 |
| V_{L} CDR3 | | 89-97 | | 91-96 | | 89-96 | 105-117 | 109-137 |

| | | | | | | | | |
|---|---|---|---|---|---|---|---|---|
| ¹Residue numbering follows the nomenclature of Kabat et al., supra. ²Residue numbering follows the nomenclature of Chothia et al., supra. ³Residue numbering follows the nomenclature of MacCallum et al., supra. ⁴Residue numbering follows the nomenclature of Lefranc et al., supra. ⁵Residue numbering follows the nomenclature of Honegger and Plückthun, supra. | | | | | | | | |

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat et al., supra. Using this numbering system, the actual linear amino acid sequence may contain fewer or additional amino acids corresponding to a shortening of, or insertion into, a FR or CDR of the variable domain. For example, a heavy-chain variable domain may include a single amino acid insert (residue 52a according to Kabat) after residue 52 of H2 and inserted residues (e.g. residues 82a, 82b, and 82c, etc. according to Kabat) after heavy-chain FR residue 82. The Kabat numbering of residues may be determined for a given antibody by alignment at regions of homology of the sequence of the antibody with a "standard" Kabat numbered sequence.

In certain embodiments, the amino acid residues which encompass the CDRs of a single domain antibody (e.g., a single domain anti-TIGIT antibody disclosed herein) is defined according to the IMGT nomenclature in Lefranc et al., supra. In certain embodiments, the amino acid residues which encompass the CDRs of a full-length antibody is defined according to the Kabat nomenclature in Kabat et al., supra. In certain embodiments, the numbering of the residues in an immunoglobulin heavy chain, e.g., in an Fc region, is that of the EU index as in Kabat et al., supra. The "EU index as in Kabat" refers to the residue numbering of the human IgG1 EU antibody.

"Framework" or "FR" refers to residues are those variable-domain residues other than the CDR residues as herein defined.

A "humanized" antibody refers to a chimeric antibody comprising amino acid residues from non-human CDRs/HVRs and amino acid residues from human FRs. In certain embodiments, a humanized antibody will comprise substantially all of at least one, and typically two, variable domains, in which all or substantially all of the HVRs/CDRs correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE^{™} technology). See also, for example, Li et al., Proc. Natl. Acad. Sci. USA, 103:3557-3562 (2006) regarding human antibodies generated via a human B-cell hybridoma technology.

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5):1792-1797, 2004; Edgar, R.C., BMC Bioinformatics 5(1):113, 2004).

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

The term "constant domain" refers to the portion of an immunoglobulin molecule having a more conserved amino acid sequence relative to the other portion of the immunoglobulin, the variable domain, which contains the antigen-binding site. The constant domain contains the C_{H}1, C_{H}2 and C_{H}3 domains (collectively, C_{H}) of the heavy chain and the C_{L} domain of the light chain.

The "light chains" of antibodies (e.g., immunoglobulins) from any mammalian species can be assigned to one of two clearly distinct types, called kappa ("κ") and lambda ("λ"), based on the amino acid sequences of their constant domains.

The "CH1 domain" (also referred to as "C1" of "H1" domain) usually extends from about amino acid 118 to about amino acid 215 (EU numbering system).

"Hinge region" is generally defined as a region in IgG corresponding to Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "CH2 domain" of a human IgG Fc region (also referred to as "C2" domain) usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec Immunol. 22:161-206 (1985).

The "CH3 domain" (also referred to as "C2" domain) comprises the residues between a CH2 domain and the C-terminal of an Fc region (i.e. from about amino acid residue 341 to the C-terminal end of an antibody sequence, typically at amino acid residue 446 or 447 of an IgG).

The term "Fc region" or "fragment crystallizable region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies described herein include human IgG1, IgG2 (IgG2A, IgG2B), IgG3 and IgG4.

"Fc receptor" or "FcR" describes a receptor that binds the Fc region of an antibody. The preferred FcR is a native human FcR. Moreover, a preferred FcR is one which binds an IgG antibody (a gamma receptor) and includes receptors of the FcyRI, FcyRII, and FcyRIII subclasses, including allelic variants and alternatively spliced forms of these receptors, FcyRII receptors include FcγRIIA (an "activating receptor") and FcγRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibitory receptor FcγRIIB contains an immunoreceptor tyrosine-based inhibition motif (ITIM) in its cytoplasmic domain. (See M. Daëron, Annu. Rev. Immunol. 15:203-234 (1997). FcRs are reviewed in Ravetch and Kinet, Annu. Rev. Immunol. 9: 457-92 (1991); Capel et al., Immunomethods 4: 25-34 (1994); and de Haas et al., J. Lab. Clin. Med. 126: 330-41 (1995). Other FcRs, including those to be identified in the future, are encompassed by the term "FcR" herein.

The term "epitope" as used herein refers to the specific group of atoms or amino acids on an antigen to which an antibody or antigen-binding moiety binds. Two antibodies or antigen-binding moieties may bind the same epitope within an antigen if they exhibit competitive binding for the antigen.

As use herein, the terms "specifically binds," "specifically recognizing," and "is specific for" refer to measurable and reproducible interactions, such as binding between a target and an antibody or antibody moiety, which is determinative of the presence of the target in the presence of a heterogeneous population of molecules, including biological molecules. For example, an antibody or antibody moiety that specifically recognizes a target (which can be an epitope) is an antibody or antibody moiety that binds this target with greater affinity, greater avidity, greater readiness, and/or greater duration than its bindings to other targets. In some embodiments, the extent of binding of an antibody to an unrelated target is less than about 10% of the binding of the antibody to the target as measured, e.g., by a radioimmunoassay (RIA). In some embodiments, an antibody that specifically binds a target has a dissociation constant (K_{D}) of ≤10⁻⁵ M, ≤10⁻⁶ M, ≤10⁻⁷ M, ≤10⁻⁸ M, ≤10⁻⁹ M, ≤10⁻¹⁰ M, ≤10⁻¹¹ M, or ≤10⁻¹² M. In some embodiments, an antibody specifically binds an epitope on a protein that is conserved among the protein from different species. In some embodiments, specific binding can include, but does not require exclusive binding. Binding specificity of the antibody or antigen-binding domain can be determined experimentally by methods known in the art. Such methods comprise, but are not limited to Western blots, ELISA-, RIA-, ECL-, IRMA-, EIA-, BIACORE^{™} -tests and peptide scans.

An "isolated" antibody (or construct) is one that has been identified, separated and/or recovered from a component of its production environment (e.g., natural or recombinant). In certain embodiments, the isolated polypeptide is free or substantially free from association with all other components from its production environment.

An "isolated" nucleic acid molecule encoding a construct, antibody, or antigen-binding fragment thereof described herein is a nucleic acid molecule that is identified and separated from at least one contaminant nucleic acid molecule with which it is ordinarily associated in the environment in which it was produced. In certain embodiments, the isolated nucleic acid is free or substantially free from association with all components associated with the production environment. The isolated nucleic acid molecules encoding the polypeptides and antibodies described herein is in a form other than in the form or setting in which it is found in nature. Isolated nucleic acid molecules therefore are distinguished from nucleic acid encoding the polypeptides and antibodies described herein existing naturally in cells. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid, which cell includes the primary subject cell and its progeny.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell and may contain mutations. Mutant progeny that have the same function or biological activity as screened or selected for in the originally transformed cell are included herein.

The terms "subject," "individual," and "patient" are used interchangeably herein to refer to a mammal, including, but not limited to, human, bovine, horse, feline, canine, rodent, or primate. In some embodiments, the subject is a human.

An "effective amount" of an agent refers to an amount effective, at dosages and for periods of time necessary, to achieve the desired therapeutic or prophylactic result. The specific dose may vary depending on one or more of the particular agent chosen, the dosing regimen to be followed, whether it is administered in combination with other compounds, timing of administration, the tissue to be imaged, and the physical delivery system in which it is carried.

A "therapeutically effective amount" of a substance/molecule of the application, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A therapeutically effective amount may be delivered in one or more administrations.

A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount will be less than the therapeutically effective amount.

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread (e.g., metastasis) of the disease, preventing or delaying the recurrence of the disease, delaying or slowing the progression of the disease, ameliorating the disease state, providing a remission (partial or total) of the disease, decreasing the dose of one or more other medications required to treat the disease, delaying the progression of the disease, increasing or improving the quality of life, increasing weight gain, and/or prolonging survival. Also encompassed by "treatment" is a reduction of pathological consequence of cancer (such as, for example, tumor volume). The methods of the application contemplate any one or more of these aspects of treatment. "Treatment" does not necessarily mean that the condition being treated will be cured.

It is understood that embodiments of the application described herein include "consisting" and/or "consisting essentially of' embodiments.

As used herein, the term "about" or "approximately" means within an acceptable error range for the particular value as determined by one of ordinary skill in the art, which will depend in part on how the value is measured or determined, i.e., the limitations of the measurement system. In certain embodiments, "about" can mean within 3 or more than 3 standard deviations, per the practice in the art. In certain embodiments, "about" can mean a range of up to 20%, e.g., up to 10%, up to 5%, or up to 1% of a given value. In certain embodiments, particularly with respect to biological systems or processes, the term can mean within an order of magnitude, e.g., within 5-fold or within 2-fold, of a value.

As used herein, the term "modulate" means positively or negatively alter. Exemplary modulations include a about 1%, about 2%, about 5%, about 10%, about 25%, about 50%, about 75%, or about 100% change.

As used herein, the term "increase" means alter positively by at least about 5%. An alteration may be by about 5%, about 10%, about 25%, about 30%, about 50%, about 75%, about 100% or more.

As used herein, the term "reduce" means alter negatively by at least about 5%. An alteration may be by about 5%, about 10%, about 25%, about 30%, about 50%, about 75%, or even by about 100%.

The term "about X-Y" used herein has the same meaning as "about X to about Y."

As used herein and in the appended claims, the singular forms "a," "or," and "the" include plural referents unless the context clearly dictates otherwise.

"Effector functions" refer to those biological activities attributable to the Fc region of an antibody, which vary with the antibody isotype. Examples of antibody effector functions include: C1q binding and complement dependent cytotoxicity (CDC), Fc receptor binding, antibody- dependent cell-mediated cytotoxicity (ADCC), phagocytosis, down regulation of cell surface receptors (e.g., B cell receptor), and B cell activation.

An "immunoconjugate" refers to an antibody conjugated to one or more heterologous molecule(s), including but not limited to a cytotoxic agent.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier," as used herein, refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "variable region" or "variable domain" refers to the domain of an antibody heavy or light chain that is involved in binding the antibody to antigen. In certain embodiments, the variable domains of the heavy chain and light chain (VH and VL, respectively) of a native antibody generally have similar structures, with each domain comprising four conserved framework regions (FRs) and three CDRs. (See, e.g., Kindt et al. Kuby Immunology, 61ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

### 2. ANTIBODIES

In certain embodiments, the invention is based, in part, on the discovery of single domain antibodies that bind to TIGIT, which can be used in antitumor therapeutics where the antibodies selectively inhibit the TIGIT receptor and induce beneficial immune response of immune cells, e.g., T cells. Accordingly, the present disclosure provides anti-TIGIT antibodies. In certain embodiments, an anti-TIGIT antibody disclosed herein is an antagonist antibody, which inhibits TIGIT receptor functions. In certain embodiments, the anti-TIGIT antibody blocks an interaction between a TIGIT receptor and a ligand. In certain embodiments, the anti-TIGIT antibody blocks an immune inhibitory signal from a TIGIT receptor. In certain embodiments, the anti-TIGIT antibody comprises a single domain antibody, e.g., a camelid antibody or a VHH antibody. In certain embodiments, the anti-TIGIT antibody has an improved capability of tissue infiltration due to its smaller size compared to traditional antibodies in IgG, Fab and/or scFv forms.

In certain embodiments, an antibody of the present disclosure can be or comprise a monoclonal antibody, including a chimeric, humanized or human antibody. In certain embodiments, the antibody disclosed herein comprises a humanized antibody. In certain embodiments, the antibody comprises an acceptor human framework, e.g., a human immunoglobulin framework or a human consensus framework.

In certain embodiments, an antibody of the present disclosure can be an antibody fragment, e.g., a Fv, Fab, Fab', scFv, diabody, or F(ab')2 fragment. In certain embodiments, the antibody is a full-length antibody, e.g., an intact IgG 1 antibody, or other antibody class or isotype as defined herein. In certain embodiments, an antibody of the present disclosure can incorporate any of the features, singly or in combination, as described in this application, e.g., Sections 2.1-2.11 detailed herein.

Antibodies of the present disclosure are useful, e.g., for the diagnosis or treatment of a neoplasm or a cancer. In certain embodiments, the neoplasia and cancers whose growth may be inhibited using the antibodies of this disclosure include neoplasia and cancers typically responsive to immunotherapy. In certain embodiments, the neoplasia and cancers include breast cancer (e.g., breast cell carcinoma), ovarian cancer (e.g., ovarian cell carcinoma) and renal cell carcinoma (RCC). Examples of other cancers that may be treated using the methods of this disclosure include melanoma (e.g., metastatic malignant melanoma), prostate cancer, colon cancer, lung cancer, bone cancer, pancreatic cancer, skin cancer, brain tumors, chronic or acute leukemias including acute myeloid leukemia, chronic myeloid leukemia, acute lymphoblastic leukemia, chronic lymphocytic leukemia, lymphomas (e.g., Hodgkin's and non-Hodgkin's lymphoma, lymphocytic lymphoma, primary CNS lymphoma, T-cell lymphoma) nasopharangeal carcinomas, cancer of the head or neck, cutaneous or intraocular malignant melanoma, uterine cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the breast gland, sarcoma of soft tissue, cancer of the urethra, cancer of the penis, solid tumors of childhood, cancer of the bladder, cancer of the kidney or ureter, carcinoma of the breast pelvis, neoplasm of the central nervous system (CNS), tumor angiogenesis, spinal axis tumor, brain stem glioma, pituitary adenoma, Kaposi's sarcoma, epidermoid cancer, squamous cell cancer, environmentally induced cancers including those induced by asbestos, e.g., mesothelioma and combinations of said cancers.

### 2.1 Exemplary anti-TIGIT antibodies

The present disclosure provides isolated antibodies that bind to a TIGIT protein. In certain embodiments, an anti- TIGIT antibody of the present disclosure binds to the ECD of TIGIT. In certain embodiments, the anti-TIGIT antibody binds to the ECD of TIGIT that comprises the amino acid sequence set forth in SEQ ID NO: 222. In certain embodiments, the anti-TIGIT antibody binds to the same epitope as an anti-TIGIT antibody, e.g., 2A3, described herein.

In certain embodiments, the anti-TIGIT antibody disclosed herein can function as an antagonist of a TIGIT receptor. In certain embodiments, the anti-TIGIT antibody can reduce the activity of the TIGIT receptor by at least about 10%, about 20%, about 30%, about 40%, about 50%, about 60%, about 70%, about 80%, about 90%, about 99% or about 99.9%. In certain embodiments, the anti-TIGIT antibody can block the downstream immune inhibitory signaling of the TIGIT receptor. In certain embodiments, the anti-TIGIT antibody increases an immune response and/or an antitumor effect of an immune cell, e.g., a T cell and/or a NK cell. In certain embodiments, treatment using the anti-TIGIT antibody exhibits antitumor efficacy in a subject, whereby reduces tumor growth and/or lengthen the survival of a subject. In certain embodiments, the anti-TIGIT antibody comprising a single domain antibody (e.g., a VHH) has a smaller molecule size compared to a full-length antibody due to the smaller size of a single domain antibody compared to a Fab domain of a full-length antibody, which can result in superior tissue infiltration, e.g., at a tumor site, compared to a full-length antibody. In certain embodiments, treatment using the anti-TIGIT antibody exhibits superior antitumor efficacy compared to treatment using a full-length anti-TIGIT antibody, e.g., Reference Ab 1 having the same amino acid sequences of BMS 22G2 disclosed in U.S. 2016/0176963 A1 and Reference Ab 2 having the same amino acid sequences of Tiragolumab, which sequences are disclosed in U.S. 2017/0088613 A1.

In certain embodiments, the anti-TIGIT antibody comprises a single domain antibody that binds to TIGIT. In certain embodiments, the single domain antibody comprises a VHH In certain embodiments, the single domain antibody comprises a heavy chain variable region (VH). In certain embodiments, the single domain antibody is linked to a Fc region. In certain embodiments, the single domain antibody is not linked to a Fc region.

In certain embodiments, the single domain antibody binds to TIGIT with a KD of about 1×10⁻⁷ M or less. In certain embodiments, the single domain antibody binds to TIGIT with a KD of about 1×10⁻⁸ M or less. In certain embodiments, the single domain antibody binds to TIGIT with a KD of about 5×10⁻⁹ M or less. In certain embodiments, the single domain antibody binds to TIGIT with a KD of about 1×10⁻⁹ M or less. In certain embodiments, the single domain antibody binds to TIGIT with a KD of between about 1×10⁻⁹ M and about 1×10⁻⁷ M. In certain embodiments, the single domain antibody binds to TIGIT with a KD of between about 1×10⁻⁹ M and about 1×10⁻⁸ M. In certain embodiments, the single domain antibody binds to TIGIT with a KD of between about 2×10⁻⁹ M and about 1×10⁻⁸ M. In certain embodiments, the single domain antibody binds to TIGIT with a KD of between about 2×10⁻⁹ M and about 5×10⁻⁸ M. In certain embodiments, the single domain antibody binds to TIGIT with a KD of between about 1×10⁻⁹ M and about 5×10⁻⁹ M.

In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 94, a heavy chain variable region CDR2 comprising amino acids having the sequence set forth in SEQ ID NO: 95, and a heavy chain variable region CDR3 comprising amino acids having the sequence set forth in SEQ ID NO: 96. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 98, a heavy chain variable region CDR2 comprising amino acids having the sequence set forth in SEQ ID NO: 99, and a heavy chain variable region CDR3 comprising amino acids having the sequence set forth in SEQ ID NO: 100. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 102, a heavy chain variable region CDR2 comprising amino acids having the sequence set forth in SEQ ID NO: 103, and a heavy chain variable region CDR3 comprising amino acids having the sequence set forth in SEQ ID NO: 104. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 106, a heavy chain variable region CDR2 comprising amino acids having the sequence set forth in SEQ ID NO: 107, and a heavy chain variable region CDR3 comprising amino acids having the sequence set forth in SEQ ID NO: 108. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 110, a heavy chain variable region CDR2 comprising amino acids having the sequence set forth in SEQ ID NO: 111, and a heavy chain variable region CDR3 comprising amino acids having the sequence set forth in SEQ ID NO: 112. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 114, a heavy chain variable region CDR2 comprising amino acids having the sequence set forth in SEQ ID NO: 115, and a heavy chain variable region CDR3 comprising amino acids having the sequence set forth in SEQ ID NO: 116. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 118, a heavy chain variable region CDR2 comprising amino acids having the sequence set forth in SEQ ID NO: 119, and a heavy chain variable region CDR3 comprising amino acids having the sequence set forth in SEQ ID NO: 120. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 122, a heavy chain variable region CDR2 comprising amino acids having the sequence set forth in SEQ ID NO: 123, and a heavy chain variable region CDR3 comprising amino acids having the sequence set forth in SEQ ID NO: 124. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 126, a heavy chain variable region CDR2 comprising amino acids having the sequence set forth in SEQ ID NO: 127, and a heavy chain variable region CDR3 comprising amino acids having the sequence set forth in SEQ ID NO: 128. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 130, a heavy chain variable region CDR2 comprising amino acids having the sequence set forth in SEQ ID NO: 131, and a heavy chain variable region CDR3 comprising amino acids having the sequence set forth in SEQ ID NO: 132. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 134, a heavy chain variable region CDR2 comprising amino acids having the sequence set forth in SEQ ID NO: 135, and a heavy chain variable region CDR3 comprising amino acids having the sequence set forth in SEQ ID NO: 136. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 138, a heavy chain variable region CDR2 comprising amino acids having the sequence set forth in SEQ ID NO: 139, and a heavy chain variable region CDR3 comprising amino acids having the sequence set forth in SEQ ID NO: 140. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 142, a heavy chain variable region CDR2 comprising amino acids having the sequence set forth in SEQ ID NO: 143, and a heavy chain variable region CDR3 comprising amino acids having the sequence set forth in SEQ ID NO: 144. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 146, a heavy chain variable region CDR2 comprising amino acids having the sequence set forth in SEQ ID NO: 147, and a heavy chain variable region CDR3 comprising amino acids having the sequence set forth in SEQ ID NO: 148. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 150, a heavy chain variable region CDR2 comprising amino acids having the sequence set forth in SEQ ID NO: 151, and a heavy chain variable region CDR3 comprising amino acids having the sequence set forth in SEQ ID NO: 152. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 154, a heavy chain variable region CDR2 comprising amino acids having the sequence set forth in SEQ ID NO: 155, and a heavy chain variable region CDR3 comprising amino acids having the sequence set forth in SEQ ID NO: 156. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 158, a heavy chain variable region CDR2 comprising amino acids having the sequence set forth in SEQ ID NO: 159, and a heavy chain variable region CDR3 comprising amino acids having the sequence set forth in SEQ ID NO: 160. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 162, a heavy chain variable region CDR2 comprising amino acids having the sequence set forth in SEQ ID NO: 163, and a heavy chain variable region CDR3 comprising amino acids having the sequence set forth in SEQ ID NO: 164. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 166, a heavy chain variable region CDR2 comprising amino acids having the sequence set forth in SEQ ID NO: 167, and a heavy chain variable region CDR3 comprising amino acids having the sequence set forth in SEQ ID NO: 168. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 170, a heavy chain variable region CDR2 comprising amino acids having the sequence set forth in SEQ ID NO: 171, and a heavy chain variable region CDR3 comprising amino acids having the sequence set forth in SEQ ID NO: 172. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 174, a heavy chain variable region CDR2 comprising amino acids having the sequence set forth in SEQ ID NO: 175, and a heavy chain variable region CDR3 comprising amino acids having the sequence set forth in SEQ ID NO: 176. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 178, a heavy chain variable region CDR2 comprising amino acids having the sequence set forth in SEQ ID NO: 179, and a heavy chain variable region CDR3 comprising amino acids having the sequence set forth in SEQ ID NO: 180. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 182, a heavy chain variable region CDR2 comprising amino acids having the sequence set forth in SEQ ID NO: 183, and a heavy chain variable region CDR3 comprising amino acids having the sequence set forth in SEQ ID NO: 184. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 186, a heavy chain variable region CDR2 comprising amino acids having the sequence set forth in SEQ ID NO: 187, and a heavy chain variable region CDR3 comprising amino acids having the sequence set forth in SEQ ID NO: 188. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 190, a heavy chain variable region CDR2 comprising amino acids having the sequence set forth in SEQ ID NO: 191, and a heavy chain variable region CDR3 comprising amino acids having the sequence set forth in SEQ ID NO: 192.

In certain embodiments, the single domain antibody comprises a heavy chain variable region comprising: a) a heavy chain variable region CDR1 comprises an amino acid sequence of any one of SEQ ID NOs: 94, 98, 102, 106, 110, 114, 118, 122, 126, 130, 134, 138, 142, 146, 150, 154, 158, 162, 166, 170, 174, 178, 182, 186 and 190, or a variant thereof comprising up to about 3 amino acid substitutions; b) a heavy chain variable region CDR2 comprises an amino acid sequence of any one of SEQ ID NOs: 95, 99, 103, 107, 111, 115, 119, 123, 127, 131, 135, 139, 143, 147, 151, 155, 159, 163, 167, 171, 175, 179, 183, 187 and 191, or a variant thereof comprising up to about 3 amino acid substitutions; and c) a heavy chain variable region CDR3 comprises an amino acid sequence of any one of SEQ ID NOs: 96, 100, 104, 108, 112, 116, 120, 124, 128, 132, 136, 140, 144, 148, 152, 156, 160, 164, 168, 172, 176, 180, 184, 188 and 192, or a variant thereof comprising up to about 3 amino acid substitutions.

In certain embodiments, the single domain antibody comprises a heavy chain variable region that comprises a CDR1 domain, a CDR2 domain and a CDR3 domain, wherein the CDR1 domain, the CDR2 domain and the CDR3 domain respectively comprise a CDR1 domain, a CDR2 domain and a CDR3 domain comprised in a reference heavy chain variable region comprising the amino acid sequence selected from the group consisting of SEQ ID NOs: 97, 101, 105, 109, 113, 117, 121, 125, 129, 133, 137, 141, 145, 149, 153, 157, 161, 165, 169, 173, 177, 181, 185, 189 and 193.

In certain embodiments, the single domain antibody comprises a heavy chain variable region CDR1 comprising amino acids having the sequence set forth in SEQ ID NO: 94, a heavy chain variable region CDR2 comprising amino acids having the sequence set forth in SEQ ID NO: 95, and a heavy chain variable region CDR3 comprising amino acids having the sequence set forth in SEQ ID NO: 96. In certain embodiments, the single domain antibody comprises a heavy chain variable region CDR1 comprising amino acids having the sequence set forth in SEQ ID NO: 98, a heavy chain variable region CDR2 comprising amino acids having the sequence set forth in SEQ ID NO: 99, and a heavy chain variable region CDR3 comprising amino acids having the sequence set forth in SEQ ID NO: 100. In certain embodiments, the single domain antibody comprises a heavy chain variable region CDR1 comprising amino acids having the sequence set forth in SEQ ID NO: 102, a heavy chain variable region CDR2 comprising amino acids having the sequence set forth in SEQ ID NO: 103, and a heavy chain variable region CDR3 comprising amino acids having the sequence set forth in SEQ ID NO: 104. In certain embodiments, the single domain antibody comprises a heavy chain variable region CDR1 comprising amino acids having the sequence set forth in SEQ ID NO: 106, a heavy chain variable region CDR2 comprising amino acids having the sequence set forth in SEQ ID NO: 107, and a heavy chain variable region CDR3 comprising amino acids having the sequence set forth in SEQ ID NO: 108. In certain embodiments, the single domain antibody comprises a heavy chain variable region CDR1 comprising amino acids having the sequence set forth in SEQ ID NO: 110, a heavy chain variable region CDR2 comprising amino acids having the sequence set forth in SEQ ID NO: 111, and a heavy chain variable region CDR3 comprising amino acids having the sequence set forth in SEQ ID NO: 112. In certain embodiments, the single domain antibody comprises a heavy chain variable region CDR1 comprising amino acids having the sequence set forth in SEQ ID NO: 114, a heavy chain variable region CDR2 comprising amino acids having the sequence set forth in SEQ ID NO: 115, and a heavy chain variable region CDR3 comprising amino acids having the sequence set forth in SEQ ID NO: 116. In certain embodiments, the single domain antibody comprises a heavy chain variable region CDR1 comprising amino acids having the sequence set forth in SEQ ID NO: 118, a heavy chain variable region CDR2 comprising amino acids having the sequence set forth in SEQ ID NO: 119, and a heavy chain variable region CDR3 comprising amino acids having the sequence set forth in SEQ ID NO: 120. In certain embodiments, the single domain antibody comprises a heavy chain variable region CDR1 comprising amino acids having the sequence set forth in SEQ ID NO: 122, a heavy chain variable region CDR2 comprising amino acids having the sequence set forth in SEQ ID NO: 123, and a heavy chain variable region CDR3 comprising amino acids having the sequence set forth in SEQ ID NO: 124. In certain embodiments, the single domain antibody comprises a heavy chain variable region CDR1 comprising amino acids having the sequence set forth in SEQ ID NO: 126, a heavy chain variable region CDR2 comprising amino acids having the sequence set forth in SEQ ID NO: 127, and a heavy chain variable region CDR3 comprising amino acids having the sequence set forth in SEQ ID NO: 128. In certain embodiments, the single domain antibody comprises a heavy chain variable region CDR1 comprising amino acids having the sequence set forth in SEQ ID NO: 130, a heavy chain variable region CDR2 comprising amino acids having the sequence set forth in SEQ ID NO: 131, and a heavy chain variable region CDR3 comprising amino acids having the sequence set forth in SEQ ID NO: 132. In certain embodiments, the single domain antibody comprises a heavy chain variable region CDR1 comprising amino acids having the sequence set forth in SEQ ID NO: 134, a heavy chain variable region CDR2 comprising amino acids having the sequence set forth in SEQ ID NO: 135, and a heavy chain variable region CDR3 comprising amino acids having the sequence set forth in SEQ ID NO: 136. In certain embodiments, the single domain antibody comprises a heavy chain variable region CDR1 comprising amino acids having the sequence set forth in SEQ ID NO: 138, a heavy chain variable region CDR2 comprising amino acids having the sequence set forth in SEQ ID NO: 139, and a heavy chain variable region CDR3 comprising amino acids having the sequence set forth in SEQ ID NO: 140. In certain embodiments, the single domain antibody comprises a heavy chain variable region CDR1 comprising amino acids having the sequence set forth in SEQ ID NO: 142, a heavy chain variable region CDR2 comprising amino acids having the sequence set forth in SEQ ID NO: 143, and a heavy chain variable region CDR3 comprising amino acids having the sequence set forth in SEQ ID NO: 144. In certain embodiments, the single domain antibody comprises a heavy chain variable region CDR1 comprising amino acids having the sequence set forth in SEQ ID NO: 146, a heavy chain variable region CDR2 comprising amino acids having the sequence set forth in SEQ ID NO: 147, and a heavy chain variable region CDR3 comprising amino acids having the sequence set forth in SEQ ID NO: 148. In certain embodiments, the single domain antibody comprises a heavy chain variable region CDR1 comprising amino acids having the sequence set forth in SEQ ID NO: 150, a heavy chain variable region CDR2 comprising amino acids having the sequence set forth in SEQ ID NO: 151, and a heavy chain variable region CDR3 comprising amino acids having the sequence set forth in SEQ ID NO: 152. In certain embodiments, the single domain antibody comprises a heavy chain variable region CDR1 comprising amino acids having the sequence set forth in SEQ ID NO: 154, a heavy chain variable region CDR2 comprising amino acids having the sequence set forth in SEQ ID NO: 155, and a heavy chain variable region CDR3 comprising amino acids having the sequence set forth in SEQ ID NO: 156. In certain embodiments, the single domain antibody comprises a heavy chain variable region CDR1 comprising amino acids having the sequence set forth in SEQ ID NO: 158, a heavy chain variable region CDR2 comprising amino acids having the sequence set forth in SEQ ID NO: 159, and a heavy chain variable region CDR3 comprising amino acids having the sequence set forth in SEQ ID NO: 160. In certain embodiments, the single domain antibody comprises a heavy chain variable region CDR1 comprising amino acids having the sequence set forth in SEQ ID NO: 162, a heavy chain variable region CDR2 comprising amino acids having the sequence set forth in SEQ ID NO: 163, and a heavy chain variable region CDR3 comprising amino acids having the sequence set forth in SEQ ID NO: 164. In certain embodiments, the single domain antibody comprises a heavy chain variable region CDR1 comprising amino acids having the sequence set forth in SEQ ID NO: 166, a heavy chain variable region CDR2 comprising amino acids having the sequence set forth in SEQ ID NO: 167, and a heavy chain variable region CDR3 comprising amino acids having the sequence set forth in SEQ ID NO: 168. In certain embodiments, the single domain antibody comprises a heavy chain variable region CDR1 comprising amino acids having the sequence set forth in SEQ ID NO: 170, a heavy chain variable region CDR2 comprising amino acids having the sequence set forth in SEQ ID NO: 171, and a heavy chain variable region CDR3 comprising amino acids having the sequence set forth in SEQ ID NO: 172. In certain embodiments, the single domain antibody comprises a heavy chain variable region CDR1 comprising amino acids having the sequence set forth in SEQ ID NO: 174, a heavy chain variable region CDR2 comprising amino acids having the sequence set forth in SEQ ID NO: 175, and a heavy chain variable region CDR3 comprising amino acids having the sequence set forth in SEQ ID NO: 176. In certain embodiments, the single domain antibody comprises a heavy chain variable region CDR1 comprising amino acids having the sequence set forth in SEQ ID NO: 178, a heavy chain variable region CDR2 comprising amino acids having the sequence set forth in SEQ ID NO: 179, and a heavy chain variable region CDR3 comprising amino acids having the sequence set forth in SEQ ID NO: 180. In certain embodiments, the single domain antibody comprises a heavy chain variable region CDR1 comprising amino acids having the sequence set forth in SEQ ID NO: 182, a heavy chain variable region CDR2 comprising amino acids having the sequence set forth in SEQ ID NO: 183, and a heavy chain variable region CDR3 comprising amino acids having the sequence set forth in SEQ ID NO: 184. In certain embodiments, the single domain antibody comprises a heavy chain variable region CDR1 comprising amino acids having the sequence set forth in SEQ ID NO: 186, a heavy chain variable region CDR2 comprising amino acids having the sequence set forth in SEQ ID NO: 187, and a heavy chain variable region CDR3 comprising amino acids having the sequence set forth in SEQ ID NO: 188. In certain embodiments, the single domain antibody comprises a heavy chain variable region CDR1 comprising amino acids having the sequence set forth in SEQ ID NO: 190, a heavy chain variable region CDR2 comprising amino acids having the sequence set forth in SEQ ID NO: 191, and a heavy chain variable region CDR3 comprising amino acids having the sequence set forth in SEQ ID NO: 192.

In certain embodiments, the single domain antibody comprises a heavy chain variable region comprising an amino acid sequence having at least about 80%, 85%, 90%, 91%, 92%, 93%, 94%, 95%, 96%, 97%, 98%, 99%, or 100% sequence identity to the amino acid sequence selected from the group consisting of SEQ ID NOs: 97, 101, 105, 109, 113, 117, 121, 125, 129, 133, 137, 141, 145, 149, 153, 157, 161, 165, 169, 173, 177, 181, 185, 189 and 193. In certain embodiments, the single domain antibody comprises a heavy chain variable region comprising an amino acid sequence selected from the group consisting of SEQ ID NOs: 97, 101, 105, 109, 113, 117, 121, 125, 129, 133, 137, 141, 145, 149, 153, 157, 161, 165, 169, 173, 177, 181, 185, 189 and 193.

In certain embodiments, the single domain antibody comprises a heavy chain variable region comprising the amino acid sequence set forth in SEQ ID NO: 97. In certain embodiments, the single domain antibody comprises a heavy chain variable region comprising the amino acid sequence set forth in SEQ ID NO: 101. In certain embodiments, the single domain antibody comprises a heavy chain variable region comprising the amino acid sequence set forth in SEQ ID NO: 105. In certain embodiments, the single domain antibody comprises a heavy chain variable region comprising the amino acid sequence set forth in SEQ ID NO: 109. In certain embodiments, the single domain antibody comprises a heavy chain variable region comprising the amino acid sequence set forth in SEQ ID NO: 113. In certain embodiments, the single domain antibody comprises a heavy chain variable region comprising the amino acid sequence set forth in SEQ ID NO: 117. In certain embodiments, the single domain antibody comprises a heavy chain variable region comprising the amino acid sequence set forth in SEQ ID NO: 121. In certain embodiments, the single domain antibody comprises a heavy chain variable region comprising the amino acid sequence set forth in SEQ ID NO: 125. In certain embodiments, the single domain antibody comprises a heavy chain variable region comprising the amino acid sequence set forth in SEQ ID NO: 129. In certain embodiments, the single domain antibody comprises a heavy chain variable region comprising the amino acid sequence set forth in SEQ ID NO: 133. In certain embodiments, the single domain antibody comprises a heavy chain variable region comprising the amino acid sequence set forth in SEQ ID NO: 137. In certain embodiments, the single domain antibody comprises a heavy chain variable region comprising the amino acid sequence set forth in SEQ ID NO: 141. In certain embodiments, the single domain antibody comprises a heavy chain variable region comprising the amino acid sequence set forth in SEQ ID NO: 145. In certain embodiments, the single domain antibody comprises a heavy chain variable region comprising the amino acid sequence set forth in SEQ ID NO: 149. In certain embodiments, the single domain antibody comprises a heavy chain variable region comprising the amino acid sequence set forth in SEQ ID NO: 153. In certain embodiments, the single domain antibody comprises a heavy chain variable region comprising the amino acid sequence set forth in SEQ ID NO: 157. In certain embodiments, the single domain antibody comprises a heavy chain variable region comprising the amino acid sequence set forth in SEQ ID NO: 161. In certain embodiments, the single domain antibody comprises a heavy chain variable region comprising the amino acid sequence set forth in SEQ ID NO: 165. In certain embodiments, the single domain antibody comprises a heavy chain variable region comprising the amino acid sequence set forth in SEQ ID NO: 169. In certain embodiments, the single domain antibody comprises a heavy chain variable region comprising the amino acid sequence set forth in SEQ ID NO: 173. In certain embodiments, the single domain antibody comprises a heavy chain variable region comprising the amino acid sequence set forth in SEQ ID NO: 177. In certain embodiments, the single domain antibody comprises a heavy chain variable region comprising the amino acid sequence set forth in SEQ ID NO: 181. In certain embodiments, the single domain antibody comprises a heavy chain variable region comprising the amino acid sequence set forth in SEQ ID NO: 185. In certain embodiments, the single domain antibody comprises a heavy chain variable region comprising the amino acid sequence set forth in SEQ ID NO: 189. In certain embodiments, the single domain antibody comprises a heavy chain variable region comprising the amino acid sequence set forth in SEQ ID NO: 193.

In certain embodiments, any one of the amino acid sequences comprised in the heavy chain variable region can comprise up to about 1, about 2, about 3, about 4, about 5, about 6, about 7, about 8, about 9 or about 10 amino acid substitutions, deletions and/or additions. In certain embodiments, the amino acid substitution is a conservative substitution.

In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 1, a heavy chain variable region CDR2 comprising amino acids having the sequence set forth in SEQ ID NO: 2, and a heavy chain variable region CDR3 comprising amino acids having the sequence set forth in SEQ ID NO: 3. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 5, a heavy chain variable region CDR2 comprising amino acids having the sequence set forth in SEQ ID NO: 6, and a heavy chain variable region CDR3 comprising amino acids having the sequence set forth in SEQ ID NO: 7. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 9, a heavy chain variable region CDR2 comprising amino acids having the sequence set forth in SEQ ID NO: 10, and a heavy chain variable region CDR3 comprising amino acids having the sequence set forth in SEQ ID NO: 11. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 13, a heavy chain variable region CDR2 comprising amino acids having the sequence set forth in SEQ ID NO: 14, and a heavy chain variable region CDR3 comprising amino acids having the sequence set forth in SEQ ID NO: 15. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 17, a heavy chain variable region CDR2 comprising amino acids having the sequence set forth in SEQ ID NO: 18, and a heavy chain variable region CDR3 comprising amino acids having the sequence set forth in SEQ ID NO: 19. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 21, a heavy chain variable region CDR2 comprising amino acids having the sequence set forth in SEQ ID NO: 22, and a heavy chain variable region CDR3 comprising amino acids having the sequence set forth in SEQ ID NO: 23. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 25, a heavy chain variable region CDR2 comprising amino acids having the sequence set forth in SEQ ID NO: 26, and a heavy chain variable region CDR3 comprising amino acids having the sequence set forth in SEQ ID NO: 27. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 29, a heavy chain variable region CDR2 comprising amino acids having the sequence set forth in SEQ ID NO: 30, and a heavy chain variable region CDR3 comprising amino acids having the sequence set forth in SEQ ID NO: 31. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 33, a heavy chain variable region CDR2 comprising amino acids having the sequence set forth in SEQ ID NO: 34, and a heavy chain variable region CDR3 comprising amino acids having the sequence set forth in SEQ ID NO: 35. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 37, a heavy chain variable region CDR2 comprising amino acids having the sequence set forth in SEQ ID NO: 38, and a heavy chain variable region CDR3 comprising amino acids having the sequence set forth in SEQ ID NO: 39. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 41, a heavy chain variable region CDR2 comprising amino acids having the sequence set forth in SEQ ID NO: 42, and a heavy chain variable region CDR3 comprising amino acids having the sequence set forth in SEQ ID NO: 43. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 45, a heavy chain variable region CDR2 comprising amino acids having the sequence set forth in SEQ ID NO: 46, and a heavy chain variable region CDR3 comprising amino acids having the sequence set forth in SEQ ID NO: 47. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 49, a heavy chain variable region CDR2 comprising amino acids having the sequence set forth in SEQ ID NO: 50, and a heavy chain variable region CDR3 comprising amino acids having the sequence set forth in SEQ ID NO: 51. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 53, a heavy chain variable region CDR2 comprising amino acids having the sequence set forth in SEQ ID NO: 54, and a heavy chain variable region CDR3 comprising amino acids having the sequence set forth in SEQ ID NO: 55. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 57, a heavy chain variable region CDR2 comprising amino acids having the sequence set forth in SEQ ID NO: 58, and a heavy chain variable region CDR3 comprising amino acids having the sequence set forth in SEQ ID NO: 59. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 61, a heavy chain variable region CDR2 comprising amino acids having the sequence set forth in SEQ ID NO: 62, and a heavy chain variable region CDR3 comprising amino acids having the sequence set forth in SEQ ID NO: 63. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 65, a heavy chain variable region CDR2 comprising amino acids having the sequence set forth in SEQ ID NO: 66, and a heavy chain variable region CDR3 comprising amino acids having the sequence set forth in SEQ ID NO: 67. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 69, a heavy chain variable region CDR2 comprising amino acids having the sequence set forth in SEQ ID NO: 70, and a heavy chain variable region CDR3 comprising amino acids having the sequence set forth in SEQ ID NO: 71. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 73, a heavy chain variable region CDR2 comprising amino acids having the sequence set forth in SEQ ID NO: 74, and a heavy chain variable region CDR3 comprising amino acids having the sequence set forth in SEQ ID NO: 75. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 77, a heavy chain variable region CDR2 comprising amino acids having the sequence set forth in SEQ ID NO: 78, and a heavy chain variable region CDR3 comprising amino acids having the sequence set forth in SEQ ID NO: 79. In certain embodiments, the single domain antibody cross-competes for binding to TIGIT with a reference anti-TIGIT single domain antibody comprising a heavy chain variable region CDR1 comprising amino acids having the sequence set forth in SEQ ID NO: 81, a heavy chain variable region CDR2 comprising amino acids having the sequence set forth in SEQ ID NO: 82, and a heavy chain variable region CDR3 comprising amino acids having the sequence set forth in SEQ ID NO: 83.

In certain embodiments, the single domain antibody comprises a heavy chain variable region comprising a heavy chain variable region CDR1 comprising amino acids having the sequence set forth in SEQ ID NO: 1, a heavy chain variable region CDR2 comprising amino acids having the sequence set forth in SEQ ID NO: 2, and a heavy chain variable region CDR3 comprising amino acids having the sequence set forth in SEQ ID NO: 3. In certain embodiments, the single domain antibody comprises a heavy chain variable region comprising a heavy chain variable region CDR1 comprising amino acids having the sequence set forth in SEQ ID NO: 5, a heavy chain variable region CDR2 comprising amino acids having the sequence set forth in SEQ ID NO: 6, and a heavy chain variable region CDR3 comprising amino acids having the sequence set forth in SEQ ID NO: 7. In certain embodiments, the single domain antibody comprises a heavy chain variable region comprising a heavy chain variable region CDR1 comprising amino acids having the sequence set forth in SEQ ID NO: 9, a heavy chain variable region CDR2 comprising amino acids having the sequence set forth in SEQ ID NO: 10, and a heavy chain variable region CDR3 comprising amino acids having the sequence set forth in SEQ ID NO: 11. In certain embodiments, the single domain antibody comprises a heavy chain variable region comprising a heavy chain variable region CDR1 comprising amino acids having the sequence set forth in SEQ ID NO: 13, a heavy chain variable region CDR2 comprising amino acids having the sequence set forth in SEQ ID NO: 14, and a heavy chain variable region CDR3 comprising amino acids having the sequence set forth in SEQ ID NO: 15. In certain embodiments, the single domain antibody comprises a heavy chain variable region comprising a heavy chain variable region CDR1 comprising amino acids having the sequence set forth in SEQ ID NO: 17, a heavy chain variable region CDR2 comprising amino acids having the sequence set forth in SEQ ID NO: 18, and a heavy chain variable region CDR3 comprising amino acids having the sequence set forth in SEQ ID NO: 19. In certain embodiments, the single domain antibody comprises a heavy chain variable region comprising a heavy chain variable region CDR1 comprising amino acids having the sequence set forth in SEQ ID NO: 21, a heavy chain variable region CDR2 comprising amino acids having the sequence set forth in SEQ ID NO: 22, and a heavy chain variable region CDR3 comprising amino acids having the sequence set forth in SEQ ID NO: 23. In certain embodiments, the single domain antibody comprises a heavy chain variable region comprising a heavy chain variable region CDR1 comprising amino acids having the sequence set forth in SEQ ID NO: 25, a heavy chain variable region CDR2 comprising amino acids having the sequence set forth in SEQ ID NO: 26, and a heavy chain variable region CDR3 comprising amino acids having the sequence set forth in SEQ ID NO: 27. In certain embodiments, the single domain antibody comprises a heavy chain variable region comprising a heavy chain variable region CDR1 comprising amino acids having the sequence set forth in SEQ ID NO: 29, a heavy chain variable region CDR2 comprising amino acids having the sequence set forth in SEQ ID NO: 30, and a heavy chain variable region CDR3 comprising amino acids having the sequence set forth in SEQ ID NO: 31. In certain embodiments, the single domain antibody comprises a heavy chain variable region comprising a heavy chain variable region CDR1 comprising amino acids having the sequence set forth in SEQ ID NO: 33, a heavy chain variable region CDR2 comprising amino acids having the sequence set forth in SEQ ID NO: 34, and a heavy chain variable region CDR3 comprising amino acids having the sequence set forth in SEQ ID NO: 35. In certain embodiments, the single domain antibody comprises a heavy chain variable region comprising a heavy chain variable region CDR1 comprising amino acids having the sequence set forth in SEQ ID NO: 37, a heavy chain variable region CDR2 comprising amino acids having the sequence set forth in SEQ ID NO: 38, and a heavy chain variable region CDR3 comprising amino acids having the sequence set forth in SEQ ID NO: 39. In certain embodiments, the single domain antibody comprises a heavy chain variable region comprising a heavy chain variable region CDR1 comprising amino acids having the sequence set forth in SEQ ID NO: 41, a heavy chain variable region CDR2 comprising amino acids having the sequence set forth in SEQ ID NO: 42, and a heavy chain variable region CDR3 comprising amino acids having the sequence set forth in SEQ ID NO: 43. In certain embodiments, the single domain antibody comprises a heavy chain variable region comprising a heavy chain variable region CDR1 comprising amino acids having the sequence set forth in SEQ ID NO: 45, a heavy chain variable region CDR2 comprising amino acids having the sequence set forth in SEQ ID NO: 46, and a heavy chain variable region CDR3 comprising amino acids having the sequence set forth in SEQ ID NO: 47. In certain embodiments, the single domain antibody comprises a heavy chain variable region comprising a heavy chain variable region CDR1 comprising amino acids having the sequence set forth in SEQ ID NO: 49, a heavy chain variable region CDR2 comprising amino acids having the sequence set forth in SEQ ID NO: 50, and a heavy chain variable region CDR3 comprising amino acids having the sequence set forth in SEQ ID NO: 51. In certain embodiments, the single domain antibody comprises a heavy chain variable region comprising a heavy chain variable region CDR1 comprising amino acids having the sequence set forth in SEQ ID NO: 53, a heavy chain variable region CDR2 comprising amino acids having the sequence set forth in SEQ ID NO: 54, and a heavy chain variable region CDR3 comprising amino acids having the sequence set forth in SEQ ID NO: 55. In certain embodiments, the single domain antibody comprises a heavy chain variable region comprising a heavy chain variable region CDR1 comprising amino acids having the sequence set forth in SEQ ID NO: 57, a heavy chain variable region CDR2 comprising amino acids having the sequence set forth in SEQ ID NO: 58, and a heavy chain variable region CDR3 comprising amino acids having the sequence set forth in SEQ ID NO: 59. In certain embodiments, the single domain antibody comprises a heavy chain variable region comprising a heavy chain variable region CDR1 comprising amino acids having the sequence set forth in SEQ ID NO: 61, a heavy chain variable region CDR2 comprising amino acids having the sequence set forth in SEQ ID NO: 62, and a heavy chain variable region CDR3 comprising amino acids having the sequence set forth in SEQ ID NO: 63. In certain embodiments, the single domain antibody comprises a heavy chain variable region comprising a heavy chain variable region CDR1 comprising amino acids having the sequence set forth in SEQ ID NO: 65, a heavy chain variable region CDR2 comprising amino acids having the sequence set forth in SEQ ID NO: 66, and a heavy chain variable region CDR3 comprising amino acids having the sequence set forth in SEQ ID NO: 67. In certain embodiments, the single domain antibody comprises a heavy chain variable region comprising a heavy chain variable region CDR1 comprising amino acids having the sequence set forth in SEQ ID NO: 69, a heavy chain variable region CDR2 comprising amino acids having the sequence set forth in SEQ ID NO: 70, and a heavy chain variable region CDR3 comprising amino acids having the sequence set forth in SEQ ID NO: 71. In certain embodiments, the single domain antibody comprises a heavy chain variable region comprising a heavy chain variable region CDR1 comprising amino acids having the sequence set forth in SEQ ID NO: 73, a heavy chain variable region CDR2 comprising amino acids having the sequence set forth in SEQ ID NO: 74, and a heavy chain variable region CDR3 comprising amino acids having the sequence set forth in SEQ ID NO: 75. In certain embodiments, the single domain antibody comprises a heavy chain variable region comprising a heavy chain variable region CDR1 comprising amino acids having the sequence set forth in SEQ ID NO: 77, a heavy chain variable region CDR2 comprising amino acids having the sequence set forth in SEQ ID NO: 78, and a heavy chain variable region CDR3 comprising amino acids having the sequence set forth in SEQ ID NO: 79. In certain embodiments, the single domain antibody comprises a heavy chain variable region comprising a heavy chain variable region CDR1 comprising amino acids having the sequence set forth in SEQ ID NO: 81, a heavy chain variable region CDR2 comprising amino acids having the sequence set forth in SEQ ID NO: 82, and a heavy chain variable region CDR3 comprising amino acids having the sequence set forth in SEQ ID NO: 83.

In certain embodiments, the single domain antibody comprises a heavy chain variable region comprising an amino acid sequence having at least about 80%, 85%, 90%, 91%, 92%, 93%, 94%, 95%, 96%, 97%, 98%, 99%, or 100% sequence identity to the amino acid sequence selected from the group consisting of SEQ ID NOs: 4, 8, 12, 16, 20, 24, 28, 32, 36, 40, 44, 48, 52, 56, 60, 64, 68, 72, 76, 80 and 84. In certain embodiments, the single domain antibody comprises a heavy chain variable region comprising an amino acid sequence selected from the group consisting of SEQ ID NOs: 4, 8, 12, 16, 20, 24, 28, 32, 36, 40, 44, 48, 52, 56, 60, 64, 68, 72, 76, 80 and 84.

In certain embodiments, any one of the amino acid sequences comprised in the heavy chain variable region can comprise up to about 1, about 2, about 3, about 4, about 5, about 6, about 7, about 8, about 9 or about 10 amino acid substitutions, deletions and/or additions. In certain embodiments, the amino acid substitution is a conservative substitution.

In certain embodiments, the single domain antibody comprises a heavy chain variable region comprising the amino acid sequence set forth in SEQ ID NO: 4. In certain embodiments, the single domain antibody comprises a heavy chain variable region comprising the amino acid sequence set forth in SEQ ID NO: 8. In certain embodiments, the single domain antibody comprises a heavy chain variable region comprising the amino acid sequence set forth in SEQ ID NO: 12. In certain embodiments, the single domain antibody comprises a heavy chain variable region comprising the amino acid sequence set forth in SEQ ID NO: 16. In certain embodiments, the single domain antibody comprises a heavy chain variable region comprising the amino acid sequence set forth in SEQ ID NO: 20. In certain embodiments, the single domain antibody comprises a heavy chain variable region comprising the amino acid sequence set forth in SEQ ID NO: 24. In certain embodiments, the single domain antibody comprises a heavy chain variable region comprising the amino acid sequence set forth in SEQ ID NO: 28. In certain embodiments, the single domain antibody comprises a heavy chain variable region comprising the amino acid sequence set forth in SEQ ID NO: 32. In certain embodiments, the single domain antibody comprises a heavy chain variable region comprising the amino acid sequence set forth in SEQ ID NO: 36. In certain embodiments, the single domain antibody comprises a heavy chain variable region comprising the amino acid sequence set forth in SEQ ID NO: 40. In certain embodiments, the single domain antibody comprises a heavy chain variable region comprising the amino acid sequence set forth in SEQ ID NO: 44. In certain embodiments, the single domain antibody comprises a heavy chain variable region comprising the amino acid sequence set forth in SEQ ID NO: 48. In certain embodiments, the single domain antibody comprises a heavy chain variable region comprising the amino acid sequence set forth in SEQ ID NO: 52. In certain embodiments, the single domain antibody comprises a heavy chain variable region comprising the amino acid sequence set forth in SEQ ID NO: 56. In certain embodiments, the single domain antibody comprises a heavy chain variable region comprising the amino acid sequence set forth in SEQ ID NO: 60. In certain embodiments, the single domain antibody comprises a heavy chain variable region comprising the amino acid sequence set forth in SEQ ID NO: 64. In certain embodiments, the single domain antibody comprises a heavy chain variable region comprising the amino acid sequence set forth in SEQ ID NO: 68. In certain embodiments, the single domain antibody comprises a heavy chain variable region comprising the amino acid sequence set forth in SEQ ID NO: 72. In certain embodiments, the single domain antibody comprises a heavy chain variable region comprising the amino acid sequence set forth in SEQ ID NO: 76. In certain embodiments, the single domain antibody comprises a heavy chain variable region comprising the amino acid sequence set forth in SEQ ID NO: 80. In certain embodiments, the single domain antibody comprises a heavy chain variable region comprising the amino acid sequence set forth in SEQ ID NO: 84.

In certain embodiments, the single domain antibody comprises a humanized framework. In certain embodiments, the humanized framework comprises a framework sequence of the heavy chain variable region sequences selected from the group consisting of SEQ ID NOs: 85-93. In certain embodiments, the humanized framework comprises a FR2 sequence of the heavy chain variable region sequences selected from the group consisting of SEQ ID NOs: 85-93.

In certain embodiments, the anti-TIGIT antibody does not comprise a Fc region. In certain embodiments, the anti-TIGIT antibody further comprises a Fc region. In certain embodiments, the Fc region comprises a human Fc region. In certain embodiments, the Fc region comprises a Fc region selected from the group consisting of the Fc regions of IgG, IgA, IgD, IgE and IgM. In certain embodiments, the Fc region comprises a Fc region selected from the group consisting of the Fc regions of IgG1, IgG2, IgG3 and IgG4. In certain embodiments, the Fc region comprises an IgG1 Fc region. In certain embodiments, the IgG1 Fc region comprising one or more mutation that enhances an antibody-dependent cell-mediated cytotoxicity (ADCC). In certain embodiments, the IgG1 Fc region comprises the mutations of L235V, F243L, R292P, Y300L and P396L. In certain embodiments, the IgG1 Fc region comprises the mutations of S239D, A330L and I332E. In certain embodiments, the anti-TIGIT antibody comprises the amino acid sequence set forth in SEQ ID NO: 194

In certain embodiments, the heavy chain variable region is linked to a Fc region via a linker. In certain embodiments, the linker is a peptide linker. In certain embodiments, the peptide linker comprises about four to about thirty amino acids. In certain embodiments, the peptide linker comprises about four to about fifteen amino acids. In certain embodiments, the peptide linker comprise an amino acid sequence selected from the group consisting of SEQ ID NOs: 195-220.

In certain embodiments, the anti-TIGIT antibody comprises a multispecific antibody, e.g., a bispecific antibody, a full-length immunoglobulin, a single-chain Fv (scFv) fragment, a Fab fragment, a Fab' fragment, a F(ab')2, an Fv fragment, a disulfide stabilized Fv fragment (dsFv), a (dsFv)2, a VHH, a Fv-Fc fusion, a scFv-Fc fusion, a scFv-Fv fusion, a diabody, a tribody, a tetrabody or any combination thereof. In certain embodiments, the antibody comprises a multispecific antibody, e.g., a bispecific antibody, which comprises a second antibody moiety that specifically binds to a second antigen.

In certain embodiments, the second antigen is a tumor associated antigen. In certain embodiments, the tumor associated antigen is selected from the group consisting of Her-2, EGFR, PD-L1, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1) and any combination thereof.

In certain embodiments, the second antigen is an immune checkpoint regulator. In certain embodiments, the immune checkpoint regulator is selected from the group consisting of PD1, CTLA4, LAG-3, 2B4, BTLA and any combination thereof.

In certain embodiments, the anti-TIGIT antibody is conjugated to a therapeutic agent or a label. In certain embodiments, the label is selected from the group consisting of a radioisotope, a fluorescent dye and an enzyme.

### 2.2 Antibody Affinity

In certain embodiments, an antibody or an antigen-binding moiety of a multispecific antibody disclosed herein has a high binding affinity to its target antigen. In certain embodiments, the antibody or antigen-binding moiety binds to the target with a KD of about 1×10⁻⁷ M or less. In certain embodiments, the antibody or antigen-binding moiety binds to the target with a KD of about 1×10⁻⁸ M or less. In certain embodiments, the antibody or antigen-binding moiety binds to the target with a KD of about 5×10⁻⁹ M or less. In certain embodiments, the antibody or antigen-binding moiety binds to the target with a KD of about 1×10⁻⁹ M or less. In certain embodiments, the antibody or antigen-binding moiety binds to the target with a KD of between about 1×10⁻⁹ M and about 1×10⁻⁷ M. In certain embodiments, the antibody or antigen-binding moiety binds to the target with a KD of between about 1×10⁻⁹ M and about 1×10⁻⁸ M. In certain embodiments, the antibody or antigen-binding moiety binds to the target with a KD of between about 2×10⁻⁹ M and about 1×10⁻⁸ M. In certain embodiments, the antibody or antigen-binding moiety binds to the target with a KD of between about 2×10⁻⁹ M and about 5×10⁻⁸ M. In certain embodiments, the antibody or antigen-binding moiety binds to the target with a KD of between about 1×10⁻⁹ M and about 5×10⁻⁹ M.

The KD of the antibody or antigen-binding moiety can be determined by methods known in the art. Such methods comprise, but are not limited to Western blots, ELISA-, RIA-, ECL-, IRMA-, EIA-, Octet- BIACORE^{®}-tests and peptide scans.

In certain embodiments, KD can be measured using a BIACORE^{®} surface plasmon resonance assay. For example, and not by way of limitation, an assay using a BIACORE^{®}-2000 or a BIACORE^{®} 3000 (Biacore, Inc., Piscataway, NJ) is performed at 25°C with immobilized antigen CMS chips at about 10 response units (RU). In certain embodiments, carboxymethylated dextran biosensor chips (CMS, Biacore, Inc.) are activated with N-ethyl-N'-(3- dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (about 0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20TM) surfactant (PBST) at 25°C at a flow rate of approximately 25 µl/min. Association rates (kₒₙ) and dissociation rates (k_{off}) are calculated using a simple one-to-one Langmuir binding model (BIACORE^{®} Evaluation Software version 3.2) by simultaneously fitting the association and dissociation sensorgrams. The equilibrium dissociation constant (KD) can be calculated as the ratio koff/kon. See, e.g., Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO^{™} spectrophotometer (ThermoSpectronic) with a stirred cuvette.

### 2.3 Antibody Fragments

In certain embodiments, an antibody of the present disclosure comprises an antigen-binding fragment or antibody fragment. Antibody fragments include, but are not limited to, Fab, Fab', Fab'-SH, F(ab')2, Fv, and scFv fragments, and other fragments described below. For a review of certain antibody fragments, see Hudson et al. Nat. Med. 9: 129-134 (2003). For a review of scFv fragments, see, e.g., Pluckthtin, in The Pharmacology of Monoclonal Antibodies, vol. 113, Rosenburg and Moore eds., (Springer- Verlag, New York), pp. 269-31 5 (1994); see also WO 93/16185; and U.S. Patent Nos. 5,571,894 and 5,587,458. For discussion of Fab and F(ab)₂ fragments comprising salvage receptor binding epitope residues and having increased in vivo half-life, see U.S. Patent No. 5,869,046.

In certain embodiments, an antibody of the present disclosure can be a diabody. Diabodies are antibody fragments with two antigen-binding sites that may be bivalent or bispecific. See, for example, EP 404,097; WO 1993/01 161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9: 129-134 (2003).

In certain embodiments, an antibody of the present disclosure can comprise a single domain antibody. Single domain antibodies are antibody fragments that comprise all or a portion of the heavy chain variable domain or all or a portion of the light chain variable domain of an antibody. In certain embodiments, the single domain antibody is a human single-domain antibody (Domantis, Inc., Waltham, MA; see, e.g., U.S. Patent No. 6,248,516 B1). In certain embodiments, the single domain antibody is camelid single-domain antibody. In certain embodiments, the single domain antibody is a VHH. In certain embodiments, the single domain antibody is humanized.

Antibody fragments can be made by various techniques including, but not limited to, proteolytic digestion of an intact antibody as well as production by recombinant host cells (e.g., E. coli or phage), as described herein.

### 2.4 Chimeric and Humanized Antibodies

In certain embodiments, an antibody, including an antigen-binding moiety of a multispecific antibody, of the present disclosure is a chimeric antibody. Certain chimeric antibodies are described, e.g., in U.S. Patent No. 4,816,567; and Morrison et al., Proc. Natl. Acad. Sci. USA, 81:6851-6855 (1984)). In certain embodiments, a chimeric antibody comprises a non-human variable region (e.g., a variable region derived from mouse) and a human constant region. In certain embodiments, a chimeric antibody is a "class switched" antibody in which the class or subclass has been changed from that of the parent antibody. Chimeric antibodies include antigen-binding fragments thereof.

In certain embodiments, an antibody, including an antigen-binding moiety of a multispecific antibody, of the present disclosure can be a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or any portion thereof) are derived from human antibody sequences. A humanized antibody optionally can also comprise at least a portion of a human constant region. In certain embodiments, certain FR residues in a humanized antibody are substituted with corresponding residues from a non-human antibody (e.g., the antibody from which the HVR residues are derived), e.g., to restore or improve antibody specificity or affinity.

Humanized antibodies and methods of making them are described, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); Framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 2.5 Human Antibodies

In certain embodiments, an antibody of the present disclosure can be a human antibody (e.g., human domain antibody, or human DAb). Human antibodies can be produced using various techniques known in the art. Human antibodies are described generally in van Dijk and van de Winkel, Curr. Opin. Pharmacol. 5: 368-74 (2001), Lonberg, Curr. Opin. Immunol. 20:450-459 (2008), and Chen, Mol. Immunol. 47(4):912-21 (2010). Transgenic mice or rats capable of producing fully human single-domain antibodies (or DAb) are known in the art. See, e.g., US20090307787A1, U.S. Pat. No. 8,754,287, US20150289489A1, US20100122358A1, and WO2004049794.

Human antibodies (e.g., human DAbs) may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE^{™} technology; U.S. Patent No. 5,770,429 describing HuMab^{®} technology; U.S. Patent No. 7,041,870 describing K-M MOUSE^{®} technology, and U.S. Patent Application Publication No. US 2007/0061900, describing VelociMouse^{®} technology). Human variable regions from intact antibodies generated by such animals may be further modified, e.g., by combining with a different human constant region.

Human antibodies (e.g., human DAbs) can also be made by hybridoma-based methods. Human myeloma and mouse-human heteromyeloma cell lines for the production of human monoclonal antibodies have been described (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991)). Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies (e.g., human DAbs) may also be generated by isolating Fv clone variable domain sequences selected from human-derived phage display libraries. Such variable domain sequences may then be combined with a desired human constant domain. Techniques for selecting human antibodies from antibody libraries are described below.

### 2.6 Library-Derived Antibodies

The antibody moieties may be isolated by screening combinatorial libraries for antibodies with the desired activity or activities. For example, a variety of methods are known in the art for generating phage display libraries and screening such libraries for antibodies possessing the desired binding characteristics. Such methods are described, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004). Methods for constructing single-domain antibody libraries have been described, for example, see U.S. Pat. NO. 7371849.

In certain phage display methods, repertoires of V_{H} and V_{L} genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically displays antibody fragments, either as scFv fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 2.7 Antibody Variants

The presently disclosed subject matter further provides amino acid sequence variants of the disclosed antibodies. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, but are not limited to, deletions from, and/or insertions into and/or substitutions of residues within the amino acid sequences of the antibody. Any combination of deletion, insertion, and substitution can be made to arrive at the final construct, provided that the final antibody, i.e., modified, possesses the desired characteristics, e.g., antigen-binding.

### 2.7.1 Substitution, Insertion, and Deletion Variants

In certain embodiments, antibody variants having one or more amino acid substitutions are provided. Sites of interest for substitutional mutagenesis include the HVRs (or CDRs) and FRs. Conservative substitutions are shown in Table 2 under the heading of "Preferred substitutions." More substantial changes are provided in Table 2 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**Table 2. Amino acid substitutions**

| **Original** | **Exemplary Substitutions** | **Preferred** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; and (6) aromatic: Trp, Tyr, Phe. In certain embodiments, non-conservative substitutions will entail exchanging a member of one of these classes for another class.

In certain embodiments, a type of substitutional variant involves substituting one or more hypervariable region residues of a parent antibody (e.g., a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR (or CDR) residues are mutated and the variant antibodies displayed on phage and screened for a particular biological activity (e.g. binding affinity).

Alterations (e.g., substitutions) may be made in HVRs (or CDRs), e.g., to improve antibody affinity. Such alterations may be made in HVR (or CDRs) "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001)). In certain embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR (or CDRs) -directed approaches, in which several HVR (or CDRs) residues (e.g., 4-6 residues at a time) are randomized. HVR (or CDRs) residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs (or CDRs) so long as such alterations do not substantially reduce the ability of the antibody to bind antigen. For example, conservative alterations (e.g., conservative substitutions as provided herein) that do not substantially reduce binding affinity may be made in HVRs (or CDRs). Such alterations may be outside of HVR (or CDR) "hotspots" or CDRs. In certain embodiments of the variant VHH sequences provided above, each HVR (or CDR) either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g., for ADEPT) or a polypeptide which increases the serum half-life of the antibody.

### 2.7.2 Glycosylation variants

In certain embodiments, the antibody moiety is altered to increase or decrease the extent to which the construct is glycosylated. Addition or deletion of glycosylation sites to an antibody may be conveniently accomplished by altering the amino acid sequence such that one or more glycosylation sites is created or removed.

Where the antibody moiety comprises an Fc region (e.g., scFv-Fc), the carbohydrate attached thereto may be altered. Native antibodies produced by mammalian cells typically comprise a branched, biantennary oligosaccharide that is generally attached by an N-linkage to Asn297 of the C_{H}2 domain of the Fc region. See, e.g., Wright etal. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In certain embodiments, modifications of the oligosaccharide in the antibody moiety may be made in order to create antibody variants with certain improved properties.

In certain embodiments, the antibody moiety has a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e.g., complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki etal. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Patent Application No. US 2003/0157108 A1, Presta, L; and WO 2004/056312 A1, Adams et al.), and knockout cell lines, such as alpha-1,6-fucosyltransferase gene, FUT8, knockout CHO cells (see, e.g., Yamane-Ohnuki etal. Biotech. Bioeng. 87: 614 (2004); Kanda, Y. etal., Biotechnol. Bioeng., 94(4):680-688 (2006); and WO2003/085107).

In certain embodiments, the antibody moiety has bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### 2.7.3 Fc region variants

In certain embodiments, one or more amino acid modifications may be introduced into the Fc region of the antibody moiety (e.g., scFv-Fc), thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the Fc fragment possesses some but not all effector functions, which make it a desirable candidate for applications in which the half-life of the antibody moiety in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity) but retains FcRn binding ability. The primary cells for mediating ADCC, NK cells, express FcγRIII only, whereas monocytes express FcyRI, FcyRII and FcγRIII. FcR expression on hematopoietic cells is summarized in Table 2 on page 464 of Ravetch and Kinet, Annu. Rev. Immunol. 9:457-492 (1991). Non-limiting examples of in vitro assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and in vivo clearance/half-life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields etal., J. Biol. Chem. 9(2): 6591-6604 (2001).)

In certain embodiments, the Fc fragment is an IgG1 Fc fragment. In certain embodiments, the IgG1 Fc fragment comprises a L234A mutation and/or a L235A mutation. In certain embodiments, the Fc fragment is an IgG2 or IgG4 Fc fragment. In certain embodiments, the Fc fragment is an IgG4 Fc fragment comprising a S228P, F234A, and/or a L235A mutation.

In certain embodiments, the antibody moiety comprises an Fc region with one or more amino acid substitutions which improve ADCC, e.g., substitutions at positions 298, 333, and/or 334 of the Fc region (EU numbering of residues).

In certain embodiments, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

In certain embodiments, the antibody moiety (e.g., scFv-Fc) variant comprising a variant Fc region comprising one or more amino acid substitutions which alters half-life and/or changes binding to the neonatal Fc receptor (FcRn). Antibodies with increased half-lives and improved binding to the neonatal Fc receptor (FcRn), which is responsible for the transfer of maternal IgGs to the fetus (Guyer etal., J. Immunol. 117:587 (1976) and Kim et al., J. Immunol. 24:249 (1994)), are described in US2005/0014934A1 (Hinton et al.). Those antibodies comprise an Fc region with one or more substitutions therein which alters binding of the Fc region to FcRn. Such Fc variants include those with substitutions at one or more of Fc region residues, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### 2.7.4 Cysteine engineered antibody variants

In certain embodiments, it may be desirable to create cysteine engineered antibody moieties, e.g., "thioMAbs," in which one or more residues of an antibody are substituted with cysteine residues. In certain embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: A118 (EU numbering) of the heavy chain; and S400 (EU numbering) of the heavy chain Fc region. Cysteine engineered antibody moieties may be generated as described, e.g., in U.S. Patent No. 7,521,541.

### 2.8 Antibody Derivatives

In certain embodiments, the antibody moiety described herein may be further modified to comprise additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in diagnosis under defined conditions, etc.

In certain embodiments, the antibody moiety may be further modified to comprise one or more biologically active protein, polypeptides or fragments thereof. "Bioactive" or "biologically active", as used herein interchangeably, means showing biological activity in the body to carry out a specific function. For example, it may mean the combination with a particular biomolecule such as protein, DNA, etc., and then promotion or inhibition of the activity of such biomolecule. In certain embodiments, the bioactive protein or fragments thereof include proteins and polypeptides that are administered to patients as the active drug substance for prevention of or treatment of a disease or condition, as well as proteins and polypeptides that are used for diagnostic purposes, such as enzymes used in diagnostic tests or in vitro assays, as well as proteins and polypeptides that are administered to a patient to prevent a disease such as a vaccine.

### 2.9 Methods of Antibody Production

The antibodies disclosed herein can be produced using any available or known technique in the art. For example, but not by way of limitation, antibodies can be produced using recombinant methods and compositions, e.g., as described in U.S. Patent No. 4,816,567. Detailed procedures to generate antibodies are described in the Examples below.

The presently disclosed subject matter further provides an isolated nucleic acid encoding an antibody disclosed herein. For example, the isolated nucleic acid can encode an amino acid sequence comprising the VL and/or an amino acid sequence comprising the VH of the antibody, e.g., the light and/or heavy chains of the antibody.

In certain embodiments, the nucleic acid can be present in one or more vectors, e.g., expression vectors. As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, where additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the disclosed subject matter is intended to include such other forms of expression vectors, such as viral vectors (e.g., replication defective retroviruses, adenoviruses and adeno- associated viruses) that serve equivalent functions.

Different parts of the antibodies disclosed herein can be constructed in a single, multicistronic expression cassette, in multiple expression cassettes of a single vector, or in multiple vectors. Examples of elements that create polycistronic expression cassette include, but are not limited to, various viral and non-viral Internal Ribosome Entry Sites (IRES, e.g., FGF-1 IRES, FGF-2 IRES, VEGF IRES, IGF-II IRES, NF-kB IRES, RUNX1 IRES, p53 IRES, hepatitis A IRES, hepatitis C IRES, pestivirus IRES, aphthovirus IRES, picornavirus IRES, poliovirus IRES and encephalomyocarditis virus IRES) and cleavable linkers (e.g., 2A peptides , e.g., P2A, T2A, E2A and F2A peptides). Combinations of retroviral vector and an appropriate packaging line are also suitable, where the capsid proteins will be functional for infecting human cells. Various amphotropic virus- producing cell lines are known, including, but not limited to, PA12 (Miller, et al. (1985) Mol. Cell. Biol. 5:431-437); PA317 (Miller, et al. (1986) Mol. Cell. Biol. 6:2895-2902); and CRIP (Danos, et al. (1988) Proc. Natl. Acad. Sci. USA 85:6460-6464). Non- amphotropic particles are suitable too, e.g., particles pseudotyped with VSVG, RD114 or GALV envelope and any other known in the art.

In certain embodiments, the nucleic acid encoding an antibody of the present disclosure and/or the one or more vectors including the nucleic acid can be introduced into a host cell. In certain embodiments, the introduction of a nucleic acid into a cell can be carried out by any method known in the art including, but not limited to, transfection, electroporation, microinjection, infection with a viral or bacteriophage vector containing the nucleic acid sequences, cell fusion, chromosome-mediated gene transfer, microcell-mediated gene transfer, spheroplast fusion, etc. In certain embodiments, a host cell can include, e.g., has been transformed with: a vector comprising a nucleic acid that encodes an amino acid sequence comprising a single domain antibody and/or the VH of a single domain antibody. In certain embodiments, a host cell can include, e.g., has been transformed with: (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In certain embodiments, the host cell is eukaryotic, e.g., a Chinese Hamster Ovary (CHO) cell or lymphoid cell (e.g., YO, NSO, Sp20 cell).

In certain embodiments, the methods of making an antibody disclosed herein can include culturing a host cell, in which a nucleic acid encoding the antibody has been introduced, under conditions suitable for expression of the antibody, and optionally recovering the antibody from the host cell and/or host cell culture medium. In certain embodiments, the antibody is recovered from the host cell through chromatography techniques.

For recombinant production of an antibody of the present disclosure, a nucleic acid encoding an antibody, e.g., as described above, can be isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody). Suitable host cells for cloning or expression of antibody-encoding vectors include prokaryotic or eukaryotic cells described herein. For example, antibodies can be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gemgross, Nat. Biotech. 22:1409-1414 (2004), and Li et al., Nat. Biotech. 24:21 0-215 (2006). Suitable host cells for the expression of glycosylated antibody can also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. In certain embodiments, plant cell cultures can be utilized as host cells. See, e.g., US Patent Nos. 5,959,177, 6,040,498, 6,420,548, 7,125,978, and 6,417,429 (describing PLANTIBODIES^{™} technology for producing antibodies in transgenic plants).

In certain embodiments, vertebrate cells can also be used as hosts. For example, and not by way of limitation, mammalian cell lines that are adapted to grow in suspension can be useful. Non-limiting examples of useful mammalian host cell lines are monkey kidney CV1 line transformed by SY40 (COS-7); human embryonic kidney line (293 or 293 cells as described, e.g., in Graham et al., J Gen Viral. 36:59 (1977)); baby hamster kidney cells (BHK); mouse sertoli cells (TM4 cells as described, e.g., in Mather, Biol. Reprod. 23:243-251 (1980)); monkey kidney cells (CV 1); African green monkey kidney cells (VERO-76); human cervical carcinoma cells (HELA); canine kidney cells (MDCK; buffalo rat liver cells (BRL 3A); human lung cells (W138); human liver cells (Hep 02); mouse mammary tumor (MMT 060562); TRI cells, as described, e.g., in Mather et al., Annals N. Y. Acad. Sci. 383:44-68 (1982); MRC 5 cells; and FS4 cells. Other useful mammalian host cell lines include Chinese hamster ovary (CHO) cells, including DHFK CHO cells (Urlaub et al., Proc. Natl. Acad. Sci. USA 77:42 I6 (1980)); and myeloma cell lines such as YO, NSO and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ ), pp. 255-268 (2003).

In certain embodiments, techniques for making bispecific and/or multispecific antibodies include, but are not limited to, recombinant expression of two immunoglobulin heavy chain-light chain pairs having the same specificity, where one or two of the heavy chains or the light chains are fuse to an antigen binding moiety (e.g., a single domain antibody, e.g., a VHH) having a different specificity, recombinant coexpression of two immunoglobulin heavy chain- light chain pairs having different specificities (see Milstei n and Cuello, Nature 305: 537 (1983)), PCT Patent Application No. WO 93/08829, and Traunecker et al., EMBO J 10: 3655 (1991)), and "knob-in-hole" engineering (see, e.g., U.S. Patent No. 5,731 ,168). Bispecific antibodies can also be made by engineering electrostatic steering effects for making antibody Fc-heterodimeric molecules (WO 2009/089004A 1); cross-linking two or more antibodies or fragments (see, e.g., US Patent No. 4,676,980, and Brennan et al., Science , 229: 81 (1985)); using leucine zippers to produce bi specific antibodies ( see, e.g., Kostelny et al., J Immunol. , 148(5): 1547-1553 ( 1992)); using "diabody" technology for making bispecific antibody fragments (see, e.g. , Hollinger et al ., Proc. Natl. Acad. Sci. USA, 90:6444-6448 (1993)); and using single-chain Fv (sFv) dimers (see, e.g., Gruber et al., J. Immunol. , 152:5368 ( 1994)); and preparing trispecific antibodies as described, e.g., in Tutt et al. J Immunol. 147: 60 (1991).

Bispecific and multispecific molecules of the present disclosure can also be made using chemical techniques (see, e.g., Kranz (1981) Proc. Natl. Acad. Sci. USA 78:5807), "polydoma" techniques (see, e.g., U.S. Patent 4,474,893), or recombinant DNA techniques. Bispecific and multispecific molecules of the presently disclosed subject matter can also be prepared by conjugating the constituent binding specificities, e.g., a first epitope and a second epitope binding specificities, using methods known in the art and as described herein. For example, and not by way of limitation, each binding specificity of the bispecific and multispecific molecule can be generated together by recombinant fusion protein techniques, or can be generated separately and then conjugated to one another. When the binding specificities are proteins or peptides, a variety of coupling or cross-linking agents can be used for covalent conjugation. Non-limiting examples of cross-linking agents include protein A, carbodiimide, N- succinimidyl-S-acetyl-thioacetate (SATA), N-succinimidyl-3-(2-pyridyldithio )propionate (SPDP), and sulfosuccinimidyl 4-(N-maleimidomethyl ) cyclohaxane-1-carboxylate (sulfo-SMCC) (see, e.g., Karpovsky ( 1984) J. Exp. Med. 160:1686; Liu ( 1985) Proc. Natl. Acad. Sci. USA 82:8648). Other methods include those described by Paulus (Behring Ins. Mitt. (1985) No. 78, 1 18-132; Brennan (1985) Science 229:81-83), Glennie (1987) J Immunol. 139: 2367-2375). When the binding specificities are antibodies (e.g., two humanized antibodies), they can be conjugated via sulfhydryl bonding of the C-terminus hinge regions of the two heavy chains. In certain embodiments, the hinge region can be modified to contain an odd number of sulfhydryl residues, e.g., one, prior to conjugation.

In certain embodiments, both binding specificities of a bispecific antibody can be encoded in the same vector and expressed and assembled in the same host cell. This method is particularly useful where the bispecific and multispecific molecule is a MAb × MAb, MAb × Fab, Fab × F(ab')2 or ligand × Fab fusion protein. In certain embodiments, a bispecific antibody of the present disclosure can be a single chain molecule, such as a single chain bispecific antibody, a single chain bispecific molecule comprising one single chain antibody and a binding determinant, or a single chain bispecific molecule comprising two binding determinants. Bispecific and multispecific molecules can also be single chain molecules or can comprise at least two single chain molecules. Methods for preparing bi- and multispecific molecules are described, for example, in U.S. Patent No. 5,260,203; U.S. Patent No. 5,455,030; U.S. Patent No. 4,881 ,175; U.S. Patent No. 5,132,405; U.S. Patent No. 5,091 ,513; U.S. Patent No. 5,476,786; U.S. Patent No. 5,013,653; U.S. Patent No. 5,258,498; and U.S. Patent No. 5,482,858. Engineered antibodies with three or more functional antigen binding sites (e.g., epitope binding sites) including "Octopus antibodies," are also included herein (see, e.g., US 2006/0025576A1).

In certain embodiments, an animal system can be used to produce an antibody of the present disclosure. One animal system for preparing hybridomas is the murine system.

Hybridoma production in the mouse is a very well-established procedure. Immunization protocols and techniques for isolation of immunized splenocytes for fusion are known in the art. Fusion partners (e.g., murine myeloma cells) and fusion procedures are also known (see, e.g., Harlow and Lane (1988), Antibodies, A Laboratory Manual, Cold Spring Harbor Laboratory Press, Cold Spring Harbor New York).

### 2.10 Assays

The antibodies of the present disclosure provided herein can be identified, screened for, or characterized for their physical/chemical properties and/or biological activities by various assays known in the art and provided herein.

In certain embodiments, an antibody of the present disclosure can be tested for its antigen binding activity by known methods, such enzyme-linked immunosorbent assay (ELISA), a radioimmunoassay (RIA), or a Western Blot Assay. Each of these assays generally detects the presence of protein-antibody complexes of particular interest by employing a labeled reagent (e.g., an antibody) specific for the complex of interest. For example, the antibody can be detected using, e.g., an enzyme-linked antibody or antibody fragment which recognizes and specifically binds to the antibody. Alternatively, the antibody can be detected using any of a variety of other immunoassays. For example, the antibody can be radioactively labeled and used in a radioimmunoassay (RIA) (see, for example, Weintraub, B., Principles of Radioimmunoassays, Seventh Training Course on Radioligand Assay Techniques, The Endocrine Society, March 1986, which is incorporated by reference herein). The radioactive isotope can be detected by such means as the use of a Geiger counter or a scintillation counter or by autoradiography.

In certain embodiments, competition assays can be used to identify an antibody that competes with an antibody of the present disclosure, e.g., 1C12, 2A3 or 1G1, for binding to TIGIT. In certain embodiments, such a competing antibody binds to the same epitope (e.g., a linear or a conformational epitope) that is bound by 1C12, 2A3 or 1G1. Detailed exemplary methods for mapping an epitope to which an antibody binds are provided in Morris (1996) "Epitope Mapping Protocols," in Methods in Molecular Biology vol. 66 (Humana Press, Totowa, NJ).

In a non-limiting example of a competition assay, immobilized TIGIT can be incubated in a solution comprising a first labeled antibody that binds to TIGIT ( e.g., 1C12, 2A3 or 1G1) and a second unlabeled antibody that is being tested for its ability to compete with the first antibody for binding to TIGIT. The second antibody may be present in a hybridoma supernatant. As a control, immobilized TIGIT is incubated in a solution comprising the first labeled antibody but not the second unlabeled antibody. After incubation under conditions permissive for binding of the first antibody to TIGIT, excess unbound antibody is removed, and the amount of label associated with immobilized TIGIT is measured. If the amount of label associated with immobilized TIGIT is substantially reduced in the test sample relative to the control sample, then that indicates that the second antibody is competing with the first antibody for binding to TIGIT. See Harlow and Lane (1988) Antibodies: A Laboratory Manual ch.14 (Cold Spring Harbor Laboratory, Cold Spring Harbor, NY).

The present disclosure provides assays for identifying anti-TIGIT antibodies thereof having biological activity. Biological activity may include, e.g., activating an immune cell or an immune activation reporter, e.g., a NFAT reporter. Antibodies having such biological activity in vivo and/or in vitro are also provided.

### 2.11 Immunoconjugates

The presently disclosed subject matter further provides immunoconjugates comprising an antibody, disclosed herein, conjugated to one or more detection probe and/or cytotoxic agents, such as chemotherapeutic agents or drugs, growth inhibitory agents, toxins (e.g. , protein toxins, enzymatically active toxins of bacterial, fungal, plant, or animal origin, or fragments thereof), or radioactive isotopes. For example, an antibody or antigen-binding portion of the disclosed subject matter can be functionally linked (e.g., by chemical coupling, genetic fusion, noncovalent association or otherwise) to one or more other binding molecules, such as another antibody, antibody fragment, peptide or binding mimetic.

In certain embodiments, an immunoconjugate is an antibody drug conjugate (ADC) in which an antibody is conjugated to one or more drugs, including but not limited to a maytansinoid (see U.S. Patent Nos. 5,208,020, 5,416,064 and European Patent EP 0 425 235); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,1 16, 5,767,285, 5,770,701 , 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med Chem. 13:477-523 (2006); Jeffrey et al., Bioorganic & Med. Chem. Letters 16:358- 362 (2006); Torgov et al., Bioconj. Chem. 16:717-721 (2005); Nagy etal., Proc. Natl. Acad. Sci. USA 97:829-834 (2000); Dubowchik et al., Bioorg. & Med. Chem. Letters 12:1529-1532 (2002); King et al., J Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In certain embodiments, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In certain embodiments, an immunoconjugate comprises an antibody as described herein conjugated to a radioactive atom to form a radioconjugate. A variety of radioactive isotopes are available for the production of radioconjugates. Non-limiting examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it can include a radioactive atom for scintigraphic studies, for example tc99m or 1123, or a spin label for nuclear magnetic resonance (NMR) imaging (also known as magnetic resonance imaging, MRI), such as iodine-123, iodine-131, indium-11, fluorine-19, carbon- 13, nitrogen-15, oxygen-17, gadolinium, manganese or iron.

Conjugates of an antibody and cytotoxic agent can be made using a variety of bi functional protein coupling agents such as N-succinimid yl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)- ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238: 1098 (1987). Carbon- 4-labeled 1-isothiocyanatobenzyl-3-methyldiethylene triaminepentaacetic acid (MX-DTPA) is an exemplary chelating agent for conjugation of radionucleotide to the antibody. See WO94/11026. The linker can be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-1 31 (1992); U.S. Patent No. 5,208,020) can be used.

The immunuoconjugates or ADCs herein expressly contemplate, but are not limited to, such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available ( e.g., from Pierce Biotechnology, Inc., Rockford, IL., U.S.A).

### 3. METHODS OF USE

The presently disclosed subject matter further provides methods for using the disclosed antibodies, e.g., an anti-TIGIT antibody. In certain embodiments, the methods are directed to therapeutic uses of the presently disclosed antibodies. In certain embodiments, the methods are directed to diagnostic use of the presently disclosed antibodies.

### 3.1 Treatment methods

The present disclosure provides methods and use of any antibodies disclosed herein (e.g., the anti-TIGIT antibody) for treatment of diseases and disorders or for increasing an immune response. In certain embodiments, the antibody and/or pharmaceutical compositions comprising the same disclosed herein can be administered to subjects (e.g., mammals such as humans) to treat diseases and disorders or to increases an immune response. In certain embodiments, the diseases and disorders involve immune checkpoint inhibitions and/or abnormal TIGIT activity. In certain embodiments, the diseases and disorders that can be treated by the antibodies disclosed herein include, but are not limited to, neoplasia, e.g., cancer.

In certain embodiments, the present disclosure provides anti-TIGIT antibodies described herein (or fragments thereof) for use in the manufacture of a medicament. In certain embodiments, the present disclosure provides anti-TIGIT antibodies described herein (or fragments thereof) for use in the manufacture of a medicament for treating of cancer. In certain embodiments, the present disclosure provides anti-TIGIT antibodies described herein (or fragments thereof) for use in treating cancer in a subject. In certain embodiments, the present disclosure provides pharmaceutical compositions comprising an anti-TIGIT antibody provided herein (or fragments thereof) for use in treating cancer in a subject. In certain embodiments, the cancer can be blood cancers (e.g. leukemias, lymphomas, and myelomas), ovarian cancer, breast cancer, bladder cancer, brain cancer, colon cancer, intestinal cancer, liver cancer, lung cancer, pancreatic cancer, prostate cancer, skin cancer, stomach cancer, glioblastoma, throat cancer, melanoma, neuroblastoma, adenocarcinoma, glioma, soft tissue sarcoma, and various carcinomas (including prostate and small cell lung cancer). Suitable carcinomas further include any known carcinoma in the field of oncology, including, but not limited to, astrocytoma, fibrosarcoma, myxosarcoma, liposarcoma, oligodendroglioma, ependymoma, medulloblastoma, primitive neural ectodermal tumor (PNET), chondrosarcoma, osteogenic sarcoma, pancreatic ductal adenocarcinoma, small and large cell lung adenocarcinomas, chordoma, angiosarcoma, endotheliosarcoma, squamous cell carcinoma, bronchoalveolarcarcinoma, epithelial adenocarcinoma, and liver metastases thereof, lymphangiosarcoma, lymphangioendotheliosarcoma, hepatoma, cholangiocarcinoma, synovioma, mesothelioma, Ewing's tumor, rhabdomyosarcoma, colon carcinoma, basal cell carcinoma, sweat gland carcinoma, papillary carcinoma, sebaceous gland carcinoma, papillary adenocarcinoma, cystadenocarcinoma, medullary carcinoma, bronchogenic carcinoma, renal cell carcinoma, bile duct carcinoma, choriocarcinoma, seminoma, embryonal carcinoma, Wilms' tumor, testicular tumor, medulloblastoma, craniopharyngioma, ependymoma, pinealoma, hemangioblastoma, acoustic neuroma, oligodendroglioma, meningioma, neuroblastoma, retinoblastoma, leukemia, multiple myeloma, Waldenstrom's macroglobulinemia, breast tumors such as ductal and lobular adenocarcinoma, squamous and adenocarcinomas of the uterine cervix, uterine and ovarian epithelial carcinomas, prostatic adenocarcinomas, transitional squamous cell carcinoma of the bladder, B and T cell lymphomas (nodular and diffuse) plasmacytoma, acute and chronic leukemias, malignant melanoma, soft tissue sarcomas and leiomyosarcomas.

In certain embodiments, the cancer can be melanoma, NSCLC, head and neck cancer, urothelial cancer, breast cancer (e.g., triple-negative breast cancer, TNBC), gastric cancer, cholangiocarcinoma, classical Hodgkin's lymphoma (cHL), Non-Hodgkin lymphoma primary mediastinal B-Cell lymphoma (NHL PMBCL), mesothelioma, ovarian cancer, lung cancer (e.g., small-cell lung cancer), esophageal cancer, nasopharyngeal carcinoma (NPC), biliary tract cancer, colorectal cancer, cervical cancer or thyroid cancer.

In certain embodiments, the subject to be treated is a mammal (e.g., human, non-human primate, rat, mouse, cow, horse, pig, sheep, goat, dog, cat, etc.). In certain embodiments, the subject is a human. In certain embodiments, the subject is suspected of having or at risk of having a cancer or be diagnosed with a cancer or any other disease having abnormal TIGIT expression or activity.

Many diagnostic methods for cancer or any other disease exhibiting abnormal TIGIT activity and the clinical delineation of those diseases are known in the art. Such methods include, but are not limited to, e.g., immunohistochemistry, PCR, fluorescent in situ hybridization (FISH). Additional details regarding diagnostic methods for abnormal TIGIT activity or expression are described in, e.g., Gupta et al. (2009) Mod Pathol. 22(1): 128-133; Lopez-Rios et al. (2013) J Clin Pathol. 66(5): 381-385; Ellison et al. (2013) J Clin Pathol 66(2): 79-89; and Guha et al. (2013) PLoS ONE 8(6): e67782.

Administration can be by any suitable route including, e.g., intravenous, intramuscular, or subcutaneous. In some embodiments, the anti-TIGIT antibodies (or fragments thereof) and/or compositions provided herein are administered in combination with a second, third, or fourth agent (including, e.g., an antineoplastic agent, a growth inhibitory agent, a cytotoxic agent, or a chemotherapeutic agent) to treat the diseases or disorders involving abnormal TIGIT activity. Such agents include, e.g., docetaxel, gefitinib, FOLFIRI (irinotecan, 5-fluorouracil, and leucovorin), irinotecan, cisplatin, carboplatin, paclitaxel, bevacizumab (anti-VEGF antibody), FOLFOX-4, infusional fluorouracil, leucovorin, and oxaliplatin, afatinib, gemcitabine, capecitabine, pemetrexed, tivantinib, everolimus, CpG-ODN, rapamycin, lenalidomide, vemurafenib, endostatin, lapatinib, PX-866, Imprime PGG, and irlotinibm. In some embodiments, the anti-TIGIT antibodies (or fragments thereof) are conjugated to the additional agent.

In certain embodiments, the anti-TIGIT antibodies (or fragments thereof) and/or compositions provided herein are administered in combination with one or more additional therapies, such as radiation therapy, surgery, chemotherapy, and/or targeted therapy. In certain embodiments, the anti-TIGIT antibodies (or fragments thereof) and/or compositions provided herein are administered in combination with radiation therapy. In certain embodiments, the combination of an anti-TIGIT antibody (or fragment thereof) and/or composition provided herein and radiation therapy is used for treating a neoplasm or cancer disclosed herein.

Depending on the indication to be treated and factors relevant to the dosing that a physician of skill in the field would be familiar with, the anti-TIGIT antibodies or fragments thereof, provided herein will be administered at a dosage that is efficacious for the treatment of that indication while minimizing toxicity and side effects. For the treatment of a cancer, a typical dose can be, for example, in the rage of 0.001 to 1000 µg; however, doses below or above this exemplary range are within the scope of the invention. The daily dose can be about 0.1 µg /kg to about 100 mg/kg of total body weight, about 0.1 µg /kg to about 100 ¡.tg/kg of total body weight or about 1 µg /kg to about 100 µg/kg of total body weight. As noted above, therapeutic or prophylactic efficacy can be monitored by periodic assessment of treated patients. For repeated administrations over several days or longer, depending on the condition, the treatment is repeated until a desired suppression of disease symptoms occurs. However, other dosage regimens may be useful and are within the scope of the invention. The desired dosage can be delivered by a single bolus administration of the composition, by multiple bolus administrations of the composition, or by continuous infusion administration of the composition.

A pharmaceutical composition comprising the anti-TIGIT antibody or a fragment thereof can be administered one, two, three, or four times daily. The compositions can also be administered less frequently than daily, for example, six times a week, five times a week, four times a week, three times a week, twice a week, once a week, once every two weeks, once every three weeks, once a month, once every two months, once every three months, or once every six months. The compositions may also be administered in a sustained release formulation, such as in an implant which gradually releases the composition for use over a period of time, and which allows for the composition to be administered less frequently, such as once a month, once every 2-6 months, once every year, or even a single administration. The sustained release devices (such as pellets, nanoparticles, microparticles, nanospheres, microspheres, and the like) may be administered by injection.

The antibody (or a fragment thereof) may be administered in a single daily dose, or the total daily dose may be administered in divided dosages of two, three, or four times daily. The compositions can also be administered less frequently than daily, for example, six times a week, five times a week, four times a week, three times a week, twice a week, once a week, once every two weeks, once every three weeks, once a month, once every two months, once every three months, or once every six months. The antibody (or a fragment thereof) may also be administered in a sustained release formulation, such as in an implant which gradually releases the composition for use over a period of time, and which allows for the composition to be administered less frequently, such as once a month, once every 2-6 months, once every year, or even a single administration. The sustained release devices (such as pellets, nanoparticles, microparticles, nanospheres, microspheres, and the like) may be administered by injection or surgically implanted in various locations.

Cancer treatments can be evaluated by, e.g., but not limited to, tumor regression, tumor weight or size shrinkage, time to progression, duration of survival, progression free survival, overall response rate, duration of response, quality of life, protein expression and/or activity. Approaches to determining efficacy of the therapy can be employed, including for example, measurement of response through radiological imaging.

In certain embodiments, the efficacy of treatment is measured by the percentage tumor growth inhibition (% TGI), calculated using the equation 100-(T/C × 100), where T is the mean relative tumor volume of the treated tumor, and C is the mean relative tumor volume of a non-treated tumor. In certain embodiments, the %TGI is about 10%, about 20%, about 30%, about 40%, about 50%, about 60%, about 70%, about 80%, about 90%, about 91%, about 92%, about 93%), about 94%), about 95%, or more than 95%.

### 3.2 Methods of diagnosis and imaging

Labeled anti-TIGIT antibodies, fragments thereof, and derivatives and analogs thereof can be used for diagnostic purposes to detect, diagnose, or monitor diseases and/or disorders associated with the expression, aberrant expression and/or activity of TIGIT. For example, the anti-TIGIT antibodies (or fragments thereof) provided herein can be used in in situ, in vivo, ex vivo, and in vitro diagnostic assays or imaging assays. Methods for detecting expression of a TIGIT polypeptide, comprising (a) assaying the expression of the polypeptide in cells (e.g., tissue) or body fluid of an individual using one or more antibodies of this invention and (b) comparing the level of gene expression with a standard gene expression level, whereby an increase or decrease in the assayed gene expression level compared to the standard expression level is indicative of aberrant expression.

Additional embodiments provided herein include methods of diagnosing a disease or disorder associated with expression or aberrant expression of TIGIT in an animal (e.g., a mammal such as a human). The methods comprise detecting TIGIT molecules in the mammal. In certain embodiments, diagnosis comprises: (a) administering an effective amount of a labeled anti-PD-1 antibody (or fragment thereof) to a mammal (b) waiting for a time interval following the administering for permitting the labeled anti-TIGIT antibody (or fragment thereof) to preferentially concentrate at sites in the subject where the TIGIT molecule is expressed (and for unbound labeled molecule to be cleared to background level); (c) determining background level; and (d) detecting the labeled molecule in the subject, such that detection of labeled molecule above the background level indicates that the subject has a particular disease or disorder associated with expression or aberrant expression of TIGIT. Background level can be determined by various methods including, comparing the amount of labeled molecule detected to a standard value previously determined for a particular system.

Anti-TIGIT antibodies (or fragments thereof) provided herein can be used to assay protein levels in a biological sample using classical immunohistological methods known to those of skill in the art (e.g., see Jalkanen, et al., J. Cell. Biol. 101:976-985 (1985); Jalkanen, et al., J. Cell. Biol. 105:3087-3096 (1987)). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase; radioisotopes, such as iodine (¹³¹I, ¹²⁵I, ¹²³ I, ¹²¹I), carbon (¹⁴C), sulfur (³⁵S), tritium (³H), indium (^{115m}In, ^{113m}In, ¹¹²In, ¹¹¹In), and technetium (⁹⁹Tc, ^{99m}Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb , ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru; luminol; and fluorescent labels, such as fluorescein and rhodamine, and biotin.

Techniques known in the art may be applied to labeled antibodies (or fragments thereof) provided herein. Such techniques include, but are not limited to, the use of bifunctional conjugating agents (see e.g., U.S. Pat. Nos. 5,756,065; 5,714,631; 5,696,239; 5,652,361; 5,505,931; 5,489,425; 5,435,990; 5,428,139; 5,342,604; 5,274,119; 4,994,560; and 5,808,003).

Alternatively, or additionally, one can measure levels of a TIGIT polypeptide-encoding nucleic acid or mRNA in the cell, e.g., via fluorescent in situ hybridization using a nucleic acid based probe corresponding to an EGFR-encoding nucleic acid or the complement thereof; (FISH; see WO98/45479 published October, 1998), Southern blotting, Northern blotting, or polymerase chain reaction (PCR) techniques, such as real time quantitative PCR (RT-PCR). One can also study TIGIT overexpression by measuring shed antigen in a biological fluid such as serum, e.g., using antibody-based assays (see also, e.g., U.S. Patent No. 4,933,294 issued June 12, 1990; WO91/05264 published April18, 1991; U.S. Patent 5,401,638 issued March 28, 1995; and Sias et al., J. Immunol. Methods 132:73-80 (1990)). Aside from the above assays, various in vivo and ex vivo assays are available to the skilled practitioner. For example, one can expose cells within the body of the mammal to an antibody which is optionally labeled with a detectable label, e.g., a radioactive isotope, and binding of the antibody to the body cells can be evaluated, e.g., by external scanning for radioactivity or by analyzing a sample (e.g., a biopsy or other biological sample) taken from a mammal previously exposed to the antibody.

### 4. PHARMACEUTICAL FORMULATIONS

The presently disclosed subject matter further provides pharmaceutical formulations containing one or more antibodies disclosed herein, with a pharmaceutically acceptable carrier. In certain embodiments, the pharmaceutical compositions can include a combination of multiple (e.g., two or more) antibodies and/or antigen-binding portions thereof of the presently disclosed subject matter. In certain embodiments, a pharmaceutical composition of the present disclosure can include one or more anti-TIGIT antibodies.

In certain embodiments, the disclosed pharmaceutical formulations can be prepared by combining an antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. For example, but not by way of limitation, lyophilized antibody formulations are described in US Patent No. 6,267,958. In certain embodiments, aqueous antibody formulations can include those described in US Patent No. 6,171 ,586 and WO2006/044908, the latter formulations including a histidine-acetate buffer. In certain embodiments, the antibody can be of a purity greater than about 80%, greater than about 90%, greater than about 91%, greater than about 92%, greater than about 93%, greater than about 94%, greater than about 95%, greater than about 96%, greater than about 97%, greater than about 98%, greater than about 99%, greater than about 99.1%, greater than about 99.2%, greater than about 99.3%, greater than about 99.4%, greater than about 99.5%, greater than about 99.6%, greater than about 99.7%, greater than about 99.8% or greater than about 99.9%.

Pharmaceutically acceptable carriers are generally nontoxic to recipients at the dosages and concentrations employed, and include, but are not limited to: buffers such as phosphate, citrate, and other organic acids, antioxidants including ascorbic acid and methionine, preservatives (such as octadecyldimethylbenzyl ammonium chloride, hexamethonium chloride, benzalkonium chloride, benzethonium chloride, phenol, butyl or benzyl alcohol, alkyl parabens such as methyl or propyl paraben, catechol, resorcinol, cyclohexanol, 3-pentanol, and m-cresol), low molecular weight (less than about 10 residues) polypeptides, proteins, such as serum albumin, gelatin, or immunoglobulins, hydrophilic polymers such as polyvinylpyrrolidone, amino acids such as glycine, glutamine, asparagine, histidine, arginine, or lysine, monosaccharides, disaccharides, and other carbohydrates including glucose, mannose, or dextrins, chelating agents such as EDTA, sugars such as sucrose, mannitol, trehalose or sorbitol, salt-forming counter-ions such as sodium, metal complexes (e.g., Zn-protein complexes), and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In certain embodiments, a sHASEGP is combined with one or more additional glycosaminoglycanases such as chondroitinases.

The carrier can be suitable for intravenous, intramuscular, subcutaneous, parenteral, spinal or epidermal administration (e.g., by injection or infusion). Depending on the route of administration, the active compound, i.e., an anti-TIGIT antibody, can be coated in a material to protect the compound from the action of acids and other natural conditions that may inactivate the compound.

Pharmaceutical compositions of the present disclosure also can be administered in combination therapy, i.e., combined with other agents. In certain embodiments, pharmaceutical compositions disclosed herein can also contain more than one active ingredients as necessary for the particular indication being treated, for example, those with complementary activities that do not adversely affect each other. In certain embodiments, the pharmaceutical formulation can include a second active ingredient for treating the same disease treated by the first therapeutic. Such active ingredients are suitably present in combination in amounts that are effective for the purpose intended. For example, and not by way of limitation, the formulation of the present disclosure can also contain more than one active ingredients as necessary for the particular indication being treated, preferably those with complementary activities that do not adversely affect each other. For example, it may be desirable to further provide a second therapeutic useful for treatment of the same disease. Such active ingredients are suitably present in combination in amounts that are effective for the purpose intended.

A composition of the present disclosure can be administered by a variety of methods known in the art. The route and/or mode of administration vary depending upon the desired results. The active compounds can be prepared with carriers that protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are described by e.g., Sustained and Controlled Release Drug Delivery Systems, J.R. Robinson, ed., Marcel Dekker, Inc., New York, 1978. In certain embodiments, the pharmaceutical compositions are manufactured under Good Manufacturing Practice (GMP) conditions of the U.S. Food and Drug Administration.

Sustained-release preparations containing a disclosed antibody can also be prepared. Suitable examples of sustained-release preparations include semipermeable matrices of solid hydrophobic polymers containing the antibody, which matrices are in the form of shaped articles, e.g. films, or microcapsules. In certain embodiments, active ingredients can be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

To administer an antibody of the present disclosure by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al. (1984) J Neuroimmunol. 7:27).

Pharmaceutically acceptable carriers include sterile aqueous solutions or dispersions and sterile powders for the extemporaneous preparation of sterile injectable solutions or dispersion. The use of such media and agents for pharmaceutically active substances is known in the art.

Except insofar as any conventional media or agent is incompatible with the active compound, use thereof in the pharmaceutical compositions of the present disclosure is contemplated. Supplementary active compounds can also be incorporated into the compositions.

Therapeutic compositions typically must be sterile, substantially isotonic, and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it is preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating one or more disclosed antibodies in the required amount in an appropriate solvent with one or a combination of ingredients enumerated above, as required, followed by sterilization microfiltration, e.g., by filtration through sterile filtration membranes. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Therapeutic compositions can also be administered with medical devices known in the art.

For example, a therapeutic composition of the present disclosure can be administered with a needleless hypodermic injection device, such as the devices disclosed in, e.g., U.S. Patent Nos. 5,399,163, 5,383,851, 5,312,335, 5,064,413, 4,941,880, 4,790,824 or 4,596,556. Examples of implants and modules useful in the present disclosure include: U.S. Patent No. 4,487,603, which discloses an implantable micro-infusion pump for dispensing medication at a controlled rate; U.S. Patent No. 4,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known.

For the therapeutic compositions, formulations of the present disclosure include those suitable for oral, nasal, topical (including buccal and sublingual), rectal, vaginal and/or parenteral administration. The formulations can conveniently be presented in unit dosage form and may be prepared by any methods known in the art of pharmacy. The amount of antibody, which can be combined with a carrier material to produce a single dosage form, vary depending upon the subject being treated, and the particular mode of administration. The amount of the antibody which can be combined with a carrier material to produce a single dosage form generally be that amount of the composition which produces a therapeutic effect. Generally, out of one hundred percent, this amount range from about 0.01 percent to about ninety-nine percent of active ingredient, from about 0.1 percent to about 70 percent, or from about 1 percent to about 30 per cent.

Dosage forms for the topical or transdermal administration of compositions of the present disclosure include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The phrases "parenteral administration" and "administered parenterally" mean modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

These pharmaceutical compositions can also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form can be brought about by the inclusion of agents which delay absorption such as aluminum monostearate and gelatin.

In certain embodiments, when the antibodies of the present disclosure are administered as pharmaceuticals, to humans and animals, they can be given alone or as a pharmaceutical composition containing, for example, from about 0.01% to about 99.5% (or about 0.1% to about 90%) of an antibody, described herein, in combination with a pharmaceutically acceptable carrier.

### 5. ARTICLES OF MANUFACTURE

The presently disclosed subject matter further provides articles of manufacture containing materials useful for the treatment, prevention and/or diagnosis of the disorders described above.

In certain embodiments, the article of manufacture includes a container and a label or package insert on or associated with the container. Non limiting examples of suitable containers include bottles, vials, syringes, IV solution bags, etc. The containers can be formed from a variety of materials such as glass or plastic. The container can hold a composition which is by itself or combined with another composition effective for treating, preventing and/or diagnosing the condition and may have a sterile access port (for example, the container may be an intravenous solution bag or a vial having a stopper pierceable by a hypodermic injection needle).

In certain embodiments, at least one active agent in the composition is an antibody of the presently disclosed subject matter. The label or package insert can indicate that the composition is used for treating the condition of choice.

In certain embodiments, the article of manufacture can comprise (a) a first container with a composition contained therein, wherein the composition comprises an antibody of the present disclosure; and (b) a second container with a composition contained therein, wherein the composition comprises a further cytotoxic or otherwise therapeutic agent. In certain embodiments, the article of manufacture can further comprise a package insert indicating that the compositions can be used to treat a particular condition.

Alternatively, or additionally, the article of manufacture can further an additional container, e.g., a second or third container, including a pharmaceutically acceptable buffer, such as, but not limited to, bacteriostatic water for injection (BWFI), phosphate-buffered saline, Ringer's solution and dextrose solution. The article of manufacture can include other materials desirable from a commercial and user standpoint, including other buffers, diluents, filters, needles, and syringes.

### SEQUENCE LISTING

| **SEQ ID NO** | **Gene Name** | **Sequence** |
|---|---|---|
| 1. | 4A11 CDR1 | GRPFSNYT |
| 2. | 4A11 CDR2 | AWPSPST |
| 3. | 4A11 CDR3 | AADYKSLTQSWLNAALDY |
| 4. | 4A11 VHH | QVKLEESGGGEAQPGGSLRLSCTASGRPFSNYTMGWFRR APGKEREFVGLAWPSPSTYVVDSVKGRFTISRDNAKNTIY LQMNSLKPEDTAIYYCAADYKSLTQSWLNAALDYWGQG TQVTVSS |
| 5. | 4B5 CDR1 | PRTFSTFH |
| 6. | 4B5 CDR2 | FNWSGGRT |
| 7. | 4B5 CDR3 | AAARDRGLHDGTTSDSYLEGSHEYEY |
| 8. | 4B5 VHH | QVKLEESGGRSVLAGGSLRLRCAGTPRTFSTFHIGWFRQA PGKEREFVAAFNWSGGRTYYADSVKGRFTISRNNGKNM VYLQMTSLTPEDTGLYYCAAARDRGLHDGTTSDSYLEGS HEYEYWGQGTQVTVSS |
| 9. | 4C5 CDR1 | GRSVSTYF |
| 10. | 4C5 CDR2 | IDRGSTVT |
| 11. | 4C5 CDR3 | AAKAITRNFIATNDYDY |
| 12. | 4C5 VHH | QVQLVDSGGGLVQAGGSLRLSCAVSGRSVSTYFVGWFR QAPGKEREFVAAIDRGSTVTRYDDSVKGRFTISRDNAKD TVYLQMNSLKPEDTAVYYCAAKAITRNFIATNDYDYWG QGTQVTVSS |
| 13. | 4D5 CDR1 | GRAFNEYA |
| 14. | 4D5 CDR2 | ISSDGRFT |
| 15. | 4D5 CDR3 | AARDSGSGYYSRAQWYDY |
| 16. | 4D5 VHH | VDSGGGAVKAGDSLRLVCSAPGRTHGRAFNEYAMAWF RQGPGKERESVAAISSDGRFTYYAASVKGRFTISKDNAKS AAFLQMNSLKPEDTAVYYCAARDSGSGYYSRAQWYDY WGQGTQVTVSS |
| 17. | 4D11 CDR1 | GSISSINA |
| 18. | 4D11 CDR2 | ITNSGST |
| 19. | 4D11 CDR3 | TARRSTWYIS |
| 20. | 4D11 VHH | QVQLQESGGGLVQPGGSLRLSCAASGSISSINAMGWYRL APGKHREFVADITNSGSTNYAASVKGRFNISRDNAKDTV YLQMNSLKFEDTAVYYCTARRSTWYISSGRGTQVTVSS |
| 21. | 4E5CDR1 | GLTSSDIA |
| 22. | 4E5 CDR2 | ISSDGRFT |
| 23. | 4E5 CDR3 | AARDSGSGYYSRAQWYDY |
| 24. | 4E5 VHH | QVQLVDSGGGVVEVGASLTLSCETSGLTSSDIAVGWFRQ GPGKERESVAAISSDGRFTYYAASVKGRFTISKDNAKSAA FLQMNSLKPEDTAVYYCAARDSGSGYYSRAQWYDYWG QGTQVTVSS |
| 25. | 4H6 CDR1 | GTIFRLNR |
| 26. | 4H6 CDR2 | TIWSGRRT |
| 27. | 4H6 CDR3 | NYRRITPWEASGNY |
| 28. | 4H6 VHH | QVQLVESGGGLATAGASLILSCAASGTIFRLNRMGWFRQ APGKERERVAATIWSGRRTHYADSVKGRFTISTDNAKKT |
| | | VYLRMSSLKPEDTAVYYCNYRRITPWEASGNYWGQGTQ VTVSS |
| 29. | 4H9 CDR1 | GPIARSRS |
| 30. | 4H9 CDR2 | AAISSDGRFT |
| 31. | 4H9 CDR3 | AARDSGSGYYSRAQWYDY |
| 32. | 4H9 VHH | QVQLVESGGGAVQAGGSLRLSCTASGPIARSRSTGMGWF RQGPGKERESVAAISSDGRFTYYAASVKGRFTISKDNAKS AAFLQMNSLKPEDTAVYYCAARDSGSGYYSRAQWYDY WGQGTQVTVSS |
| 33. | 10H5 CDR1 | ETTFKSMA |
| 34. | 10H5 CDR2 | TNYNGGRT |
| 35. | 10H5 CDR3 | AAKATEGTTFPSRTYEF |
| 36. | 10H5 VHH | GGGLVQAGGSLRLACTASDPPFANYETTFKSMAMGWVR HIPGKERELVAATNYNGGRTWYSNSAKARSTISRDNAKN TVYLQMSSLKPEDTAVYYCAAKATEGTTFPSRTYEFWGQ GIQVTVSS |
| 37. | 12H7 CDR1 | GNFLSVSD |
| 38. | 12H7 CDR2 | VTEHGRT |
| 39. | 12H7 CDR3 | KASDVFTDAGAHEAVLIRDY |
| 40. | 12H7 VHH | QVQLVDSGGGLVQAGGSLRLSCKVSGNFLSVSDMSWYR QAPGMERDVVATVTEHGRTTYTDSVKGRFTISRDNAEHT TYLEMNNLKPEDTAVYFCKASDVFTDAGAHEAVLIRDY WGQGTQVTVSS |
| 41. | 13H11 CDR1 | GLTFSMYA |
| 42. | 13H11 CDR2 | ISSDGRFT |
| 43. | 13H11 CDR3 | AARDSGSGYYSRAQWYDY |
| 44. | 13H11 VHH | QVQLVESGGGLVQAGGSLRLSCAASGLTFSMYAMGWFR QGPGKERESVAAISSDGRFTYYAASVKGRFTISKDNAKSA AFLQMNSLKPEDTAVYYCAARDSGSGYYSRAQWYDYW GQGTQVTVSS |
| 45. | 15A5 CDR1 | ERTFSSFA |
| 46. | 15A5 CDR2 | IDPSGRYI |
| 47. | 15A5 CDR3 | AARIRGEGYYTRS SFYHY |
| 48. | 15A5 VHH | QVQLVESGGGLVQAGGSLRLSCAASERTFSSFAMGWFRQ APGKEREVVAAIDPSGRYIYYKDSVKGRFTMSRDNAKST VYLQMNSLKPDDTARYYCAARIRGEGYYTRSSFYHYWG QGTQVTVSS |
| 49. | 2B7 CDR1 | GRTFSSYP |
| 50. | 2B7 CDR2 | ISSDGRFT |
| 51. | 2B7 CDR3 | AARDSGSGYYSRAQWYDY |
| 52. | 2B7 VHH | QVQLVESGGGLVQAGGSLRLACAASGRTFSSYPMGWFR QGPGKERESVAAISSDGRFTYYAASVKGRFTISKDNAKSA AFLQMNSLKPEDTAVYYCAARDSGSGYYSRAQWYDYW GQGTQVTVSS |
| 53. | 2B10 CDR1 | SRIFRRYA |
| 54. | 2B10 CDR2 | ITWSGAST |
| 55. | 2B10 CDR3 | AADPWGSVIVGTAEYEY |
| 56. | 2B10 VHH | QVKLEESGGGLVQTGDSLRLSCAASSRIFRRYAMGWFRQ APGKEREFVAAITWSGASTTYTDSVKGRFTISRDSAENTT |
| | | YLQMTSLRPEDTAVYYCAADPWGSVIVGTAEYEYWGQG TLVTVSS |
| 57. | 3F10 CDR1 | EHTFSNFP |
| 58. | 3F10 CDR2 | IDSSGRLT |
| 59. | 3F10 CDR3 | AARTGGVGYYSRSSFYNY |
| 60. | 3F10 VHH | QVQLVESGGGLVQAGGSLRLSCASSEHTFSNFPMGWFRQ APGKERNVVAAIDSSGRLTYYADSVKGRFTISKDNAKST VYLQMNSLKSEDTARYYCAARTGGVGYYSRSSFYNYWG QGTLVTVSS |
| 61. | 3G6 CDR1 | GSIFGISV |
| 62. | 3G6 CDR2 | LTRAGLT |
| 63. | 3G6 CDR3 | HANIMESAASTFGRY |
| 64. | 3G6 VHH | QVQLVESGGGLVQAGGSLSLSCAASGSIFGISVMGWYRQ APGEQRDLVATLTRAGLTTYGDSVKGRFSISRDSAKNTV YLQMNNLKPEDTAVYYCHANIMESAASTFGRYWGQGTQ VTVSS |
| 65. | 3G7 CDR1 | GRTLS TYT |
| 66. | 3G7 CDR2 | AWPSPST |
| 67. | 3G7 CDR3 | AADYKSLTQSWLNAALDY |
| 68. | 3G7 VHH | QVQLVESGGGLVQAGDSLRLSCEASGRTLSTYTMGWFR RAPGKEREFVGLAWPSPSTYVVDSVKGRFTISRDNAKNTI YLQMNSLKPEDTAIYYCAADYKSLTQSWLNAALDYWGQ GTQVTVSS |
| 69. | 3H7 CDR1 | GSILSAGV |
| 70. | 3H7 CDR2 | IALDGSTG |
| 71. | 3H7 CDR3 | NANIRTDMRSAPFDH |
| 72. | 3H7 VHH | QVKLEESGGGLVQAGGSLRLSCAASGSILSAGVMRWYR QAPGKQRELVASIALDGSTGYYIDSVKGRFTISRDNAKNI VYLDMRSLEPADTAVYLCNANIRTDMRSAPFDHWGHGT QVTVSS |
| 73. | 4C6 CDR1 | GRTFSSYP |
| 74. | 4C6 CDR2 | ISSDGRFT |
| 75. | 4C6 CDR3 | AVDPTGWGTIEADFRS |
| 76. | 4C6 VHH | QVQLVESGGGLVQAGGSLRLACAASGRTFSSYPMGWFR QGPGKERESVAAISSDGRFTYYAASVKGRFTISKDNAKSA AFLQMNSLKPEDTAVYRCAVDPTGWGTIEADFRSWGQG TQVTVSS |
| 77. | 1C12 CDR1 | SRIFSRYG |
| 78. | 1C12 CDR2 | ISWNGAST |
| 79. | 1C12 CDR3 | AADPWGAVKLGTAEYEY |
| 80. | 1C12 VHH | QVQLVESGGGLVQTGDSLRLSCAASSRIFSRYGMGWFRQ APGKEREFVAAISWNGASTTYTDSVKGRFTISRDSAENTT YLQINSLRPEDTAVYYCAADPWGAVKLGTAEYEYWGQG TQVTVSS |
| 81. | 1G1 CDR1 | GPSFSSYP |
| 82. | 1G1 CDR2 | ISSDGRFT |
| 83. | 1G1 CDR3 | AARDSGSGYYSRAQWYDY |
| 84. | 1G1 VHH | QVQLVESGGGLVQAGDSLRLSCVASGPSFSSYPMGWFRQ GPGKERESVAAISSDGRFTYYAASVKGRFTISKDNAKSAA |
| | | FLQMNSLKPEDTAVYYCAARDSGSGYYSRAQWYDYWG QGTLVTVSS |
| 85. | 1G1-F-G-ERES VHH | QVQLVESGGGVVQPGRSLRLSCAASGPSFSSYPMGWFRQ GPGKERESVAAISSDGRFTYYADSVKGRFTISRDNSKNTL YLQMNSLRAEDTAVYYCAARDSGSGYYSRAQWYDYWG QGTLVTVSS |
| 86. | 1G1-F-A-ERES VHH | QVQLVESGGGVVQPGRSLRLSCAASGPSFSSYPMGWFRQ APGKERESVAAISSDGRFTYYADSVKGRFTISRDNSKNTL YLQMNSLRAEDTAVYYCAARDSGSGYYSRAQWYDYWG QGTLVTVSS |
| 87. | 1G1-F-A-EREW VHH | QVQLVESGGGVVQPGRSLRLSCAASGPSFSSYPMGWFRQ APGKEREWVAAISSDGRFTYYADSVKGRFTISRDNSKNT LYLQMNSLRAEDTAVYYCAARDSGSGYYSRAQWYDYW GQGTLVTVSS |
| 88. | 1G1-F-A-GLEW VHH | QVQLVESGGGVVQPGRSLRLSCAASGPSFSSYPMGWFRQ APGKGLEWVAAISSDGRFTYYADSVKGRFTISRDNSKNT LYLQMNSLRAEDTAVYYCAARDSGSGYYSRAQWYDYW GQGTLVTVSS |
| 89. | 1G1-F-A-GREL VHH | QVQLVESGGGVVQPGRSLRLSCAASGPSFSSYPMGWFRQ APGKGRELVAAISSDGRFTYYADSVKGRFTISRDNSKNTL YLQMNSLRAEDTAVYYCAARDSGSGYYSRAQWYDYWG QGTLVTVSS |
| 90. | 1G1-F-A-GRES VHH | QVQLVESGGGVVQPGRSLRLSCAASGPSFSSYPMGWFRQ APGKGRESVAAISSDGRFTYYADSVKGRFTISRDNSKNTL YLQMNSLRAEDTAVYYCAARDSGSGYYSRAQWYDYWG QGTLVTVSS |
| 91. | 1C12-EREF VHH | QVQLVESGGGVVQPGRSLRLSCAASSRIFSRYGMGWFRQ APGKEREFVAAISWNGASTTYTDSVKGRFTISRDNSKNTL YLQMNSLRAEDTAVYYCAADPWGAVKLGTAEYEYWGQ GTQVTVSS |
| 92. | 1C12-EREW VHH | QVQLVESGGGVVQPGRSLRLSCAASSRIFSRYGMGWFRQ APGKEREWVAAISWNGASTTYTDSVKGRFTISRDNSKNT LYLQMNSLRAEDTAVYYCAADPWGAVKLGTAEYEYWG QGTQVTVSS |
| 93. | 1C12_GLEW VHH | QVQLVESGGGVVQPGRSLRLSCAASSRIFSRYGMGWFRQ APGKGLEWVAAISWNGASTTYTDSVKGRFTISRDNSKNT LYLQMNSLRAEDTAVYYCAADPWGAVKLGTAEYEYWG QGTQVTVSS |
| 94. | 2A3 CDR1 | GGSFSSYP |
| 95. | 2A3 CDR2 | ISSDMRFT |
| 96. | 2A3 CDR3 | AARDSGVGYYSRAQWYDY |
| 97. | 2A3 VHH | QVQLVESGGGVVQPGRSLRLSCAASGGSFSSYPMGWFRQ APGKERESVAAISSDMRFTYYADSVKGRFTISRDNSKNTL YLQMNSLRAEDTAVYYCAARDSGVGYYSRAQWYDYW GQGTLVTVSS |
| 98. | 1A8 CDR1 | GPSFSSYP |
| 99. | 1A8 CDR2 | ISSRGRFT |
| 100. | 1A8 CDR3 | AARDSGSGYYSRAQWYDY |
| 101. | 1A8 VHH | QVQLVESGGGVVQPGRSLRLSCAASGPSFSSYPMGWFRQ APGKERESVAAISSRGRFTYYADSVKGRFTISRDNSKNTL YLQMNSLRAEDTAVYYCAARDSGSGYYSRAQWYDYWG QGTLVTVSS |
| 102. | 1D11 CDR1 | GPSFSSSP |
| 103. | 1D11 CDR2 | ISSMGRFT |
| 104. | 1D11 CDR3 | AARDSGSGYYSRAQWYDY |
| 105. | 1D11 VHH | QVQLVESGGGVVQPGRSLRLSCAASGPSFSSSPMGWFRQ APGKERESVAAISSMGRFTYYADSVKGRFTISRDNSKNTL YLQMNSLRAEDTAVYYCAARDSGSGYYSRAQWYDYWG QGTLVTVSS |
| 106. | 5E8 CDR1 | GPSFSSYP |
| 107. | 5E8 CDR2 | QSSDGRFT |
| 108. | 5E8 CDR3 | AARDSGSGYYSRAQWYDY |
| 109. | 5E8 VHH | QVQLVESGGGVVQPGRSLRLSCAASGPSFSSYPMGWFRQ APGKERESVAAQSSDGRFTYYADSVKGRFTISRDNSKNT LYLQMNSLRAEDTAVYYCAARDSGSGYYSRAQWYDYW GQGTLVTVSS |
| 110. | 2A5 CDR1 | GPSFSSYP |
| 111. | 2A5 CDR2 | ISSVGRFT |
| 112. | 2A5 CDR3 | AARDSGSGYYSRWQWYDY |
| 113. | 2A5 VHH | QVQLVESGGGVVQPGRSLRLSCAASGPSFSSYPMGWFRQ APGKERESVAAISSVGRFTYYADSVKGRFTISRDNSKNTL YLQMNSLRAEDTAVYYCAARDSGSGYYSRWQWYDYW GQGTLVTVSS |
| 114. | 5G10 CDR1 | GPRFSSYP |
| 115. | 5G10 CDR2 | ISSDGRFT |
| 116. | 5G10 CDR3 | AARDSGSGYYSRAQWYDG |
| 117. | 5G10 VHH | QVQLVESGGGVVQPGRSLRLSCAASGPRFSSYPMGWFRQ APGKERESVAAISSDGRFTYYADSVKGRFTISRDNSKNTL YLQMNSLRAEDTAVYYCAARDSGSGYYSRAQWYDGWG QGTLVTVSS |
| 118. | 2A6 CDR1 | GPSFSLYP |
| 119. | 2A6 CDR2 | ISSDRRFT |
| 120. | 2A6 CDR3 | AARDSGSGYYSRAQWYDY |
| 121. | 2A6 VHH | QVQLVESGGGVVQPGRSLRLSCAASGPSFSLYPMGWFRQ APGKERESVAAISSDRRFTYYADSVKGRFTISRDNSKNTL YLQMNSLRAEDTAVYYCAARDSGSGYYSRAQWYDYWG QGTLVTVSS |
| 122. | 1G1-1C12 CDR1 | GPSFSSYP |
| 123. | 1G1-1C12 CDR2 | ISSDLRFT |
| 124. | 1G1-1C12 CDR3 | AARDSGSGYYSRKQWYDY |
| 125. | 1C12 VHH | QVQLVESGGGVVQPGRSLRLSCAASGPSFSSYPMGWFRQ APGKERESVAAISSDLRFTYYADSVKGRFTISRDNSKNTL YLQMNSLRAEDTAVYYCAARDSGSGYYSRKQWYDYWG QGTLVTVSS |
| 126. | 5B5 CDR1 | GPSFSSYP |
| 127. | 5B5 CDR2 | ISSDTRFT |
| 128. | 5B5 CDR3 | AARDSGSGYYSRAQWYDR |
| 129. | 5B5 VHH | QVQLVESGGGVVQPGRSLRLSCAASGPSFSSYPMGWFRQ APGKERESVAAISSDTRFTYYADSVKGRFTISRDNSKNTL YLQMNSLRAEDTAVYYCAARDSGSGYYSRAQWYDRWG QGTLVTVSS |
| 130. | 6F12 CDR1 | GPSFTSYP |
| 131. | 6F12 CDR2 | ISSDGRFK |
| 132. | 6F12 CDR3 | AAEDSGSGYYSRAQWYDY |
| 133. | 6F12 VHH | QVQLVESGGGVVQPGRSLRLSCAASGPSFTSYPMGWFRQ APGKERESVAAISSDGRFKYYADSVKGRFTISRDNSKNTL YLQMNSLRAEDTAVYYCAAEDSGSGYYSRAQWYDYWG QGTLVTVSS |
| 134. | 1C7 CDR1 | GPSFSSYP |
| 135. | 1 C7 CDR2 | ISSRGRFT |
| 136. | 1 C7 CDR3 | AARGSGSGYYSRAQWYDY |
| 137. | 1 C7 VHH | QVQLVESGGGVVQPGRSLRLSCAASGPSFSSYPMGWFRQ APGKERESVAAISSRGRFTYYADSVKGRFTISRDNSKNTL YLQMNSLRAEDTAVYYCAARGSGSGYYSRAQWYDYWG QGTLVTVSS |
| 138. | 6E9 CDR1 | GPSRSSYP |
| 139. | 6E9 CDR2 | ISSDGKFT |
| 140. | 6E9 CDR3 | AARDSGSGYYSRANWYDY |
| 141. | 6E9 VHH | QVQLVESGGGVVQPGRSLRLSCAASGPSRSSYPMGWFRQ APGKERESVAAISSDGKFTYYADSVKGRFTISRDNSKNTL YLQMNSLRAEDTAVYYCAARDSGSGYYSRANWYDYWG QGTLVTVSS |
| 142. | 1A10 CDR1 | GNSFSSYP |
| 143. | 1A10 CDR2 | ISSDGRFS |
| 144. | 1A10 CDR3 | ACRDSGSGYYSRAQWYDY |
| 145. | 1A10 VHH | QVQLVESGGGVVQPGRSLRLSCAASGNSFSSYPMGWFRQ APGKERESVAAISSDGRFSYYADSVKGRFTISRDNSKNTL YLQMNSLRAEDTAVYYCACRDSGSGYYSRAQWYDYWG QGTLVTVSS |
| 146. | 6B11 CDR1 | GPSFPSYP |
| 147. | 6B11 CDR2 | ISSRGRFT |
| 148. | 6B11 CDR3 | AARDSGSGYYSRLQWYDY |
| 149. | 6B11 VHH | QVQLVESGGGVVQPGRSLRLSCAASGPSFPSYPMGWFRQ APGKERESVAAISSRGRFTYYADSVKGRFTISRDNSKNTL YLQMNSLRAEDTAVYYCAARDSGSGYYSRLQWYDYWG QGTLVTVSS |
| 150. | 6D8 CDR1 | GPSFSSKP |
| 151. | 6D8 CDR2 | RSSDGRFT |
| 152. | 6D8 CDR3 | AARDSGSGRYSRAQWYDY |
| 153. | 6D8 VHH | QVQLVESGGGVVQPGRSLRLSCAASGPSFSSKPMGWFRQ APGKERESVAARSSDGRFTYYADSVKGRFTISRDNSKNTL YLQMNSLRAEDTAVYYCAARDSGSGRYSRAQWYDYWG QGTLVTVSS |
| 154. | 6D12 CDR1 | GPSFSTYP |
| 155. | 6D12 CDR2 | ISSDGVFT |
| 156. | 6D12 CDR3 | AARDSGSGYYSREQWYDY |
| 157. | 6D12 VHH | QVQLVESGGGVVQPGRSLRLSCAASGPSFSTYPMGWFRQ APGKERESVAAISSDGVFTYYADSVKGRFTISRDNSKNTL YLQMNSLRAEDTAVYYCAARDSGSGYYSREQWYDYWG QGTLVTVSS |
| 158. | 2C5 CDR1 | GPSFSTYP |
| 159. | 2C5 CDR2 | ISSQGRFT |
| 160. | 2C5 CDR3 | AARDSGSGYYSRAQWYDY |
| 161. | 2C5 VHH | QVQLVESGGGVVQPGRSLRLSCAASGPSFSTYPMGWFRQ APGKERESVAAISSQGRFTYYADSVKGRFTISRDNSKNTL YLQMNSLRAEDTAVYYCAARDSGSGYYSRAQWYDYWG QGTLVTVSS |
| 162. | 7F6 CDR1 | GPMFSSYP |
| 163. | 7F6 CDR2 | ISSDPRFT |
| 164. | 7F6 CDR3 | AARDSGSGYYSRAQWYDY |
| 165. | 7F6 VHH | QVQLVESGGGVVQPGRSLRLSCAASGPMFSSYPMGWFR QAPGKERESVAAISSDPRFTYYADSVKGRFTISRDNSKNT LYLQMNSLRAEDTAVYYCAARDSGSGYYSRAQWYDYW GQGTLVTVSS |
| 166. | 2D5 CDR1 | GPSFSSSP |
| 167. | 2D5 CDR2 | ISWDGRFT |
| 168. | 2D5 CDR3 | AARDSGSGYYSRAQWYVY |
| 169. | 2D5 VHH | QVQLVESGGGVVQPGRSLRLSCAASGPSFSSSPMGWFRQ APGKERESVAAISWDGRFTYYADSVKGRFTISRDNSKNT LYLQMNSLRAEDTAVYYCAARDSGSGYYSRAQWYVYW GQGTLVTVSS |
| 170. | 7B11 CDR1 | GPSFLIYP |
| 171. | 7B11 CDR2 | ISSDGRFW |
| 172. | 7B11 CDR3 | AARDSGSGYYSRVQWYDY |
| 173. | 7B11 VHH | QVQLVESGGGVVQPGRSLRLSCAASGPSFLIYPMGWFRQ APGKERESVAAISSDGRFWYYADSVKGRFTISRDNSKNTL YLQMNSLRAEDTAVYYCAARDSGSGYYSRVQWYDYWG QGTLVTVSS |
| 174. | 7D12 CDR1 | GPSFLSYP |
| 175. | 7D12 CDR2 | ISSDGRFS |
| 176. | 7D12 CDR3 | AARDWGSGYYSRAQWYDY |
| 177. | 7D12 VHH | QVQLVESGGGVVQPGRSLRLSCAASGPSFLSYPMGWFRQ APGKERESVAAISSDGRFSYYADSVKGRFTISRDNSKNTL YLQMNSLRAEDTAVYYCAARDWGSGYYSRAQWYDYW GQGTLVTVSS |
| 178. | 2A3 ML CDR1 | GGSFSSYP |
| 179. | 2A3 ML CDR2 | ISSDLRFT |
| 180. | 2A3 ML CDR3 | AARDSGVGYYSRAQWYDY |
| 181. | 2A3 ML VHH | QVQLVESGGGVVQPGRSLRLSCAASGGSFSSYPMGWFRQ APGKERESVAAISSDLRFTYYADSVKGRFTISRDNSKNTL YLQMNSLRAEDTAVYYCAARDSGVGYYSRAQWYDYW GQGTLVTVSS |
| 182. | 2A3 MI CDR1 | GGSFSSYP |
| 183. | 2A3 MI CDR2 | ISSDIRFT |
| 184. | 2A3 MI CDR3 | AARDSGVGYYSRAQWYDY |
| 185. | 2A3 MI VHH | QVQLVESGGGVVQPGRSLRLSCAASGGSFSSYPMGWFRQ APGKERESVAAISSDIRFTYYADSVKGRFTISRDNSKNTLY LQMNSLRAEDTAVYYCAARDSGVGYYSRAQWYDYWG QGTLVTVSS |
| 186. | 2A3 ML DT CDR1 | GGSFSSYP |
| 187. | 2A3 ML DT CDR2 | ISSDLRFT |
| 188. | 2A3 ML DT CDR3 | AARTSGVGYYSRAQWYDY |
| 189. | 2A3 ML DT VHH | QVQLVESGGGVVQPGRSLRLSCAASGGSFSSYPMGWFRQ APGKERESVAAISSDLRFTYYADSVKGRFTISRDNSKNTL YLQMNSLRAEDTAVYYCAARTSGVGYYSRAQWYDYWG QGTLVTVSS |
| 190. | 2A3 ML DE CDR1 | GGSFSSYP |
| 191. | 2A3 ML DE CDR2 | ISSDLRFT |
| 192. | 2A3 ML DE CDR3 | AARESGVGYYSRAQWYDY |
| 193. | 2A3 ML DE VHH | QVQLVESGGGVVQPGRSLRLSCAASGGSFSSYPMGWFRQ APGKERESVAAISSDLRFTYYADSVKGRFTISRDNSKNTL YLQMNSLRAEDTAVYYCAARESGVGYYSRAQWYDYWG QGTLVTVSS |
| 194. | 2A3 LT Fc | QVQLVESGGGVVQPGRSLRLSCAASGGSFSSYPMGWFRQ APGKERESVAAISSDLRFTYYADSVKGRFTISRDNSKNTL YLQMNSLRAEDTAVYYCAARTSGVGYYSRAQWYDYWG QGTLVTVSSEPKSCDKTHTCPPCPAPELLGGPSVFLFPPKP KDTLMISRTPEVTCVVVDVSHEDPEVKFNWYVDGVEVH NAKTKPREEQYNSTYRVVSVLTVLHQDWLNGKEYKCKV SNKALPAPIEKTISKAKGQPREPQVYTLPPSREEMTKNQV SLTCLVKGFYPSDIAVEWESNGQPENNYKTTPPVLDSDGS FFLYSKLTVDKSRWQQGNVFSCSVMHEALHNHYTQKSL SLSPGK |
| 195. | Exemplary linker | GSGGSGGSGGSG |
| 196. | Exemplary linker | GGGGSGGGGSGGGGS |
| 197. | Exemplary linker | GGGSG |
| 198. | Exemplary linker | GGGSGGGGSG |
| 199. | Exemplary linker | GGSGGGSG |
| 200. | Exemplary linker | GGSGGGSGGGSG |
| 201. | Exemplary linker | GSGGSG |
| 202. | Exemplary linker | GSGGSGGSG |
| 203. | Exemplary linker | GSGSGSG |
| 204. | Exemplary linker | GGGGSGGGGSGGGGSGGG |
| 205. | Exemplary linker | PAPAP |
| 206. | Exemplary linker | PAPAPPAPAPPAPAP |
| 207. | Exemplary linker | IKRTVAA |
| 208. | Exemplary linker | VSSASTK |
| 209. | Exemplary linker | AEAAAKA |
| 210. | Exemplary linker | AEAAAKEAAAKA |
| 211. | Exemplary linker | GRPGS GRPGS |
| 212. | Exemplary linker | GRPGS GRPGS GRPGS GRPGS |
| 213. | Exemplary linker | GRGGS GRGGS |
| 214. | Exemplary linker | GRGGS GRGGS GRGGS GRGGS |
| 215. | Exemplary linker | GKPGS GKPGS |
| 216. | Exemplary linker | GKPGS GKPGS GKPGS GKPGS |
| 217. | Exemplary linker | GEPGS GEPGS |
| 218. | Exemplary linker | GEGGS GEGGS GEGGS GEGGS |
| 219. | Exemplary linker | GDPGS GDPGS |
| 220. | Exemplary linker | GDPGS GDPGS GDPGS GDPGS |
| 221. | Human TIGIT polypeptide | MRWCLLLIWA QGLRQAPLAS GMMTGTIETT GNISAEKGGS IILQCHLSST TAQVTQVNWEQQDQLLAICN ADLGWHISPS FKDRVAPGPG LGLTLQSLTV NDTGEYFCIY HTYPDGTYTGRIFLEVLESS VAEHGARFQI PLLGAMAATL VVICTAVIVV VALTRKKKAL RIHSVEGDLRRKSAGQEEWS PSAPSPPGSC VQAEAAPAGL CGEQRGEDCA ELHDYFNVLS YRSLGNCSFFTETG |
| 222. | ECD of Human TIGIT polypeptide | MRWCLLLIWA QGLRQAPLAS GMMTGTIETT GNISAEKGGS IILQCHLSST TAQVTQVNWEQQDQLLAICN ADLGWHISPS FKDRVAPGPG LGLTLQSLTV NDTGEYFCIY HTYPDGTYTGRIFLEVLESS VAEHGARF |

The following examples are merely illustrative of the presently disclosed subject matter and should not be considered as limitations in any way.

### EXAMPLES

### Example 1. Immunization, generation of anti-human TIGIT VHH antibodies & hits identification

Antigen of recombinant human TIGIT extra cellular domain (ECD) protein was purchased from Arco Bio. Immunization of TIGIT was performed using llama under protocols known in the art. The titer of serum antibodies was measured by ELISA assays. After 3 rounds of immunization, a high titer (1:100,000) was observed. Whole blood was then collected, and PBMCs were isolated. RNA was then isolated from the PBMCs.

The VHH antibody genes were amplified by PCR under protocols know in the art, purified by DNA agarose gel, constructed into a phagemid vector pADL-23c (Antibody Design Labs) and transformed to TGI electrocompetent cells (from Lucigen). Transformed TGI cells were cultured in Y2T medium. Phages with target VHH displayed were produced by adding helper phage and co-culturing overnight. Phages in supernatants of culture were harvested by centrifugation, and panning of binders to human-TIGIT (h-TIGIT) or cynomolgus-TIGIT (cyno-TIGIT) antigen was performed using streptavidin-coupled Dynabeads coated with biotinylated h-TIGIT or cyno-TIGIT ECD. After 3 rounds of panning, binders of h-TIGIT or cyno-TIGIT were eluted, which were used to infect SS320 cells. Colonies of SS320 cells were picked and cultured in Y2T medium, and IPTG was added for secretion of VHH antibodies. Supernatants with VHH antibodies were screened by ELISA assays using h-TIGIT ECD coated plates. Positive h-TIGIT binders were picked for sequencing. 29 clones with different sequences were selected. Binding ability of VHH antibodies on cyno-TIGIT was also examined by ELISA. The top 21 binders and their CDRs and VHs are shown in the Sequence Table (SEQ ID NOS: 1-84).

The effects of VHH antibody clones on blocking Poliovirus Receptor (PVR, a.k.a. CD155) binding to TIGIT were also determined using blocking ELISA assay. 9 clones with over 90% inhibition of PVR binding to h-TIGIT were further selected (clone name: 2B7, 1G1, 1C12, 3G6, 2B10, 3G7, 3F10, 13H11 and 15A5).

### Example 2 - Characterization and selection of TIGIT VHH antibodies

Antibody clones identified from Example 1 were constructed to make bivalent antibody by adding human constant heavy chain2 (CH2) and constant heavy chain 3 (CH3) domains shown in Figure 1C. Constructed bivalent VHH antibodies were expressed in ExpiCHO cells, and proteins in supernatants were harvested and purified by Protein A.

Binding affinities of the bivalent clones to human TIGIT transfected Jurkat cells were confirmed by flowcytometry assays. Jurkat cells stably expressing human TIGIT and NFAT reporter gene were established. Specifically, Jurkat cells were transfected with human TIGIT expression vector by electroporation, and cells stably expressing human TIGIT were selected by 1 µg/ml puromycin during cell culture. Representative antibody clones were incubated at different concentrations with h-TIGIT stably expressing Jurkat cells (0.2×106/ml) in 100 µl/well in 96-well plate in FACS buffer (PBS with 1.5 % FBS) for 30 min. After washing, Alexaflour 488 conjugated anti-human IgG Fc secondary antibody (Alexa Fluor^{®} 488 AffiniPure Goat Anti-Human IgG, Fcγ fragment specific, Jackson labs, 1:500 dilution) was added and incubated for 30 min. After washing, mean fluorescence intensity was measured using CytoFlex (Beckman Coulter) by gating live cell population. Binding affinity was calculated using GraphPad Prism. Representative results are shown in Figures 1A and 1B. All tested antibodies showed high binding affinity to cells expressing human TIGIT compared to Reference Ab 1, a reference anti-human-TIGIT antibody disclosed in U.S. 2016/0176963 A1 as BMS 22G2, which was synthesized in-house based on the disclosed sequences.

Furthermore, Jurkat cells stably expressing human TIGIT were transfected with NFAT reporter gene using electroporation. NFAT reporter expressing cells were selected by 300 µg/ml hygromycin in culture medium. Moreover, Raji cells were stably transfected with human PVR, and transfected cells were selected by hygromycin at 125 µg/ml in culture medium. The antibodies' effects on PVR-mediated suppression of TCR-induced NFAT reporter activity were then determined by co-incubation of Raji cells expressing human PVR and Jurkat cells expressing human TIGIT and NFAT reporter gene in the presence of staphylococcal enterotoxin (SEE, 0.01 ng/ml) for 5 hours. Bright-Glo luciferase assay buffer with substrate (Promega) was added, and luciferase activity was measured by chemiluminescence activity using a plate reader. Potency of antibody in blocking TIGIT activity was calculated using nonlinear regression method by GraphPad Prims. Representative results are shown in Figure 2. All tested clones except 3F10 showed similar TIGIT blocking effects compared to the reference antibody Reference Ab 1.

### Example 3 - Humanization of anti-TIGIT antibodies

Two representative clones 1G1 and 1C12 were selected for humanization of their framework. Briefly, Igblast was performed using the sequences of the two clones to search database of human germline genes. Ideal germline sequences were selected, and mutations of framework sequences were made to change the framework sequences from llama to human. For 1C12 clone, human germline IGHV-3-30^{∗}10 was used, and three version of humanized 1C12 were made (1C12-EREF, 1C12-EREW and 1C12-GLEW). For 1G1, human germline IGHV-3-30^{∗}01 was used, and six versions of humanized 1G1 were made (1G1-F-G-ERES, 1G1-F-A-ERES, 1G1-F-A-EREW, 1G1-F-A-GLEW, 1G1-F-A-GREL and 1G1-F-A-GRES). The constructs were cloned into expression vectors, and antibody proteins were produced by transient transfection of ExpiCHO and purified by protein A.

The binding affinities of the humanized bivalent antibodies to human TIGIT were determined by whole cell binding to h-TIGIT stably expressed on Jurkat cells. Briefly, antibodies were incubated with h-TIGIT transfected Jurkat cells (0.2×10⁶/well in 100 µl) for 30 min in FACS buffer. The cells were washed once, then incubated with anti-human IgG Fc AlexaFluor488 (1:500). Mean fluorescence intensity was determined by CytoFlex, and binding affinities of antibodies to h-TIGIT were calculated using non-liner regression by GraphPad Prism 8.0 as shown in Figure 3A. Similar binding affinities were observed for all three versions of 1C12 clones compared to the chimeric parental clone. For 1G1 clone, two humanized versions, F-G-ERES and F-A-ERES, exhibited similar affinity to h-TIGIT compared to chimeric clone, whereas two versions (F-A-EREW and F-A-GLEW) lost binding affinity to h-TIGIT as shown in Figure 3B.

The activity of humanized antibodies in blocking PVR-mediated suppressive effects on TCR-mediated NFAT reporter activity was determined using a method descripted previously. Briefly, human TIGIT and NFAT reporter transfected Jurkat cells were incubated with antibodies in different concentrations, in the presence of PVR transfected Raji cells and a low concentration of staphylococcal enterotoxin (SEE, 0.01 ng/ml) for 5 hours. Bright-Glo luciferase assay buffer with substrate (Promega) was added, and luciferase activity was measured. Representative results are shown in Figures 4A and 4B. All humanized versions of 1C12 clone had similar potency in blocking TIGIT compared to the chimeric parental clone and Reference Ab 1, a reference anti-h-TIGIT antibody. All humanized versions of 1G1 had similar potency compared to the chimeric parental clone in blocking TIGIT, except two versions 1G1-(F-A-EREW) and 1G1-(F-A-GLEW) lost the blocking effect.

### Example 4 - Affinity maturation, selection & modification

Affinity maturation was performed for the 1G1-F-A-ERES clone. Primers for making single mutation of amino acid for each CDR region were designed. A library of mutations was prepared using assembly PCR and cloned into phagemil vectors. Library quality was measured by transformation of TG1 cells and DNA sequencing of clones. Phage production was carried out using helper phages, and phage panning was performed using streptavidin-coupled Dynabeads coated with biotinylated h-TIGIT ECD or cyno-TIGIT ECD. After two round of panning, elution of panning products was used to infect SS320 cells, and colonies were picked and cultured in Y2T medium with IPTG. VHH antibodies in supernatants were examined by ELISA assays. Positive clones against h- & cyno-TIGIT were selected for whole cell binding to h- & cyno-TIGIT in stable cells. PVR blocking ELISA and human TIGIT blockade NFAT reporter assays were also performed for selected clones. EC50 or IC50 values were calculated using GraphPad Prism. The top 25 binders and their CDRs and VHHs are shown in the Sequence Table (SEQ ID NOS: 94-177).

Correlations between EC50/IC50 in whole cell binding and blocking ELISA were graphed using GraphPad Prism, and representative data are shown in Figure 5. 2A3 was identified with the highest affinity and potency.

The thermostability of 12 representative clones was tested by heat treatment at 25 to 70 °C for 60 min, then the binding of the treated samples to human TIGIT was examined using ELISA and whole cell binding flow cytometry assays, the results of which are shown in Figures 6A and 6B. Clone 2A3 exhibited superior thermostability compared to other clones.

Bivalent 2A3 antibody (2A3-Fc) was constructed using human IgGI CH2 and CH3 domains. The antibody was expressed in ExpiCHO cells and purified by protein A column. Epitope of 2A3-Fc binding to human TIGIT was studied using Octet binding assay in comparison with anti-TIGIT reference antibodies Reference Ab 1 and References Ab 2 (a reference anti-human-TIGIT antibody having the same amino acid sequences of Tiragolumab, which was synthesized in-house based on the disclosed sequences in U.S. 2017/0088613 A1). As shown in Figures 7A-7C, 2A3 clone bound to a different epitope compared to Reference Ab 1 and Reference Ab 2.

Cross-species binding activity of 2A3-Fc to human, cynomolgus and mouse TIGIT was determined by ELISA assays, results of which are shown in Figures 8A-8C. 2A3-Fc bound to human, cynomolgus monkey but not mouse TIGIT.

Analysis of CDR regions of 2A3 identified two hotspots, a methionine in CDR2 and an aspartic acid in CDR3 followed by serine. Mutations of methionine to leucine and isoleucine, and mutations of aspartic acid to threonine and glutamic acid were carried out. CDRs and VHs of the modified antibodies (2A3 ML, 2A3 MI, 2A3 ML_DT (a.k.a 2A3 LT) and 2A3 ML_DE) are shown in the Sequence Table (SEQ ID NOs:178-193). The modified versions were tested in whole cell binding and NFAT luciferase reporter assays, all of which exhibited similar properties compared to the parental 2A3 clone . The representative data of 2A3-LT-Fc with changes of M to L and D to T (2A3 ML DT) are shown in Figure 9, where similar human TIGIT binding affinity in whole cell binding assay and similar potency in NFAT reporter assay were observed compared to parental 2A3-Fc.

The affinity and potency of 2A3-Fc were compared to a reference anti-TIGIT antibody Reference Ab 2. 2A3-Fc exhibited significantly higher affinity than Reference Ab 2. The EC50 value of 2A3-Fc was 0.32 ± 0.06 nM compared to 0.61±10.13 nM of Reference Ab 2 (n=3) in whole cell binding assays (Figure 10A). 2A3-Fc also exhibited similar potency of blocking TIGIT in NFAT luciferase reporter assays compared to Reference Ab 2. The EC50 value of 2A3-Fc was 0.48±1 0.18 nM compared to 0.72±0.42 nM of Reference Ab 2 (n=3) (Figure 10B).

### Example 5 - In vitro efficacy study

The anti-TIGIT antibody's antitumor effects were further examined in vitro. In the tumor microenvironment, when TIGIT+ T cells are in contact with PVR+ dendritic cells (DCs), PVR expressed on the DCs can bind to TIGIT expressed on the T cells and thereby suppresses the T cells' antitumor activity, e.g., antitumor cytokine secretion. Treatment using an effective anti-TIGIT antibody can block the interaction between TIGIT and PVR and thereby enhance the antitumor activity of T cells.

Dendritic cell-T-cell mixed lymphocyte reaction assay (MLR) was used to model this phenomenon in the tumor microenvironment. CD14+ monocytes isolated from PBMCs of one healthy donor were cultured with 50 ng/ml IL-4 and 50 ng/ml GM-CSF in RPMI for 7 days (medium was changed on Day 4) to differentiate into DCs. Mature DCs were obtained by further culturing the DCs with 100 ng/ml LPS in RPMI for 1 day on Day 7. The DCs obtained by such method stably expressed CD11c (mature DC biomarker), MHC class II, CD80 (CD28 ligand), and PVR (TIGIT ligand). CD3+ T cells were isolated from PBMCs of another healthy donor using ThermoFisher T cell isolation kit. Next, the CD3+ T cells (200,000) were mixed with the mature DCs (10,000) in RPMI 1640 culture medium with 10% FBS-HI, and anti-TIGIT antibody or control antibodies were added at various concentrations. After incubation for 48 hours at 37°C and 5.0% CO2, IL-2 secretion from the T cells were measured in culture supernatant by PerkinElmer (AlphaLisa human IL-2 kit: Cat#AL221C). An anti-PD1 antibody was used as a positive control. An anti-HER2 antibody was used as a negative control.

As shown in Figure 11, 2A3-LT-Fc enhanced the IL-2 secretion from T cells in a dose dependent manner comparable to the anti-PD1 antibody. In contrast, Tiragolumab analog, did not enhance the IL-2 secretion from T-cell. As IL-2 is an important antitumor cytokine, the results indicate that the anti-TIGIT antibody 2A3-LT-Fc can significantly enhance T cell antitumor activity in the tumor microenvironment, and it exhibited superior antitumor effects compared to the Tiragolumab analog.

### Example 6 - Fc engineering

The mechanism of action of anti-TIGIT antibodies in enhancing immune function & anti-tumor activity can involve not only blockade effect on TIGIT but also bridging effect between antigen presenting cells (APC) and effective T cells (CD8+ T cells) through the binding of the antibody Fc region by FcyRIIIA on APC and the binding of the VHH domain to TIGIT on effective T cells. To take advantage of this mechanism, two different Fc-enhanced versions were made, one with mutations of DLE (S239D, A330L and I332E), one with mutations of VPVLL (L235V, F243L, R292P, Y300L and P396L). The binding of the Fc-mutants to human FcyRIIIA and FcyRIIB was examined by Octet binding assay using recombinant proteins of the ECD of FcγRIIIA and FcyRIIB, the results of which are shown in Figure 12. Both DLE and VPVLL mutants had enhanced binding affinity to human FcyRIIIA. DLE mutant also had enhanced binding affinity to human FcyRIIB, whereas VPVLL mutant had reduced binding affinity to human FcyRIIB.

The TIGIT blockade activity of a Fc-mutant was also examined, where the DLE mutant showed reduced TIGIT blockade function as shown in Figure 13A.

Moreover, when antibody variable region binds to its specific antigen, its Fc region can cross-link the FcyRIIIA and triggers down-stream signaling. Using this mode of action, the effect of Fc-mutants on FcyRIIIA-mediated activity was examined using a human-FcyRIIIA and NFAT reporter transfected Jurkat cells and 293T cells overexpressing TIGIT. In this assay system, the DLE mutant significantly increased human-FcyRIIIA-mediated NFAT reporter activity compared to the wild type Fc version as shown in Figure 13B.

**Table 3. Test results shown in Figure 13A**

| **EC50 (nM)** | **2A3 LT-Fc** | **DLE** |
|---|---|---|
| Exp 1 | 0.58 | 0.67 |
| Exp 2 | 0.687 | 0.92 |
| Exp 3 | 0.45 | 1.32 |
| Average | 0.57 | 0.97 |
| SD | 0.12 | 0.3279 |

**Table 4. Test results shown in Figure 13B**

| **EC50 (nM)** | **2A3 LT-Fc** | **DLE** |
|---|---|---|
| Exp 1 | 0.226 | 0.027 |
| Exp 2 | 0.175 | 0.0295 |
| Exp 3 | 0.144 | 0.018 |
| Exp 4 | 0.18 | 0.02 |
| Average | 0.18 | 0.024 |
| SD | 0.03 | 0.005 |

### Example 7 - In vivo efficacy study

Using h-TIGIT Knock-In C57BL/6 mice and MC38 murine colon cancer model, efficacies of 2A3-LT-Fc wt and 2A3-LT-Fc-DLE were studied and compared to Reference Ab 2. MC38 tumor cells were implanted one week before treatment. Treatment started when tumor volume reached about 51 mm³, when drugs were dosed intraperitoneally twice a week for 2.5 weeks at 6 mg/kg for 2A3-LT-Fc antibodies, or 11 mg/kg for reference antibody (equal to 6 mg/kg of 2A3-LT-Fc in mole/kg dosage). On day 16 post-treatment, tumor volumes in multiple mice of the control group reached tumor size limits (2000 mm³). Therefore, day 16 post-treatment was the data end point for the analyses. Average tumor volume in the control group was 1548.76±191 mm³ (mean±SEM) on day 16, the treatment with 2A3-LT-Fc wt and 2A3-LT-Fc-DLE significantly reduced tumor growth compared to vehicle control, resulting in respectively 38% and 50% of TGI (tumor growth inhibition) and tumor volume of 985.05±123 mm³ and 802.20±126 mm³ (P=0.037 and P=0.007 compared to Control by Mann-Whitney test), respectively. The treatment with the reference antibody also reduced tumor growth, but the reduction caused by the antibody was not statistically significant compared to the control group with the TGI of 26%, and tumor volume of 1156.16±195 mm³(Figure 14A, Table 5). The results of individual tumor volume are shown in Figure 14B. These results indicated that both 2A3-LT-Fc and 2A3-LT-Fc-DLE were more efficacious than Reference Ab 2. Body weight changes were not significant between the groups throughout the study (Figure 14C), indicating the treatments were well tolerated.

**Table 5. Mean Tumor Volumes on Day 16 in MC38 Syngeneic h-TIGIT Mouse Model**

| **Groups** | **Treatment** | **N** | **TV (mm³)^{a}** | **P^{b}** | **% TGI (Day 16)** |
|---|---|---|---|---|---|
| **1** | **Vehicle** | | **8 1548.76±191** | | |
| **2** | **2A3-LT-Fc-wt** | **8** | **985.05±123** | **0.037** | **38** |
| | **6 mg/kg** | | | | |
| **3** | **2A3-LT-Fc-DLE** | **8** | **802.20±126** | **0.007** | **50** |
| | **6 mg/kg** | | | | |
| **4** | **Reference Ab 2,** | **8** | **1156.16±195** | **0.234** | **26** |
| | **11 mg/kg** | | | | |

| | | | | | |
|---|---|---|---|---|---|
| ^{a} Mean ± SEM ^{b} Compared to vehicle control by Mann-Whitney test on day 16 post-treatment | | | | | |

In addition to the various embodiments depicted and claimed, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the compositions and methods of the disclosed subject matter without departing from the spirit or scope of the disclosed subject matter. Thus, it is intended that the disclosed subject matter include modifications and variations that are within the scope of the appended claims and their equivalents.

Various publications, patents and patent applications are cited herein, the contents of which are hereby incorporated by reference in their entireties.

## Claims

1. An antibody that binds to TIGIT, comprising a single domain antibody that binds to TIGIT with a KD of 1×10⁻⁷ M or less.

2. The antibody of claim 1, wherein the single domain antibody binds to TIGIT with a KD of 1×10⁻⁸ M or less.

3. The antibody of claim 1 or 2, wherein the single domain antibody binds to TIGIT with a KD of 5×10⁻⁹ M or less.

4. The antibody of any one of claims 1-3, wherein the single domain antibody binds to TIGIT with a KD of 2×10⁻⁹ M or less.

5. The antibody of any one of claims 1-4, wherein the single domain antibody comprises a VHH.

6. The antibody of any one of claims 1-5, wherein the single domain antibody or the VHH comprises a heavy chain variable region (VH).

7. The antibody of any one of claims 1-6, wherein the single domain antibody cross-competes for binding to TIGIT with a reference anti-TIGIT single domain antibody comprising a heavy chain variable region comprising:
a) a heavy chain variable region CDR1 comprising amino acids having the sequence set forth in SEQ ID NO: 94, a heavy chain variable region CDR2 comprising amino acids having the sequence set forth in SEQ ID NO: 95, and a heavy chain variable region CDR3 comprising amino acids having the sequence set forth in SEQ ID NO: 96,
b) a heavy chain variable region CDR1 comprising amino acids having the sequence set forth in SEQ ID NO: 98, a heavy chain variable region CDR2 comprising amino acids having the sequence set forth in SEQ ID NO: 99, and a heavy chain variable region CDR3 comprising amino acids having the sequence set forth in SEQ ID NO: 100,
c) a heavy chain variable region CDR1 comprising amino acids having the sequence set forth in SEQ ID NO: 102, a heavy chain variable region CDR2 comprising amino acids having the sequence set forth in SEQ ID NO: 103, and a heavy chain variable region CDR3 comprising amino acids having the sequence set forth in SEQ ID NO: 104,
d) a heavy chain variable region CDR1 comprising amino acids having the sequence set forth in SEQ ID NO: 106, a heavy chain variable region CDR2 comprising amino acids having the sequence set forth in SEQ ID NO: 107, and a heavy chain variable region CDR3 comprising amino acids having the sequence set forth in SEQ ID NO: 108,
e) a heavy chain variable region CDR1 comprising amino acids having the sequence set forth in SEQ ID NO: 110, a heavy chain variable region CDR2 comprising amino acids having the sequence set forth in SEQ ID NO: 111, and a heavy chain variable region CDR3 comprising amino acids having the sequence set forth in SEQ ID NO: 112,
f) a heavy chain variable region CDR1 comprising amino acids having the sequence set forth in SEQ ID NO: 114, a heavy chain variable region CDR2 comprising amino acids having the sequence set forth in SEQ ID NO: 115, and a heavy chain variable region CDR3 comprising amino acids having the sequence set forth in SEQ ID NO: 116,
g) a heavy chain variable region CDR1 comprising amino acids having the sequence set forth in SEQ ID NO: 118, a heavy chain variable region CDR2 comprising amino acids having the sequence set forth in SEQ ID NO: 119, and a heavy chain variable region CDR3 comprising amino acids having the sequence set forth in SEQ ID NO: 120,
h) a heavy chain variable region CDR1 comprising amino acids having the sequence set forth in SEQ ID NO: 122, a heavy chain variable region CDR2 comprising amino acids having the sequence set forth in SEQ ID NO: 123, and a heavy chain variable region CDR3 comprising amino acids having the sequence set forth in SEQ ID NO: 124,
i) a heavy chain variable region CDR1 comprising amino acids having the sequence set forth in SEQ ID NO: 126, a heavy chain variable region CDR2 comprising amino acids having the sequence set forth in SEQ ID NO: 127, and a heavy chain variable region CDR3 comprising amino acids having the sequence set forth in SEQ ID NO: 128,
j) a heavy chain variable region CDR1 comprising amino acids having the sequence set forth in SEQ ID NO: 130, a heavy chain variable region CDR2 comprising amino acids having the sequence set forth in SEQ ID NO: 131, and a heavy chain variable region CDR3 comprising amino acids having the sequence set forth in SEQ ID NO: 132,
k) a heavy chain variable region CDR1 comprising amino acids having the sequence set forth in SEQ ID NO: 134, a heavy chain variable region CDR2 comprising amino acids having the sequence set forth in SEQ ID NO: 135, and a heavy chain variable region CDR3 comprising amino acids having the sequence set forth in SEQ ID NO: 136,
l) a heavy chain variable region CDR1 comprising amino acids having the sequence set forth in SEQ ID NO: 138, a heavy chain variable region CDR2 comprising amino acids having the sequence set forth in SEQ ID NO: 139, and a heavy chain variable region CDR3 comprising amino acids having the sequence set forth in SEQ ID NO: 140,
m) a heavy chain variable region CDR1 comprising amino acids having the sequence set forth in SEQ ID NO: 142, a heavy chain variable region CDR2 comprising amino acids having the sequence set forth in SEQ ID NO: 143, and a heavy chain variable region CDR3 comprising amino acids having the sequence set forth in SEQ ID NO: 144,
n) a heavy chain variable region CDR1 comprising amino acids having the sequence set forth in SEQ ID NO: 146, a heavy chain variable region CDR2 comprising amino acids having the sequence set forth in SEQ ID NO: 147, and a heavy chain variable region CDR3 comprising amino acids having the sequence set forth in SEQ ID NO: 148,
o) a heavy chain variable region CDR1 comprising amino acids having the sequence set forth in SEQ ID NO: 150, a heavy chain variable region CDR2 comprising amino acids having the sequence set forth in SEQ ID NO: 151, and a heavy chain variable region CDR3 comprising amino acids having the sequence set forth in SEQ ID NO: 152,
p) a heavy chain variable region CDR1 comprising amino acids having the sequence set forth in SEQ ID NO: 154, a heavy chain variable region CDR2 comprising amino acids having the sequence set forth in SEQ ID NO: 155, and a heavy chain variable region CDR3 comprising amino acids having the sequence set forth in SEQ ID NO: 156,
q) a heavy chain variable region CDR1 comprising amino acids having the sequence set forth in SEQ ID NO: 158, a heavy chain variable region CDR2 comprising amino acids having the sequence set forth in SEQ ID NO: 159, and a heavy chain variable region CDR3 comprising amino acids having the sequence set forth in SEQ ID NO: 160,
r) a heavy chain variable region CDR1 comprising amino acids having the sequence set forth in SEQ ID NO: 162, a heavy chain variable region CDR2 comprising amino acids having the sequence set forth in SEQ ID NO: 163, and a heavy chain variable region CDR3 comprising amino acids having the sequence set forth in SEQ ID NO: 164,
s) a heavy chain variable region CDR1 comprising amino acids having the sequence set forth in SEQ ID NO: 166, a heavy chain variable region CDR2 comprising amino acids having the sequence set forth in SEQ ID NO: 167, and a heavy chain variable region CDR3 comprising amino acids having the sequence set forth in SEQ ID NO: 168,
t) a heavy chain variable region CDR1 comprising amino acids having the sequence set forth in SEQ ID NO: 170, a heavy chain variable region CDR2 comprising amino acids having the sequence set forth in SEQ ID NO: 171, and a heavy chain variable region CDR3 comprising amino acids having the sequence set forth in SEQ ID NO: 172,
u) a heavy chain variable region CDR1 comprising amino acids having the sequence set forth in SEQ ID NO: 174, a heavy chain variable region CDR2 comprising amino acids having the sequence set forth in SEQ ID NO: 175, and a heavy chain variable region CDR3 comprising amino acids having the sequence set forth in SEQ ID NO: 176,
v) a heavy chain variable region CDR1 comprising amino acids having the sequence set forth in SEQ ID NO: 178, a heavy chain variable region CDR2 comprising amino acids having the sequence set forth in SEQ ID NO: 179, and a heavy chain variable region CDR3 comprising amino acids having the sequence set forth in SEQ ID NO: 180,
w) a heavy chain variable region CDR1 comprising amino acids having the sequence set forth in SEQ ID NO: 182, a heavy chain variable region CDR2 comprising amino acids having the sequence set forth in SEQ ID NO: 183, and a heavy chain variable region CDR3 comprising amino acids having the sequence set forth in SEQ ID NO: 184,
x) a heavy chain variable region CDR1 comprising amino acids having the sequence set forth in SEQ ID NO: 186, a heavy chain variable region CDR2 comprising amino acids having the sequence set forth in SEQ ID NO: 187, and a heavy chain variable region CDR3 comprising amino acids having the sequence set forth in SEQ ID NO: 188, or
y) a heavy chain variable region CDR1 comprising amino acids having the sequence set forth in SEQ ID NO: 190, a heavy chain variable region CDR2 comprising amino acids having the sequence set forth in SEQ ID NO: 191, and a heavy chain variable region CDR3 comprising amino acids having the sequence set forth in SEQ ID NO: 192.

8. The antibody of any one of claims 1-7, wherein the single domain antibody comprises a heavy chain variable region comprising:
a) a heavy chain variable region CDR1 comprising an amino acid sequence of any one of SEQ ID NOs: 94, 98, 102, 106, 110, 114, 118, 122, 126, 130, 134, 138, 142, 146, 150, 154, 158, 162, 166, 170, 174, 178, 182, 186 and 190, or a variant thereof comprising up to about 3 amino acid substitutions;
b) a heavy chain variable region CDR2 comprising an amino acid sequence of any one of SEQ ID NOs: 95, 99, 103, 107, 111, 115, 119, 123, 127, 131, 135, 139, 143, 147, 151, 155, 159, 163, 167, 171, 175, 179, 183, 187 and 191, or a variant thereof comprising up to about 3 amino acid substitutions; and
c) a heavy chain variable region CDR3 comprising an amino acid sequence of anyone of SEQ ID NOs: 96, 100, 104, 108, 112, 116, 120, 124, 128, 132, 136, 140, 144, 148, 152, 156, 160, 164, 168, 172, 176, 180, 184, 188 and 192, or a variant thereof comprising up to about 3 amino acid substitutions.

9. The antibody of any one of claims 1-8, wherein the single domain antibody comprises a heavy chain variable region that comprises a CDR1 domain, a CDR2 domain and a CDR3 domain, wherein the CDR1 domain, the CDR2 domain and the CDR3 domain respectively comprise a CDR1 domain, a CDR2 domain and a CDR3 domain comprised in a reference heavy chain variable region comprising the amino acid sequence selected from the group consisting of SEQ ID NOs: 97, 101, 105, 109, 113, 117, 121, 125, 129, 133, 137, 141, 145, 149, 153, 157, 161, 165, 169, 173, 177, 181, 185, 189 and 193.

10. The antibody of any one of claims 1-9, wherein the single domain antibody comprises a heavy chain variable region CDR1 comprising amino acids having the sequence set forth in SEQ ID NO: 94, a heavy chain variable region CDR2 comprising amino acids having the sequence set forth in SEQ ID NO: 95, and a heavy chain variable region CDR3 comprising amino acids having the sequence set forth in SEQ ID NO: 96.

11. The antibody of any one of claims 1-9, wherein the single domain antibody comprises a heavy chain variable region CDR1 comprising amino acids having the sequence set forth in SEQ ID NO: 98, a heavy chain variable region CDR2 comprising amino acids having the sequence set forth in SEQ ID NO: 99, and a heavy chain variable region CDR3 comprising amino acids having the sequence set forth in SEQ ID NO: 100.

12. The antibody of any one of claims 1-9, wherein the single domain antibody comprises a heavy chain variable region CDR1 comprising amino acids having the sequence set forth in SEQ ID NO: 102, a heavy chain variable region CDR2 comprising amino acids having the sequence set forth in SEQ ID NO: 103, and a heavy chain variable region CDR3 comprising amino acids having the sequence set forth in SEQ ID NO: 104.

13. The antibody of any one of claims 1-9, wherein the single domain antibody comprises a heavy chain variable region CDR1 comprising amino acids having the sequence set forth in SEQ ID NO: 106, a heavy chain variable region CDR2 comprising amino acids having the sequence set forth in SEQ ID NO: 107, and a heavy chain variable region CDR3 comprising amino acids having the sequence set forth in SEQ ID NO: 108.

14. The antibody of any one of claims 1-9, wherein the single domain antibody comprises a heavy chain variable region CDR1 comprising amino acids having the sequence set forth in SEQ ID NO: 110, a heavy chain variable region CDR2 comprising amino acids having the sequence set forth in SEQ ID NO: 111, and a heavy chain variable region CDR3 comprising amino acids having the sequence set forth in SEQ ID NO: 112.

15. The antibody of any one of claims 1-9, wherein the single domain antibody comprises a heavy chain variable region CDR1 comprising amino acids having the sequence set forth in SEQ ID NO: 114, a heavy chain variable region CDR2 comprising amino acids having the sequence set forth in SEQ ID NO: 115, and a heavy chain variable region CDR3 comprising amino acids having the sequence set forth in SEQ ID NO: 116.

16. The antibody of any one of claims 1-9, wherein the single domain antibody comprises a heavy chain variable region CDR1 comprising amino acids having the sequence set forth in SEQ ID NO: 118, a heavy chain variable region CDR2 comprising amino acids having the sequence set forth in SEQ ID NO: 119, and a heavy chain variable region CDR3 comprising amino acids having the sequence set forth in SEQ ID NO: 120.

17. The antibody of any one of claims 1-9, wherein the single domain antibody comprises a heavy chain variable region CDR1 comprising amino acids having the sequence set forth in SEQ ID NO: 122, a heavy chain variable region CDR2 comprising amino acids having the sequence set forth in SEQ ID NO: 123, and a heavy chain variable region CDR3 comprising amino acids having the sequence set forth in SEQ ID NO: 124.

18. The antibody of any one of claims 1-9, wherein the single domain antibody comprises a heavy chain variable region CDR1 comprising amino acids having the sequence set forth in SEQ ID NO: 126, a heavy chain variable region CDR2 comprising amino acids having the sequence set forth in SEQ ID NO: 127, and a heavy chain variable region CDR3 comprising amino acids having the sequence set forth in SEQ ID NO: 128.

19. The antibody of any one of claims 1-9, wherein the single domain antibody comprises a heavy chain variable region CDR1 comprising amino acids having the sequence set forth in SEQ ID NO: 130, a heavy chain variable region CDR2 comprising amino acids having the sequence set forth in SEQ ID NO: 131, and a heavy chain variable region CDR3 comprising amino acids having the sequence set forth in SEQ ID NO: 132.

20. The antibody of any one of claims 1-9, wherein the single domain antibody comprises a heavy chain variable region CDR1 comprising amino acids having the sequence set forth in SEQ ID NO: 134, a heavy chain variable region CDR2 comprising amino acids having the sequence set forth in SEQ ID NO: 135, and a heavy chain variable region CDR3 comprising amino acids having the sequence set forth in SEQ ID NO: 136.

21. The antibody of any one of claims 1-9, wherein the single domain antibody comprises a heavy chain variable region CDR1 comprising amino acids having the sequence set forth in SEQ ID NO: 138, a heavy chain variable region CDR2 comprising amino acids having the sequence set forth in SEQ ID NO: 139, and a heavy chain variable region CDR3 comprising amino acids having the sequence set forth in SEQ ID NO: 140.

22. The antibody of any one of claims 1-9, wherein the single domain antibody comprises a heavy chain variable region CDR1 comprising amino acids having the sequence set forth in SEQ ID NO: 142, a heavy chain variable region CDR2 comprising amino acids having the sequence set forth in SEQ ID NO: 143, and a heavy chain variable region CDR3 comprising amino acids having the sequence set forth in SEQ ID NO: 144.

23. The antibody of any one of claims 1-9, wherein the single domain antibody comprises a heavy chain variable region CDR1 comprising amino acids having the sequence set forth in SEQ ID NO: 146, a heavy chain variable region CDR2 comprising amino acids having the sequence set forth in SEQ ID NO: 147, and a heavy chain variable region CDR3 comprising amino acids having the sequence set forth in SEQ ID NO: 148.

24. The antibody of any one of claims 1-9, wherein the single domain antibody comprises a heavy chain variable region CDR1 comprising amino acids having the sequence set forth in SEQ ID NO: 150, a heavy chain variable region CDR2 comprising amino acids having the sequence set forth in SEQ ID NO: 151, and a heavy chain variable region CDR3 comprising amino acids having the sequence set forth in SEQ ID NO: 152.

25. The antibody of any one of claims 1-9, wherein the single domain antibody comprises a heavy chain variable region CDR1 comprising amino acids having the sequence set forth in SEQ ID NO: 154, a heavy chain variable region CDR2 comprising amino acids having the sequence set forth in SEQ ID NO: 155, and a heavy chain variable region CDR3 comprising amino acids having the sequence set forth in SEQ ID NO: 156.

26. The antibody of any one of claims 1-9, wherein the single domain antibody comprises a heavy chain variable region CDR1 comprising amino acids having the sequence set forth in SEQ ID NO: 158, a heavy chain variable region CDR2 comprising amino acids having the sequence set forth in SEQ ID NO: 159, and a heavy chain variable region CDR3 comprising amino acids having the sequence set forth in SEQ ID NO: 160.

27. The antibody of any one of claims 1-9, wherein the single domain antibody comprises a heavy chain variable region CDR1 comprising amino acids having the sequence set forth in SEQ ID NO: 162, a heavy chain variable region CDR2 comprising amino acids having the sequence set forth in SEQ ID NO: 163, and a heavy chain variable region CDR3 comprising amino acids having the sequence set forth in SEQ ID NO: 164.

28. The antibody of any one of claims 1-9, wherein the single domain antibody comprises a heavy chain variable region CDR1 comprising amino acids having the sequence set forth in SEQ ID NO: 166, a heavy chain variable region CDR2 comprising amino acids having the sequence set forth in SEQ ID NO: 167, and a heavy chain variable region CDR3 comprising amino acids having the sequence set forth in SEQ ID NO: 168.

29. The antibody of any one of claims 1-9, wherein the single domain antibody comprises a heavy chain variable region CDR1 comprising amino acids having the sequence set forth in SEQ ID NO: 170, a heavy chain variable region CDR2 comprising amino acids having the sequence set forth in SEQ ID NO: 171, and a heavy chain variable region CDR3 comprising amino acids having the sequence set forth in SEQ ID NO: 172.

30. The antibody of any one of claims 1-9, wherein the single domain antibody comprises a heavy chain variable region CDR1 comprising amino acids having the sequence set forth in SEQ ID NO: 174, a heavy chain variable region CDR2 comprising amino acids having the sequence set forth in SEQ ID NO: 175, and a heavy chain variable region CDR3 comprising amino acids having the sequence set forth in SEQ ID NO: 176.

31. The antibody of any one of claims 1-9, wherein the single domain antibody comprises a heavy chain variable region CDR1 comprising amino acids having the sequence set forth in SEQ ID NO: 178, a heavy chain variable region CDR2 comprising amino acids having the sequence set forth in SEQ ID NO: 179, and a heavy chain variable region CDR3 comprising amino acids having the sequence set forth in SEQ ID NO: 180.

32. The antibody of any one of claims 1-9, wherein the single domain antibody comprises a heavy chain variable region CDR1 comprising amino acids having the sequence set forth in SEQ ID NO: 182, a heavy chain variable region CDR2 comprising amino acids having the sequence set forth in SEQ ID NO: 183, and a heavy chain variable region CDR3 comprising amino acids having the sequence set forth in SEQ ID NO: 184.

33. The antibody of any one of claims 1-9, wherein the single domain antibody comprises a heavy chain variable region CDR1 comprising amino acids having the sequence set forth in SEQ ID NO: 186, a heavy chain variable region CDR2 comprising amino acids having the sequence set forth in SEQ ID NO: 187, and a heavy chain variable region CDR3 comprising amino acids having the sequence set forth in SEQ ID NO: 188.

34. The antibody of any one of claims 1-9, wherein the single domain antibody comprises a heavy chain variable region CDR1 comprising amino acids having the sequence set forth in SEQ ID NO: 190, a heavy chain variable region CDR2 comprising amino acids having the sequence set forth in SEQ ID NO: 191, and a heavy chain variable region CDR3 comprising amino acids having the sequence set forth in SEQ ID NO: 192.

35. The antibody of any one of claims 1-34, wherein the single domain antibody comprises a heavy chain variable region comprising an amino acid sequence having at least about 90% sequence identity to an amino acid sequence selected from the group consisting of SEQ ID NOs: 97, 101, 105, 109, 113, 117, 121, 125, 129, 133, 137, 141, 145, 149, 153, 157, 161, 165, 169, 173, 177, 181, 185, 189 and 193.

36. The antibody of any one of claims 1-35, wherein the single domain antibody comprises a heavy chain variable region comprising the amino acid sequence set forth in SEQ ID NO: 97.

37. The antibody of any one of claims 1-35, wherein the single domain antibody comprises a heavy chain variable region comprising the amino acid sequence set forth in SEQ ID NO: 101.

38. The antibody of any one of claims 1-35, wherein the single domain antibody comprises a heavy chain variable region comprising the amino acid sequence set forth in SEQ ID NO: 105.

39. The antibody of any one of claims 1-35, wherein the single domain antibody comprises a heavy chain variable region comprising the amino acid sequence set forth in SEQ ID NO: 109.

40. The antibody of any one of claims 1-35, wherein the single domain antibody comprises a heavy chain variable region comprising the amino acid sequence set forth in SEQ ID NO: 113.

41. The antibody of any one of claims 1-35, wherein the single domain antibody comprises a heavy chain variable region comprising the amino acid sequence set forth in SEQ ID NO: 117.

42. The antibody of any one of claims 1-35, wherein the single domain antibody comprises a heavy chain variable region comprising the amino acid sequence set forth in SEQ ID NO: 121.

43. The antibody of any one of claims 1-35, wherein the single domain antibody comprises a heavy chain variable region comprising the amino acid sequence set forth in SEQ ID NO: 125.

44. The antibody of any one of claims 1-35, wherein the single domain antibody comprises a heavy chain variable region comprising the amino acid sequence set forth in SEQ ID NO: 129.

45. The antibody of any one of claims 1-35, wherein the single domain antibody comprises a heavy chain variable region comprising the amino acid sequence set forth in SEQ ID NO: 133.

46. The antibody of any one of claims 1-35, wherein the single domain antibody comprises a heavy chain variable region comprising the amino acid sequence set forth in SEQ ID NO: 137.

47. The antibody of any one of claims 1-35, wherein the single domain antibody comprises a heavy chain variable region comprising the amino acid sequence set forth in SEQ ID NO: 141.

48. The antibody of any one of claims 1-35, wherein the single domain antibody comprises a heavy chain variable region comprising the amino acid sequence set forth in SEQ ID NO: 145.

49. The antibody of any one of claims 1-35, wherein the single domain antibody comprises a heavy chain variable region comprising the amino acid sequence set forth in SEQ ID NO: 149.

50. The antibody of any one of claims 1-35, wherein the single domain antibody comprises a heavy chain variable region comprising the amino acid sequence set forth in SEQ ID NO: 153.

51. The antibody of any one of claims 1-35, wherein the single domain antibody comprises a heavy chain variable region comprising the amino acid sequence set forth in SEQ ID NO: 157.

52. The antibody of any one of claims 1-35, wherein the single domain antibody comprises a heavy chain variable region comprising the amino acid sequence set forth in SEQ ID NO: 161.

53. The antibody of any one of claims 1-35, wherein the single domain antibody comprises a heavy chain variable region comprising the amino acid sequence set forth in SEQ ID NO: 165.

54. The antibody of any one of claims 1-35, wherein the single domain antibody comprises a heavy chain variable region comprising the amino acid sequence set forth in SEQ ID NO: 169.

55. The antibody of any one of claims 1-35, wherein the single domain antibody comprises a heavy chain variable region comprising the amino acid sequence set forth in SEQ ID NO: 173.

56. The antibody of any one of claims 1-35, wherein the single domain antibody comprises a heavy chain variable region comprising the amino acid sequence set forth in SEQ ID NO: 177.

57. The antibody of any one of claims 1-35, wherein the single domain antibody comprises a heavy chain variable region comprising the amino acid sequence set forth in SEQ ID NO: 181.

58. The antibody of any one of claims 1-35, wherein the single domain antibody comprises a heavy chain variable region comprising the amino acid sequence set forth in SEQ ID NO: 185.

59. The antibody of any one of claims 1-35, wherein the single domain antibody comprises a heavy chain variable region comprising the amino acid sequence set forth in SEQ ID NO: 189.

60. The antibody of any one of claims 1-35, wherein the single domain antibody comprises a heavy chain variable region comprising the amino acid sequence set forth in SEQ ID NO: 193.

61. The antibody of any one of claims 1-60, wherein the single domain antibody cross-competes for binding to TIGIT with a reference anti-TIGIT single domain antibody comprising a heavy chain variable region comprising:
(a) a heavy chain variable region CDR1 comprising amino acids having the sequence set forth in SEQ ID NO: 1, a heavy chain variable region CDR2 comprising amino acids having the sequence set forth in SEQ ID NO: 2, and a heavy chain variable region CDR3 comprising amino acids having the sequence set forth in SEQ ID NO: 3,
(b) a heavy chain variable region CDR1 comprising amino acids having the sequence set forth in SEQ ID NO: 5, a heavy chain variable region CDR2 comprising amino acids having the sequence set forth in SEQ ID NO: 6, and a heavy chain variable region CDR3 comprising amino acids having the sequence set forth in SEQ ID NO: 7,
(c) a heavy chain variable region CDR1 comprising amino acids having the sequence set forth in SEQ ID NO: 9, a heavy chain variable region CDR2 comprising amino acids having the sequence set forth in SEQ ID NO: 10, and a heavy chain variable region CDR3 comprising amino acids having the sequence set forth in SEQ ID NO: 11,
(d) a heavy chain variable region CDR1 comprising amino acids having the sequence set forth in SEQ ID NO: 13, a heavy chain variable region CDR2 comprising amino acids having the sequence set forth in SEQ ID NO: 14, and a heavy chain variable region CDR3 comprising amino acids having the sequence set forth in SEQ ID NO: 15,
(e) a heavy chain variable region CDR1 comprising amino acids having the sequence set forth in SEQ ID NO: 17, a heavy chain variable region CDR2 comprising amino acids having the sequence set forth in SEQ ID NO: 18, and a heavy chain variable region CDR3 comprising amino acids having the sequence set forth in SEQ ID NO: 19,
(f) a heavy chain variable region CDR1 comprising amino acids having the sequence set forth in SEQ ID NO: 21, a heavy chain variable region CDR2 comprising amino acids having the sequence set forth in SEQ ID NO: 22, and a heavy chain variable region CDR3 comprising amino acids having the sequence set forth in SEQ ID NO: 23,
(g) a heavy chain variable region CDR1 comprising amino acids having the sequence set forth in SEQ ID NO: 25, a heavy chain variable region CDR2 comprising amino acids having the sequence set forth in SEQ ID NO: 26, and a heavy chain variable region CDR3 comprising amino acids having the sequence set forth in SEQ ID NO: 27,
(h) a heavy chain variable region CDR1 comprising amino acids having the sequence set forth in SEQ ID NO: 29, a heavy chain variable region CDR2 comprising amino acids having the sequence set forth in SEQ ID NO: 30, and a heavy chain variable region CDR3 comprising amino acids having the sequence set forth in SEQ ID NO: 31,
(i) a heavy chain variable region CDR1 comprising amino acids having the sequence set forth in SEQ ID NO: 33, a heavy chain variable region CDR2 comprising amino acids having the sequence set forth in SEQ ID NO: 34, and a heavy chain variable region CDR3 comprising amino acids having the sequence set forth in SEQ ID NO: 35,
(j) a heavy chain variable region CDR1 comprising amino acids having the sequence set forth in SEQ ID NO: 37, a heavy chain variable region CDR2 comprising amino acids having the sequence set forth in SEQ ID NO: 38, and a heavy chain variable region CDR3 comprising amino acids having the sequence set forth in SEQ ID NO: 39,
(k) a heavy chain variable region CDR1 comprising amino acids having the sequence set forth in SEQ ID NO: 41, a heavy chain variable region CDR2 comprising amino acids having the sequence set forth in SEQ ID NO: 42, and a heavy chain variable region CDR3 comprising amino acids having the sequence set forth in SEQ ID NO: 43,
(l) a heavy chain variable region CDR1 comprising amino acids having the sequence set forth in SEQ ID NO: 45, a heavy chain variable region CDR2 comprising amino acids having the sequence set forth in SEQ ID NO: 46, and a heavy chain variable region CDR3 comprising amino acids having the sequence set forth in SEQ ID NO: 47,
(m) a heavy chain variable region CDR1 comprising amino acids having the sequence set forth in SEQ ID NO: 49, a heavy chain variable region CDR2 comprising amino acids having the sequence set forth in SEQ ID NO: 50, and a heavy chain variable region CDR3 comprising amino acids having the sequence set forth in SEQ ID NO: 51,
(n) a heavy chain variable region CDR1 comprising amino acids having the sequence set forth in SEQ ID NO: 53, a heavy chain variable region CDR2 comprising amino acids having the sequence set forth in SEQ ID NO: 54, and a heavy chain variable region CDR3 comprising amino acids having the sequence set forth in SEQ ID NO: 55,
(o) a heavy chain variable region CDR1 comprising amino acids having the sequence set forth in SEQ ID NO: 57, a heavy chain variable region CDR2 comprising amino acids having the sequence set forth in SEQ ID NO: 58, and a heavy chain variable region CDR3 comprising amino acids having the sequence set forth in SEQ ID NO: 59,
(p) a heavy chain variable region CDR1 comprising amino acids having the sequence set forth in SEQ ID NO: 61, a heavy chain variable region CDR2 comprising amino acids having the sequence set forth in SEQ ID NO: 62, and a heavy chain variable region CDR3 comprising amino acids having the sequence set forth in SEQ ID NO: 63,
(q) a heavy chain variable region CDR1 comprising amino acids having the sequence set forth in SEQ ID NO: 65, a heavy chain variable region CDR2 comprising amino acids having the sequence set forth in SEQ ID NO: 66, and a heavy chain variable region CDR3 comprising amino acids having the sequence set forth in SEQ ID NO: 67,
(r) a heavy chain variable region CDR1 comprising amino acids having the sequence set forth in SEQ ID NO: 69, a heavy chain variable region CDR2 comprising amino acids having the sequence set forth in SEQ ID NO: 70, and a heavy chain variable region CDR3 comprising amino acids having the sequence set forth in SEQ ID NO: 71,
(s) a heavy chain variable region CDR1 comprising amino acids having the sequence set forth in SEQ ID NO: 73, a heavy chain variable region CDR2 comprising amino acids having the sequence set forth in SEQ ID NO: 74, and a heavy chain variable region CDR3 comprising amino acids having the sequence set forth in SEQ ID NO: 75,
(t) a heavy chain variable region CDR1 comprising amino acids having the sequence set forth in SEQ ID NO: 77, a heavy chain variable region CDR2 comprising amino acids having the sequence set forth in SEQ ID NO: 78, and a heavy chain variable region CDR3 comprising amino acids having the sequence set forth in SEQ ID NO: 79, or
(u) a heavy chain variable region CDR1 comprising amino acids having the sequence set forth in SEQ ID NO: 81, a heavy chain variable region CDR2 comprising amino acids having the sequence set forth in SEQ ID NO: 82, and a heavy chain variable region CDR3 comprising amino acids having the sequence set forth in SEQ ID NO: 83.

62. The antibody of any one of claims 1-61, wherein the single domain antibody comprises a heavy chain variable region comprising:
(a) a heavy chain variable region CDR1 comprising amino acids having the sequence set forth in SEQ ID NO: 1, a heavy chain variable region CDR2 comprising amino acids having the sequence set forth in SEQ ID NO: 2, and a heavy chain variable region CDR3 comprising amino acids having the sequence set forth in SEQ ID NO: 3,
(b) a heavy chain variable region CDR1 comprising amino acids having the sequence set forth in SEQ ID NO: 5, a heavy chain variable region CDR2 comprising amino acids having the sequence set forth in SEQ ID NO: 6, and a heavy chain variable region CDR3 comprising amino acids having the sequence set forth in SEQ ID NO: 7,
(c) a heavy chain variable region CDR1 comprising amino acids having the sequence set forth in SEQ ID NO: 9, a heavy chain variable region CDR2 comprising amino acids having the sequence set forth in SEQ ID NO: 10, and a heavy chain variable region CDR3 comprising amino acids having the sequence set forth in SEQ ID NO: 11,
(d) a heavy chain variable region CDR1 comprising amino acids having the sequence set forth in SEQ ID NO: 13, a heavy chain variable region CDR2 comprising amino acids having the sequence set forth in SEQ ID NO: 14, and a heavy chain variable region CDR3 comprising amino acids having the sequence set forth in SEQ ID NO: 15,
(e) a heavy chain variable region CDR1 comprising amino acids having the sequence set forth in SEQ ID NO: 17, a heavy chain variable region CDR2 comprising amino acids having the sequence set forth in SEQ ID NO: 18, and a heavy chain variable region CDR3 comprising amino acids having the sequence set forth in SEQ ID NO: 19,
(f) a heavy chain variable region CDR1 comprising amino acids having the sequence set forth in SEQ ID NO: 21, a heavy chain variable region CDR2 comprising amino acids having the sequence set forth in SEQ ID NO: 22, and a heavy chain variable region CDR3 comprising amino acids having the sequence set forth in SEQ ID NO: 23,
(g) a heavy chain variable region CDR1 comprising amino acids having the sequence set forth in SEQ ID NO: 25, a heavy chain variable region CDR2 comprising amino acids having the sequence set forth in SEQ ID NO: 26, and a heavy chain variable region CDR3 comprising amino acids having the sequence set forth in SEQ ID NO: 27,
(h) a heavy chain variable region CDR1 comprising amino acids having the sequence set forth in SEQ ID NO: 29, a heavy chain variable region CDR2 comprising amino acids having the sequence set forth in SEQ ID NO: 30, and a heavy chain variable region CDR3 comprising amino acids having the sequence set forth in SEQ ID NO: 31,
(i) a heavy chain variable region CDR1 comprising amino acids having the sequence set forth in SEQ ID NO: 33, a heavy chain variable region CDR2 comprising amino acids having the sequence set forth in SEQ ID NO: 34, and a heavy chain variable region CDR3 comprising amino acids having the sequence set forth in SEQ ID NO: 35,
(j) a heavy chain variable region CDR1 comprising amino acids having the sequence set forth in SEQ ID NO: 37, a heavy chain variable region CDR2 comprising amino acids having the sequence set forth in SEQ ID NO: 38, and a heavy chain variable region CDR3 comprising amino acids having the sequence set forth in SEQ ID NO: 39,
(k) a heavy chain variable region CDR1 comprising amino acids having the sequence set forth in SEQ ID NO: 41, a heavy chain variable region CDR2 comprising amino acids having the sequence set forth in SEQ ID NO: 42, and a heavy chain variable region CDR3 comprising amino acids having the sequence set forth in SEQ ID NO: 43,
(l) a heavy chain variable region CDR1 comprising amino acids having the sequence set forth in SEQ ID NO: 45, a heavy chain variable region CDR2 comprising amino acids having the sequence set forth in SEQ ID NO: 46, and a heavy chain variable region CDR3 comprising amino acids having the sequence set forth in SEQ ID NO: 47,
(m) a heavy chain variable region CDR1 comprising amino acids having the sequence set forth in SEQ ID NO: 49, a heavy chain variable region CDR2 comprising amino acids having the sequence set forth in SEQ ID NO: 50, and a heavy chain variable region CDR3 comprising amino acids having the sequence set forth in SEQ ID NO: 51,
(n) a heavy chain variable region CDR1 comprising amino acids having the sequence set forth in SEQ ID NO: 53, a heavy chain variable region CDR2 comprising amino acids having the sequence set forth in SEQ ID NO: 54, and a heavy chain variable region CDR3 comprising amino acids having the sequence set forth in SEQ ID NO: 55,
(o) a heavy chain variable region CDR1 comprising amino acids having the sequence set forth in SEQ ID NO: 57, a heavy chain variable region CDR2 comprising amino acids having the sequence set forth in SEQ ID NO: 58, and a heavy chain variable region CDR3 comprising amino acids having the sequence set forth in SEQ ID NO: 59,
(p) a heavy chain variable region CDR1 comprising amino acids having the sequence set forth in SEQ ID NO: 61, a heavy chain variable region CDR2 comprising amino acids having the sequence set forth in SEQ ID NO: 62, and a heavy chain variable region CDR3 comprising amino acids having the sequence set forth in SEQ ID NO: 63,
(q) a heavy chain variable region CDR1 comprising amino acids having the sequence set forth in SEQ ID NO: 65, a heavy chain variable region CDR2 comprising amino acids having the sequence set forth in SEQ ID NO: 66, and a heavy chain variable region CDR3 comprising amino acids having the sequence set forth in SEQ ID NO: 67,
(r) a heavy chain variable region CDR1 comprising amino acids having the sequence set forth in SEQ ID NO: 69, a heavy chain variable region CDR2 comprising amino acids having the sequence set forth in SEQ ID NO: 70, and a heavy chain variable region CDR3 comprising amino acids having the sequence set forth in SEQ ID NO: 71,
(s) a heavy chain variable region CDR1 comprising amino acids having the sequence set forth in SEQ ID NO: 73, a heavy chain variable region CDR2 comprising amino acids having the sequence set forth in SEQ ID NO: 74, and a heavy chain variable region CDR3 comprising amino acids having the sequence set forth in SEQ ID NO: 75,
(t) a heavy chain variable region CDR1 comprising amino acids having the sequence set forth in SEQ ID NO: 77, a heavy chain variable region CDR2 comprising amino acids having the sequence set forth in SEQ ID NO: 78, and a heavy chain variable region CDR3 comprising amino acids having the sequence set forth in SEQ ID NO: 79, or
(u) a heavy chain variable region CDR1 comprising amino acids having the sequence set forth in SEQ ID NO: 81, a heavy chain variable region CDR2 comprising amino acids having the sequence set forth in SEQ ID NO: 82, and a heavy chain variable region CDR3 comprising amino acids having the sequence set forth in SEQ ID NO: 83.

63. The antibody of any one of claims 1-62, wherein the single domain antibody comprises a heavy chain variable region comprising the amino acid sequence having at least about 90% sequence identity to an amino acid sequence selected from the group consisting of SEQ ID NOs: 4, 8, 12, 16, 20, 24, 28, 32, 36, 40, 44, 48, 52, 56, 60, 64, 68, 72, 76, 80 and 84.

64. The antibody of any one of claims 1-63, wherein the single domain antibody comprises a humanized framework.

65. The antibody of any one of claims 1-64, wherein the antibody comprises a Fc region.

66. The antibody of any one of claims 1-65, wherein the Fc region comprises a human Fc region.

67. The antibody of any one of claims 1-66, wherein the Fc region comprises a Fc region selected from the group consisting of the Fc regions of IgG, IgA, IgD, IgE and IgM.

68. The antibody of any one of claims 1-67, wherein the Fc region comprises a Fc region selected from the group consisting of the Fc regions of IgG1, IgG2, IgG3 and IgG4.

69. The antibody of any one of claims 1-68, wherein the Fc region comprises an IgG1 Fc region.

70. The antibody of claim 69, wherein the IgGI Fc region comprising one or more mutation that enhances an antibody-dependent cell-mediated cytotoxicity (ADCC).

71. The antibody of claim 70, wherein the IgG1 Fc region comprises the mutations of L235V, F243L, R292P, Y300L and P396L.

72. The antibody of claim 70, wherein the IgGI Fc region comprises the mutations of S239D, A330L and I332E.

73. The antibody of any one of claims 1-72, wherein the heavy chain variable region is linked to a Fc region via a linker.

74. The antibody of claim 73, wherein the linker is a peptide linker.

75. The antibody of claim 74, wherein the peptide linker comprises about four to about thirty amino acids.

76. The antibody of claim 74 or 75, wherein the peptide linker comprise an amino acid sequence selected from the group consisting of SEQ ID NOs: 195-220.

77. The antibody of any one of claims 1-76, wherein the antibody comprises a multispecific antibody, e.g., a bispecific antibody, a full-length immunoglobulin, a single-chain Fv (scFv) fragment, a Fab fragment, a Fab' fragment, a F(ab')2, an Fv fragment, a disulfide stabilized Fv fragment (dsFv), a (dsFv)2, a VHH, a Fv-Fc fusion, a scFv-Fc fusion, a scFv-Fv fusion, a diabody, a tribody, a tetrabody or any combination thereof.

78. The antibody of any one of claims 1-77, wherein the antibody comprises a multispecific antibody, e.g., a bispecific antibody, which comprises a second antibody moiety that specifically binds to a second antigen.

79. The antibody of claim 78, wherein the second antigen is a tumor associated antigen.

80. The antibody of claim 79, wherein the tumor associated antigen is selected from the group consisting of Her-2, EGFR, PDL1, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor-associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1) and any combination thereof.

81. The antibody of claim 80, wherein the second antigen is an immune checkpoint regulator.

82. The antibody of claim 81, wherein the immune checkpoint regulator is selected from the group consisting of PD1, CTLA4, LAG-3, 2B4, BTLA and any combination thereof.

83. The antibody of any one of claims 1-82, wherein the antibody is conjugated to a therapeutic agent or a label.

84. The antibody of claim 83, wherein the label is selected from the group consisting of a radioisotope, a fluorescent dye and an enzyme.

85. An immunoconjugate comprising the antibody of any one of claims 1-84, linked to a therapeutic agent.

86. The immunoconjugate of claim 85, wherein the therapeutic agent is a cytotoxin.

87. The immunoconjugate of claim 85, wherein the therapeutic agent is a radioactive isotope.

88. A pharmaceutical composition comprising a) the antibody of any one of claims 1-84 or the immunoconjugate of any one of claims 85-87, and b) a pharmaceutically acceptable carrier.

89. A nucleic acid encoding the antibody of any one of claims 1-84.

90. A vector comprising the nucleic acid of claim 89.

91. A host cell comprising the nucleic acid of claim 89 or the vector of claim 90.

92. A method for preparing an antibody of any one of claims 1-84 comprising expressing the antibody in the host cell of claim 91 and isolating the antibody from the host cell.

93. A method of reducing tumor burden in a subject, the method comprising administering to the subject an effective amount of an antibody of any one of claims 1-84, an immunoconjugate of any one of claims 85-87, or a pharmaceutical composition of claim 88.

94. The method of claim 93, wherein the method reduces the number of tumor cells.

95. The method of claim 93 or 94, wherein the method reduces tumor size.

96. The method of any one of claims 93-95, wherein the method eradicates the tumor in the subject.

97. The method of any one of claims 93-95, wherein the tumor is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer and a combination thereof.

98. A method of treating and/or preventing a neoplasm, the method comprising administering to the subject an effective amount of an antibody of any one of claims 1-84, an immunoconjugate of any one of claims 85-87, or a pharmaceutical composition of claim 88.

99. A method of lengthening survival of a subject having a neoplasm, the method comprising administering to the subject an effective amount of an antibody of any one of claims 1-84, an immunoconjugate of any one of claims 85-87, or a pharmaceutical composition of claim 88.

100. The method of claim 98 or 99, wherein the neoplasm is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer and a combination thereof.

101. An antibody of any one of claims 1-84 for use as a medicament.

102. An antibody of any one of claims 1-84 for use in treating cancer.

103. A pharmaceutical composition of claim 88 for use as a medicament.

104. A pharmaceutical composition of claim 88 for use in treating cancer.

105. The antibody of claim 102 or the pharmaceutical composition of claim 104, wherein the cancer is selected from the group consisting of mesothelioma, lung cancer, pancreatic cancer, ovarian cancer, breast cancer, colon cancer, pleural tumor, glioblastoma, esophageal cancer, gastric cancer, synovial sarcoma, thymic carcinoma, endometrial carcinoma, stomach cancer, cholangiocarcinoma, head and neck cancer, blood cancer and a combination thereof.

106. A kit comprising an antibody of any one of claims 1-84, an immunoconjugate of any one of claims 85-87, a pharmaceutical composition of claim 88, a nucleic acid of claim 89, a vector of claim 90 or a host cell of claim 91.

107. The kit of claim 106, further comprising a written instruction for treating and/or preventing a neoplasm.
